(19) Europäisches Patentamt
European Patent Office
Office européen des brevets

(11) Veröffentlichungsnummer: **0 070 803**
**A2**

# (12) EUROPÄISCHE PATENTANMELDUNG

(21) Anmeldenummer: **82810299.6**

(22) Anmeldetag: **12.07.82**

(51) Int. Cl.³: **C 07 D 501/20**, C 07 D 501/57, C 07 D 501/59, C 07 D 249/14, A 61 K 31/545

(30) Priorität: **16.07.81 CH 4668/81**

(43) Veröffentlichungstag der Anmeldung: **26.01.83 Patentblatt 83/4**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Csendes, Ivan, Dr., Finkenstrasse 9, CH-4104 Oberwil (CH)**
Erfinder: **Müller, Beat, Dr., Vogesenstrasse 31, CH-4106 Therwil (CH)**

## (54) Antibiotisch wirksame Aminotriazolyl-Cephalosporinderivate und ihre Herstellung.

(57) 7β-Aminotriazolylacetylamino-3-cephem-4-carbonsäureverbindungen der Formel

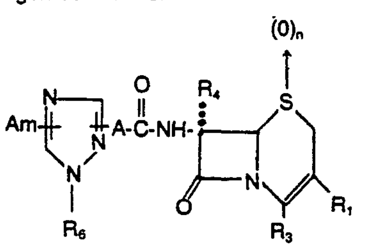

(I)

worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegenenfalls geschützte Carboxylgruppe bedeutet, $R_4$ für Wasserstoff oder Niederalkoxy steht, A eine Gruppe $-CH_2-$, $-C(=O)-$ oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenfalls substituierten Kohlenwasserstoffrest, organisches Sulfonyl oder Acyl darstellt und Am eine gegebenenfalls substituierte Aminogruppe ist, und Salze von solchen Verbindungen, die eine salzbildende Gruppierung aufweisen, besitzen antibiotische Eigenschaften und sind gegen grampositive und gramnegative Mikroorganismen wirksam. Die neuen Verbindungen werden in an sich bekannter Weise hergestellt. Ebenfalls umfasst sind Ausgangsstoffe.

EP 0 070 803 A2

BEZEICHNUNG GEÄNDERT siehe Titelseite

CIBA-GEIGY AG
Basel (Schweiz)

4-13481/+

Aminotriazolyl-Verbindungen, Verfahren zu ihrer Herstellung, pharmazeutische Präparate enthaltend solche Verbindungen und Verwendung von letzteren.

Die vorliegende Erfindung betrifft neue 7β-Aminotriazolylacetylamino-3-cephem-4-carbonsäureverbindungen, Verfahren zu ihrer Herstellung, pharmazeutische Präparate, welche solche Verbindungen enthalten, und ihre Verwendung zur Herstellung von pharmazeutischen Präparaten oder als pharmakologisch wirksame Verbindungen.

Die Erfindung betrifft insbesondere 7β-Aminotriazolylacetylamino-3-cephem-4-carbonsäureverbindungen der Formel

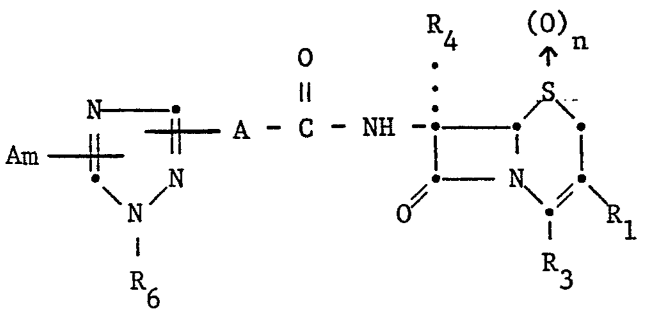

(I),

worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$ oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und Salze von

solchen Verbindungen, die salzbildende Gruppen aufweisen, Verfahren zur Herstellung dieser Verbindungen, solche Verbindungen enthaltende pharmazeutische Mittel und deren Verwendung zur Herstellung von pharmazeutischen Präparaten oder als pharmakologisch wirksame Verbindungen.

In Verbindungen der Formel I hat der Index n in erster Linie den Wert 0. Falls n den Wert 1 hat, kann das entsprechende 1-Oxid in der α- oder β-Form vorliegen.

Der 1,2,4-Triazol-Ring ist in 3- oder in 5-Stellung mit der gegebenenfalls substituierten Aminogruppe Am bzw. der Gruppe -A- verknüpft. In den Triazolyl-Verbindungen der vorliegenden Erfindung kann der 1,2,4-Triazol-Ring daher in der isomeren (bzw., wenn $R_6$ Wasserstoff ist, tautomeren) Formen

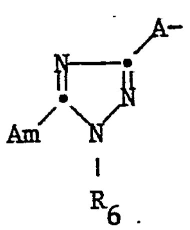

oder

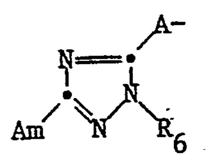

oder als Gemisch dieser isomeren Formen vorliegen. Weiterhin können Verbindungen der Formel I, worin Am Amino ist, auch tautomer als 3- oder 5-Iminotriazolinyl-Verbindungen der Teilformeln

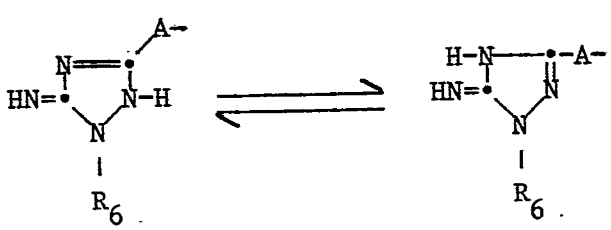

oder

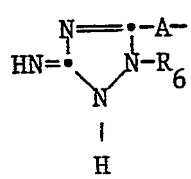

oder als Mischungen der tautomeren Formen vorliegen. Das Gleichgewicht zwischen den Tautomeren hängt von der Art der Substituenten A und/oder $R_6$ und/oder von äusseren Faktoren, z.B. Temperatur, Lösungsmittel und pH-Wert, ab. Im Rahmen der vorliegenden Erfindung werden diese Gruppen in der Beschreibung und in den Ansprüchen als Aminotriazolyl-Gruppen bezeichnet, obgleich die Iminotriazolinylformen ebenfalls umfasst werden sollen.

Die bevorzugten Verbindungen der Formel I enthalten einen 5-Am-triazol-3-yl-Rest.

In der vorliegenden Beschreibung bedeutet der im Zusammenhang mit Definitionen von Substituenten oder Verbindungen verwendete Ausdruck "nieder", dass die entsprechenden Substituenten oder Verbindungen, sofern nicht ausdrücklich anders definiert, bis zu 7, bevorzugt bis zu 4 Kohlenstoffatome enthalten.

Die vor- und nachstehend verwendeten Allgemeindefinitionen haben im Rahmen der vorliegenden Beschreibung vorzugsweise die folgenden Bedeutungen:

Veräthertes Hydroxy $R_1$ ist in erster Linie Niederalkoxy, z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy, aber auch Arylniederalkoxy, worin Aryl insbesondere gegebenenfalls, z.B. durch Niederalkoxy, Halogen oder Nitro,substituiertes Phenyl bedeutet, z.B. Benzyloxy, Diphenylmethoxy, p-Methoxybenzyloxy oder p-Nitrobenzyloxy.

Verestertes Hydroxy $R_1$ ist durch eine anorganische oder organische Säure verestertes Hydroxy, in erster Linie Halogen, ferner Niederalkanoyloxy, Niederalkansulfonyloxy oder Arensulfonyloxy, z.B. insbesondere Chlor, aber auch Fluor, Brom oder Jod, Acetyloxy, Propionyloxy, Methansulfonyloxy, Aethansulfonyloxy, Benzolsulfonyloxy oder o- oder p-Toluolsulfonyloxy.

Veräthertes Mercapto $R_1$ ist in erster Linie Heterocyclylthio, worin Heterocyclyl vorzugsweise einen fünf- oder sechsgliedrigen, gegebenenfalls teilweise gesättigten aromatischen Heterocyclylrest darstellt, der mindestens ein Ringstickstoffatom und gegebenenfalls weitere Ringstickstoff-, Ringsauerstoff- und/oder Ringschwefelatome enthält

und über ein Ringkohlenstoffatom mit dem Thioschwefel verbunden ist, und der gegebenenfalls substituiert sein kann, z.B. einer der unten genannten Heterocyclylthioreste $R_2$. $R_1$ kann aber auch für gegebenenfalls, z.B. durch Hydroxy, Amino oder Carboxy, substituiertes Niederalkylmercapto, wie für Methylmercapto, Aethylmercapto oder Carboxymethylmercapto, stehen.

Funktionell abgewandeltes Carboxy $R_2$ ist beispielsweise durch Niederalkyl, wie Methyl oder Aethyl, oder durch eine physiologisch abspaltbare Gruppe, z.B. Pivaloyloxymethyl, verestertes, oder durch Ammoniak oder durch ein primäres oder sekundäres Amin, wie ein Mono- oder Diniederalkylamin, z.B. Methyl-, Aethyl-, Dimethyl- oder Diäthylamin, amidiertes, oder, wie für den Rest $R_3$ angegeben, geschütztes Carboxy.

In einer Acyloxygruppe $R_2$ ist Acyl beispielsweise der Acylrest einer gegebenenfalls substituierten niederaliphatischen Carbonsäure, eines Kohlensäureniederalkylesters, einer Niederalkansulfonsäure, einer aromatischen Sulfonsäure, einer aromatischen Carbonsäure, einer durch Aryl substituierten Niederalkancarbonsäure oder einer gegebenenfalls N-substituierten Carbaminsäure.

Solche Acyloxygruppen $R_2$ sind beispielsweise gegebenenfalls substituiertes Niederalkanoyloxy, insbesondere Acetyloxy, ferner Formyloxy, Propionyloxy, Butyryloxy, Isobutyryloxy, Valeryloxy, Isovaleryloxy, Hexanoyloxy, Heptanoyloxy, Pivaloyloxy, Acetoacetoxy, Oxalyloxy, Succinyloxy und dergleichen, Niederalkoxycarbonyloxy, wie Methoxycarbonyloxy, Aethoxycarbonyloxy, Propoxycarbonyloxy, 1-Cyclopropyläthoxycarbonyloxy, Isopropoxycarbonyloxy, Butoxycarbonyloxy, tert.-Butoxycarbonyloxy, Pentyloxycarbonyloxy, tert.-Pentyloxycarbonyloxy, Hexyloxycarbonyloxy und dergleichen, Niederalkansulfonyloxy, wie Mesyloxy, Aethansulfonyloxy, Propansulfonyloxy, Isopropansulfonyloxy, Butansulfonyloxy und dergleichen, Arensulfonyloxy, wie Benzolsulfonyloxy, Tosyloxy und dergleichen, Aroyloxy, wie Benzoyloxy, Toluoyloxy, Naphthoyloxy, Phthaloyloxy, Indancarbonyloxy und dergleichen, Arylniederalkanoyloxy, wie Phenylacetyloxy und dergleichen, wobei der oben angegebene Acylrest geeignete Substituenten, wie Halogen, z.B. Chlor, Brom, Jod oder Fluor, Hydroxy, Cyano, Nitro, niederes Alkoxy, z.B. Methoxy, Aethoxy, Propoxy, Isopropoxy und dergleichen, niederes Alkyl, z.B. Methyl, Aethyl, Propyl, Isopropyl, Butyl und dergleichen, niederes Alkenyl, z.B. Vinyl, Allyl und dergleichen, oder z.B. Phenyl, Tolyl und dergleichen aufweisen kann. Beispiele für Acyloxy mit einem oder mehreren solchen Substituenten sind vorzugsweise Halogenniederalkanoyloxy, z.B. Chloracetyloxy, Dichloracetyloxy, Trichloracetyloxy oder Trifluoracetyloxy.

In dem Acylrest einer gegebenenfalls N-substituierten Carbaminsäure sind N-Substituenten gegebenenfalls durch Halogen, z.B. Chlor, oder durch Niederalkanoyloxy, z.B. Acetyloxy oder Propionyloxy, substituierte Niederalkylreste, z.B. Methyl, Aethyl, 2-Chloräthyl oder 2-Acetoxyäthyl. Solche Acyloxygruppen $R_2$ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy.

In einer Heterocyclylthiogruppe $R_2$ ist Heterocyclyl insbesondere ein gegebenenfalls substituierter, über ein Ringkohlenstoffatom an die Thiogruppe gebundener, monoheterocyclischer Rest mit 1 bis 4 Ringstickstoffatomen und gegebenenfalls einem weiteren Ringheteroatom der Gruppe Sauerstoff und Schwefel oder biheterocyclischer Rest mit 1 bis 5 Ringstickstoffatomen.

Solche Heterocyclylreste sind in erster Linie gegebenenfalls substituierte, z.B. die unten genannten Substituenten enthaltende, monocyclische, fünf- oder sechsgliedrige diaza-, triaza-, tetraza-, thiaza-, thiadiaza-, thiatriaza-, oxaza- oder oxadiazacyclische oder

fünf oder sechs Ringatome pro Ring enthaltende aza-, diaza-, triaza-, tetraza- oder pentazabicyclische Reste aromatischen oder partiell gesättigten Charakters.

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 2-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethyl-

carbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte Heterocyclylthiogruppen $R_2$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio,3-Methyl-1-phenyl-1H-1,2,4-triazol-5-ylthio, 4,5-Dimethyl-4H-1,2,4-triazol-3-ylthio, 3- oder 4-Carboxymethyl- oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiazolylthio, 4,5-Dimethyl-2-thiazolylthio, 3-Isothiazolylthio, 4-Isothiazolylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 2-Carboxymethyl-1,3,4-thiadiazol-5-ylthio, 2-(3-Carboxypropionylamino)-1,3,4-thiadiazol-5-ylthio, 1,2,4-Thiadiazol-5-ylthio, 3-Methyl-1,2,4-thiadiazol-5-ylthio oder 1,2,5-Thiadiazol-3-ylthio, Thiatriazolylthio, z.B. 1,2,3,4-Thiatriazolyl-5-ylthio, gegebenenfalls wie angegeben substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben

substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4-oxadiazol-5-ylthio.

Bevorzugte Heterocyclylthiogruppen $R_2$, worin der heterocyclische Rest einen entsprechenden monocyclischen, sechsgliedrigen Rest oder einen entsprechenden partiell gesättigten Rest darstellt, sind u.a. gegebenenfalls durch Halogen substituiertes 1-Oxido-pyridylthio, z.B. 1-Oxido-2-pyridylthio oder 4-Chlor-1-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio, 3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, oder gegebenenfalls durch Niederalkyl, Amino, Diniederalkylamino oder Carboxy substituiertes 2-Oxo-1,2-dihydro-pyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Carboxy-2-oxo-1,2-dihydro-4-pyrimidinylthio oder gegebenenfalls auf die gleiche Weise substituiertes 4-Oxo-3,4-dihydro-2-pyrimidinylthio, wie insbesondere das unsubstituierte 4-Oxo-3,4-dihydro-2-pyrimidinylthio, oder gegebenenfalls durch Niederalkyl, wie Methyl oder Aethyl, und bis zu zwei Oxo substituiertes Tetrahydrotriazinylthio, insbesondere N-Niederalkyl-5,6-dioxo-1,2,5,6-tetrahydro-1,2,4-triazin-3-ylthio, z.B. 1-, 2- oder 4-Methyl-5,6-dioxo-1,2,5,6-tetrahydro-1,2,4-triazin-3-ylthio, oder die den Oxoverbindungen entsprechenden tautomeren aromatischen Hydroxyverbindungen.

Bevorzugte Heterocycylthiogruppen, worin der Heterocyclus einen entsprechenden fünf oder sechs Ringatome pro Ring enthaltenden bicyclischen Rest darstellt, sind u.a. gegebenenfalls durch Niederalkyl substituiertes Indolylthio, z.B. Indol-2-ylthio oder N-Methylindol-2-ylthio, Isoindolylthio, z.B. Isoindol-2-ylthio, Chinolylthio, z.B. 2-, 4- oder 8-Chinolylthio, gegebenenfalls durch Niederalkyl oder Carboxyniederalkyl substituiertes Benzimidazolylthio, z.B. 1-Methyl-, 1-Carboxymethyl- oder 1-(2-Carboxyäthyl)-benzimidazol-2-ylthio, gegebenenfalls durch Niederalkyl oder Carboxyniederalkyl substituiertes Benzotriazolylthio, z.B. 1-Methyl- oder 1-Carboxymethyl-1H-benzo[d]triazol-5-ylthio, oder insbesondere gegebenenfalls durch Niederalkyl, Carboxy, Carboxyniederalkyl, Carbamoyl, N-mono- oder N-diniederalkyliertes Carbamoylamino, oder N-mono- oder N-diniederalkyliertes Amino substituiertes Tetrazolopyridazinylthio, z.B. 8-Methyl-, 8-Aethyl-, 8-Carboxy-, 8-Carboxymethyl-, 8-(2-Carboxyäthyl)-, 8-(3-Carboxypropyl)-, 8-Carbamoyl-, 8-(N-Methylcarbamoyl)-, 8-(N,N-Dimethylcarbamoyl)-, 8-Amino-, 8-Dimethylamino- oder 8-Diäthylamino-tetrazolo[1,5-b]pyridazin-6-ylthio.

Ammonio $R_2$ ist z.B. von tertiären organischen Basen, vorzugsweise von entsprechenden aliphatischen Aminen oder in erster Linie von entsprechenden heterocyclischen Stickstoffbasen abgeleitetes, über das Stickstoffatom mit dem Methylkohlenstoffatom verbundenes Ammonio und liegt in quaternisierter, positiv geladener Form vor. Ammonio ist u.a. Triniederalkylammonio, z.B. Trimethylammonio, Triäthylammonio, Tripropylammonio oder Tributylammonio, insbesondere aber gegebenenfalls substituiertes, z.B. durch Niederalkyl, wie Methyl, Hydroxyniederalkyl, wie Hydroxymethyl, Niederalkoxyniederalkyl, z.B. Methoxymethyl oder 2-Methoxyäthyl, Sulfoniederalkyl, z.B. Sulfomethyl, Amino, substituiertes Sulfonamido, wie 4-Aminophenylsulfonamido, Hydroxy, Halogen, wie Fluor, Chlor, Brom oder Jod, Halogenniederalkyl, wie Trifluormethyl, Sulfo, gegebenenfalls funktionell abgewandeltes Carboxy, wie Carboxy, Niederalkoxycarbonyl, z.B. Methoxycarbonyl, Cyan, gegebenenfalls durch Niederalkyl, z.B. Methyl oder

Aethyl, oder Hydroxyniederalkyl, z.B. Hydroxymethyl, N-mono- oder N,N-disubstituiertes Carbamoyl, z.B. Carbamoyl, N-Methylcarbamoyl oder N,N-Dimethyl-carbamoyl, oder gegebenenfalls durch Niederalkyl N-substituiertes Hydrazinocarbonyl, z.B. Hydrazinocarbonyl, Carboxyniederalkyl, wie Carboxymethyl oder 2-Carboxyäthyl, Niederalkanoyl, wie Acetyl, oder 1-Niederalkyl- pyrrolidinyl, wie 1-Methyl-2-pyrrolidinyl, mono- oder polysubstituiertes, monocyclisches oder bicyclisches Ammonio aromatischen Charakters, mit 1, 2 oder 3 Ringstickstoff- und gegebenenfalls einem Ringschwefelatom, wie Pyrimidinio, Pyridazinio, Thiazolio, Chinolinio und in erster Linie Pyridinio, Triazolio, vor allem 1,2,3-Triazolio, oder Pyrazolio.

Heterocyclisches Ammonio $R_2$ ist in erster Linie gegebenenfalls durch Niederalkyl, Hydroxyniederalkyl, Niederalkoxyniederalkyl, Carboxyniederalkyl, Sulfoniederalkyl, Amino, substituiertes Sulfonamido, Hydroxy, Halogen, Trifluormethyl, Sulfo, Carboxy, Niederalkoxycarbonyl, Cyan, Niederalkanoyl, 1-Niederalkyl-pyrrolidinyl oder gegebenenfalls durch Niederalkyl oder Hydroxyniederalkyl N-substituiertes Carbamoyl substituiertes Pyridinio, z.B. Pyridinio, 2-, 3- oder 4-Methyl-pyridinio, 3,5-Dimethyl-pyridinio, 2,4,6-Trimethylpyridinio, 2-, 3- oder 4-Aethylpyridinio, 2-, 3- oder 4-Propyl-pyridinio oder insbesondere 3- oder 4-Hydroxymethylpyridinio, 3- oder 4-Methoxymethylpyridinio, 3- oder 4-Carboxymethylpyridinio, ferner 2-Amino-, 2-Amino-5-carbamoyl-pyridinio oder 2-Amino-6-methyl-pyridinio, 2-(4-Aminophenylsulfonylamido)-pyridinio, 3-Hydroxy-pyridinio, 3- oder 4-Fluor-, 3- oder 4-Chlor-, 3- oder 4-Jod- oder insbesondere 3- oder 4-Brompyridinio, 4-Trifluormethyl-pyridinio, 4-Sulfoäthylpyridinio, 3-Sulfopyridinio, 2-, 3- oder 4-Carboxy- oder 2,3- oder 3,4-Dicarboxy-pyridinio, 4-Methoxycarbonyl-pyridinio, 3- oder 4-Cyan-pyridinio, 3-Carboxymethyl-pyridinio, 3- oder 4-Acetyl-pyridinio, 3-(1-Methyl-2-pyrrolidinyl)-pyridinio, und insbesondere 4-Carbamoyl-, 3-Carbamoyl-, 3-Carboxymethyl-4-carbamoyl-, 3- oder 4-N-Methylcarbamoyl-, 4-N,N-Dimethylcarbamoyl-, 4-N-Aethylcarbamoyl-, 3-N,N-Diäthylcarbamoyl, 4-N-Propylcarbamoyl-, 4-Isopropylcarbamoyl-und 4-Hydroxymethyl-carbamoyl-pyridinio. Als Ammonio $R_2$ ist weiterhin bevorzugt 1,2,3-Triazolio, insbesondere 1-(1,2,3-Triazolio), wie 3-Niederalkyl-1-(1,2,3-triazolio), z.B. 3-Methyl-, 3-Aethyl-, 3-Propyl-, 3-Butyl- oder 3-tert.Butyl-1-(1,2,3-triazolio) und Pyrazolio, insbesondere 2-Niederalkyl-1-pyrazolio, z.B. 2-Methyl-, 2-Aethyl-, 2-Propyl-, 2-Butyl- oder 2-tert.-Butyl-1-pyrazolio und 2-Carboxyniederalkyl-1-pyrazolio, z.B. 2-Carboxymethyl- oder 2-(2-Carboxyäthyl)-1-pyrazolio.

Eine geschützte Carboxylgruppe $R_3$ ist insbesondere eine veresterte Carboxylgruppe, welche unter schonenden chemischen oder unter physiologischen Bedingungen leicht spaltbar ist.

Veresterte Carboxylgruppen $R_3$, welche unter schonenden chemischen Bedingungen leicht spaltbar sind, enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl, mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Nitro-benzyloxycarbonyl oder 4-Methoxy-benzyloxycarbonyl, oder gegebenenfalls,z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyäthoxycarbonyl oder 1-Aethoxymethoxycarbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl,

z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen und/oder Nitro substituierten, aliphatischen, araliphatischen, cycloaliphatischen oder aromatischen Kohlenwasserstoffrest mit z.B. bis zu 15 Kohlenstoffatomen, wie entsprechendes, gegebenenfalls substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, in veresterter Form vorliegende geschützte Carboxylgruppen sind entsprechende Silyloxycarbonyl-, insbesondere organische Silyloxycarbonylgruppen, ferner entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxyniederalkyl-halogen-silyl, z.B. Methoxy-methyl-chlor-silyl, oder Diniederalkyl-halogen-silyl, z.B. Dimethyl-chlor-silyl, oder entsprechend substituierte Stannylgruppen, z.B. Tri-n-butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen

sind Niederalkanoyloxymethoxycarbonyl, z.B. Acetyloxymethoxycarbonyl oder Pivaloyloxymethoxycarbonyl, Aminoniederalkanoyloxymethoxycarbonyl, insbesondere α-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl. Eine weitere bevorzugte, unter physiologischen Bedingungen spaltbare Carboxylgruppe ist die Niederalkoxycarbonyloxymethoxycarbonyl-Gruppe, z.B. Methoxy-, Aethoxy- oder Propoxycarbonyloxymethoxycarbonyl.

Eine Niederalkoxy-Gruppe $R_4$ enthält bevorzugt 1 bis 4 C-Atome und ist beispielsweise Aethoxy, Propoxy, Butoxy oder insbesondere Methoxy.

Die Gruppe der Formel $-C(=N-O-R_5)-$ liegt in der syn(oder Z)-Form oder in der anti(oder E)-Form vor, wobei die syn(oder Z)-Form bevorzugt ist.

Eine Niederalkylgruppe $R_5$ enthält vorzugsweise 1-4 C-Atome und ist beispielsweise Aethyl, Propyl, Butyl oder insbesondere Methyl.

Eine Cycloalkylgruppe $R_5$ enthält vorzugsweise 3-8, in erster Linie 3-6 Ringglieder, und ist z.B. Cyclobutyl, Cyclopentyl oder Cyclohexyl, oder insbesondere Cyclopropyl.

Substituenten von substituiertem Niederalkyl oder Cycloalkyl $R_5$ sind z.B. Aryl, wie Phenyl oder Furyl, gegebenenfalls veräthertes Hydroxy, z.B. Niederalkoxy, primäres, sekundäres oder tertiäres Amino z.B. Amino oder Diniederalkylamino, gegebenenfalls funktionell abgewandeltes, inkl. verestertes, amidiertes oder geschütztes Carboxyl oder Sulfo, sowie gegebenenfalls durch Niederalkyl N-substituiertes Ureidocarbonyl. Bevorzugt sind die substituierten Niederalkyl- und

Cycloalkylgruppen durch eine Carboxyl- oder Sulfogruppe substituiert, wobei diese bevorzugt am Kohlenstoffatom, das mit dem Sauerstoffatom der Hydroxyiminogruppe verbunden ist, stehen.

Solche substituierte Niederalkyl- und Cycloalkylgruppen $R_5$ sind beispielsweise Benzyl, 2-Phenyläthyl, Furylmethyl, 2-Aminoäthyl, 2-Dimethylaminoäthyl, Carboxymethyl, 1- oder 2-Carboxyäthyl, 1-, 2- oder 3-Carboxyprop-1-yl, 1-, 2- oder 3-Carboxyprop-2-yl, 1-Carboxybut-1-yl, 1-Carboxycycloprop-1-yl und 1-Carboxycyclobut-1-yl, sowie entsprechende durch Sulfo substituierte Niederalkyl- und Cycloalkylgruppen.

Die Carboxy- und Sulfogruppen im Rest $R_5$ können beispielsweise durch Niederalkyl, wie Methyl, Aethyl oder tert.-Butyl, oder eine physiologisch abspaltbaren Gruppe, z.B. durch Pivaloyloxymethyl, verestert oder durch Ammoniak oder durch ein primäres oder sekundäres Amin, z.B. ein Mono- oder Diniederalkylamin, z.B. Methyl- oder Aethylamin oder Dimethyl- oder Diäthylamin, amidiert sein oder durch die weiter unten genannten Gruppen geschützt sein.

Eine Cycloalkenylgruppe $R_5$ enthält vorzugsweise 3 bis 8, in erster Linie 3 bis 6 Ringglieder und bedeutet z.B. Cyclopropenyl, Cyclobutenyl, Cyclopentenyl oder Cyclohexenyl.

Niederalkenyl $R_5$ ist z.B. Vinyl, 1-Propenyl, 1-, 2-, oder 3-Butenyl oder insbesondere Allyl.

Gegebenenfalls N-substituiertes Carbamoyl als Rest $R_5$ ist beispielsweise eine Gruppe $-C(=O)-NHR_5'$ worin $R_5'$ Wasserstoff, Niederalkyl, z.B. Methyl, Aethyl oder 1- oder 2-Propyl, gegebenenfalls geschütztes Carboxyniederalkyl, z.B. Carboxymethyl, 1- oder 2-Carboxyäthyl oder

1-, 2- oder 3-Carboxypropyl, worin Carboxy durch eine der üblichen Carboxylschutzgruppen geschützt und beispielsweise durch Niederalkyl, z.B. Methyl, Aethyl, n- oder iso-Propyl, oder n- oder tert.-Butyl verestert sein kann, gegebenenfalls geschütztes Sulfoniederalkyl, z.B. Sulfomethyl, 1- oder 2-Sulfoäthyl oder 1-, 2- oder 3-Sulfopropyl, worin Sulfo durch eine der üblichen Sulfoschutzgruppen geschützt und beispielsweise durch Niederalkyl, z.B. Methyl oder Aethyl, verestert sein kann, gegebenenfalls geschütztes Hydroxyniederalkyl, z.B. Hydroxymethyl, 2-Hydroxyäthyl oder 2- oder 3-Hydroxypropyl, worin Hydroxy durch eine der üblichen Hydroxyschutzgruppen geschützt und beispielsweise acyliert, z.B. acetyliert, sein kann, gegebenenfalls geschütztes Aminoniederalkyl, z.B. 2-Aminoäthyl, 2- oder 3-Aminopropyl oder 2-, 3- oder 4-Aminobutyl, worin Amino durch eine der üblichen Aminoschutzgruppen geschützt und beispielsweise acyliert, z.B. acetyliert, sein kann, Arylniederalkyl, beispielsweise Phenylniederalkyl, z.B. Benzyl oder 1- oder 2-Phenyläthyl, Halogenniederalkyl, beispielsweise Fluor-, Chlor- oder Bromniederalkyl, z.B. 2-Chloräthyl, 3-Chlorpropyl- oder 4-Chlorbutyl, Aryl, z.B. Phenyl, oder durch Niederalkyl, z.B. Methyl, Niederalkoxy, z.B. Methoxy, Halogen, z.B. Chlor oder Nitro, ein- bis dreifach substituiertes Phenyl bedeutet.

Ein gegebenenfalls substituierter Kohlenwasserstoffrest $R_6$ ist in erster Linie ein entsprechender aliphatischer, cycloaliphatischer, aromatischer oder araliphatischer Kohlenwasserstoffrest mit bis zu 20 C-Atomen, insbesondere gegebenenfalls substituiertes Niederalkyl, Niederalkenyl, Cycloalkyl, Phenyl oder Phenylniederalkyl.

Niederalkyl $R_6$ ist insbesondere Methyl, Aethyl, Propyl, Isopropyl, Butyl oder Isobutyl.

Als Niederalkenyl besitzt $R_6$ vorzugsweise 2 bis 5 C-Atome. Solche Reste sind beispielsweise Vinyl, Allyl, 1-Propenyl oder 1-Isobutenyl.

Cycloalkyl $R_6$ enthält 3 bis 8, vorzugsweise 3 bis 6 C-Atome und ist vor allem Cyclopropyl, Cyclobutyl, Cyclopentyl oder Cyclohexyl.

Phenylniederalkyl $R_6$ ist z.B. Benzyl, 1- oder 2-Phenyläthyl, Diphenylmethyl oder Trityl.

Substituenten für Niederalkenyl-, Cycloalkyl- und insbesondere Niederalkylreste $R_6$ sind z.B. Hydroxy, Niederalkoxy, z.B. Methoxy oder Aethoxy, Carboxy, durch übliche Schutzgruppen geschütztes oder durch Niederalkyl, z.B. Methyl oder Aethyl, verestertes Carboxy, Amino, Cyano, Niederalkylamino, z.B. Methylamino oder Aethylamino, Diniederalkylamino, z.B. Dimethyl- oder Diäthylamino, Carbamoyl, Halogen, z.B. Chlor oder Brom, Sulfo, Niederalkylthio, z.B. Methylthio oder Aethylthio, oder Aminosulfinyl. Solche substituierten Niederalkyl-, Niederalkenyl- und Cycloalkylgruppen $R_6$ sind beispielsweise Carboxymethyl, Cyanoäthyl, 2-Carboxyäthyl, 2-Dimethylaminoäthyl, 2-Aminoäthyl, 2-Methoxyäthyl, 2-Carbamoyläthyl, Sulfomethyl, 2-Sulfoäthyl und 2-Carboxyvinyl.

Phenyl- und Phenylniederalkylreste $R_6$ können im Phenylring z.B. durch Hydroxy, Niederalkoxy, z.B. Methoxy oder Aethoxy, Carboxy, durch übliche Schutzgruppen geschütztes oder durch Niederalkyl, z.B. Methyl oder Aethyl, verestertes Carboxy, Amino, Carbamoyl, Halogen, z.B. Fluor, Chlor, Brom oder Jod, Sulfo, Niederalkylthio, z.B. Methylthio oder Aethylthio, Trifluormethyl oder Aminosulfinyl substituiert sein. Solche Reste sind beispielsweise 2-, 3- oder 4-Carboxyphenyl, 2-, oder 4-Carbamoylphenyl, sowie 3- oder 4-Sulfophenyl.

Organisches Sulfonyl $R_6$ ist z.B. Niederalkylsulfonyl oder gegebenenfalls durch Niederalkyl, z.B. Methyl oder Aethyl, oder Halogen, z.B.

Fluor, Chlor oder Brom, substituiertes Phenylsulfonyl oder Phenylniederalkylsulfonyl.

Niederalkylsulfonyl ist z.B. Methylsulfonyl, Aethylsulfonyl, n-Propylsulfonyl, Isopropylsulfonyl oder n-Butylsulfonyl.

Gegebenenfalls wie angegeben substituiertes Phenylsulfonyl oder Phenylniederalkylsulfonyl ist z.B. Phenylsulfonyl, 4-Methylphenylsulfonyl, 4-Bromphenylsulfonyl oder Benzylsulfonyl.

Ein Acylrest $R_6$ ist in erster Linie ein von einer Carbonsäure mit bis zu 12 C-Atomen, einem Halbester der Kohlensäure oder einer Carbaminsäure abgeleiteter Acylrest und bedeutet vorzugsweise Aroyl, Niederalkanoyl, Niederalkoxycarbonyl oder gegebenenfalls mono- oder diniederalkyliertes Carbamoyl.

Aroyl $R_6$ ist in erster Linie ein unsubstituierter oder durch Halogen, z.B. Chlor oder Brom, Niederalkyl, z.B. Methyl, Hydroxy, Niederalkoxy, z.B. Methoxy oder Aethoxy, Diniederalkylamino, z.B. Dimethyl- oder Diäthylamino, Niederalkylsulfonylamino, z.B. Mesylamino, Niederalkanoylamino, z.B. Acetamino, Amino oder Nitro substituierter Benzoyl- oder Naphthoyl-Rest oder ein unsubstituierter oder durch Niederalkyl, z.B. Methyl, oder Halogen, z.B. Fluor, Chlor oder Brom, substituierter Pyridoyl-, wie 2-, 3- oder 4-Pyridoyl-, Thenoyl-, wie 2- oder 3-Thenoyl-, Furoyl-, wie 2- oder 3-Furoyl-, oder Pyrroyl-, wie 2- oder 3-Pyrroyl-Rest.

Niederalkanoyl $R_6$ ist z.B. Formyl, Acetyl, Propionyl, Butyryl oder Isobutyryl.

Niederalkoxycarbonyl $R_6$ ist z.B. Methoxy-, Aethoxy-, n-Propoxy-, Isopropoxy-, n-Butoxy-, Isobutoxy- oder tert.Butoxycarbonyl.

Mononiederalkyliertes Carbamoyl $R_6$ ist z.B. N-Methyl- oder N-Aethylcarbamoyl. Diniederalkyliertes Carbamoyl ist z.B. N,N-Dimethyl- oder N,N-Diäthylcarbamoyl.

Eine substituierte Aminogruppe Am ist z.B. eine Diniederalkylamino-niederalkylidenamino-Gruppe und in erster Linie eine 1-Diniederalkyl-amino-niederalkylidenamino-Gruppe. In solchen Gruppen bedeutet Diniederalkylamino z.B. Dimethylamino, Diäthylamino, Di-n-propylamino, Diisopropylamino oder Di-n-butylamino und Niederalkyliden z.B. Aethyliden, 1-Propyliden, 2-Propyliden oder insbesondere Methyliden. Geeignete Diniederalkylamino-niederalkylidenamino-Gruppen sind beispielsweise 1-Dimethylaminoäthylidenamino,1-Diäthylaminoäthylidenamino oder insbesondere Dimethylaminomethylidenamino und Diäthylaminomethylidenamino. Eine substituierte Aminogruppe Am kann aber auch eine geschützte Aminogruppe sein.
Die in Verbindungen der Formel (I) vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in J.F.W.Mc Omie "Protective Groups in Organic Chemistry", Plenum Press, London, New York, 1973, ferner in "The Peptides", Vol. I, Schröder und Lübke, Academic Press, London, New York, 1965, sowie in "Methoden der organischen Chemie", Houben-Weyl, 4. Auflage, Bd. 5/1, Georg Thieme Verlag, Stuttgart, 1974.

So sind Carboxylgruppen z.B. in veresterter Form geschützt, wobei solche Estergruppierungen unter schonenden Bedingungen leicht abspaltbar sind. Geeignete Schutzgruppen sind beispielsweise die bei der geschützten Carboxylgruppe $R_3$ genannten.

Eine geschützte Aminogruppe kann z.B. in Form einer leicht spaltbaren Acylamino-, Arylmethylamino-, verätherten Mercaptoamino-, 1-Acylniederalk-1-en-2-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder einer gegebenenfalls z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder gegebenenfallls substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten aliphatischen, araliphatischen, cycloaliphatischen oder aromatischen Kohlenwasserstoffrest mit z.B. bis zu 15 C-Atomen bedeuten, beispielsweise entsprechendes, gegebenenfalls substituiertes Niederalkyl, Phenylniederalkyl, Cycloalkyl oder Phenyl, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-phenylniederalkylphosphoryl, z.B. Dibenzylphosphoryl oder Di-4-nitrobenzylphosphoryl, gegebenenfalls substituiertes Phenyloxy-phenyl-phosphonyl, z.B. Phenyloxyphenyl-phosphonyl, Diniederalkylphosphinyl, z.B. Diäthylphosphinyl, oder gegebenenfalls substituiertes Diphenylphosphinyl, z.B. Diphenylphosphinyl.

In einer Mono-, Di- oder insbesondere Triarylmethylaminogruppe sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro, substituiertes Phenyl ist. Eine entsprechende Aminoschutzgruppe ist z.B. 4-Nitrophenylthio.

In einem als Aminoschutzgruppe verwendbaren 1-Acyl-niederalk-1-en-2-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butylsilyl, Niederalkoxyniederalkylhalogensilyl, z.B. Methoxymethylchlorsilyl, oder Diniederalkylhalogensilyl, z.B. Dimethylchlorsilyl, oder entsprechend substituiertes Stannyl, z.B. Tri-n-butylstannyl.

Eine Aminogruppe kann auch in protonierter Form geschützt werden; als korrespondierende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl oder Diphenylmethoxycarbonyl, oder 2-Halogen-niederalkoxycarbonyl, wie 2,2,2-Trichloräthoxycarbonyl, ferner Trityl oder Formyl.

Hydroxyschutzgruppen sind z.B. Acylreste, wie gegebenenfalls, z.B. durch Halogen, substituiertes Niederalkanoyl, wie Dichloracetyl oder Trichloracetyl, oder insbesondere die im Zusammenhang mit einer geschützten Aminogruppe genannten Acylreste von Kohlensäurehalbestern, insbesondere 2,2,2-Trichloräthoxycarbonyl, oder organische Silyl- oder Stannylreste, ferner leicht abspaltbare veräthernde Gruppen, wie tert.-Niederalkyl, z.B. tert.-Butyl, 2-oxa- oder 2-thia-aliphatische oder -cycloaliphatische Kohlenwasserstoffreste, in erster Line α-Niederalkoxy-niederalkyl oder 1-Niederalkylthio-niederalkyl, z.B. Methoxymethyl, 1-Methoxyäthyl, 1-Aethoxy-äthyl, 2-Methoxy-2-propyl, 1-Methylthiomethyl, 1-Methylthio-äthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste, z.B. Halogen, wie Chlor, Niederalkoxy, wie Methoxy, und/oder Nitro in Frage kommen.

Eine geschützte Sulfogruppe ist in erster Linie eine veresterte, wie mit einem aliphatischen, cycloaliphatischen, cycloaliphatischaliphatischen, aromatischen oder araliphatischen Alkohol, z.B. einem

Niederalkanol, oder mit einem Silyl- oder Stannylrest, wie Triniederalkylsilyl, veresterte Sulfogruppe. In einer Sulfogruppe kann die Hydroxygruppe, beispielsweise wie die Hydroxygruppe in einer veresterten Carboxygruppe, veräthert sein.

Salze von erfindungsgemässen Verbindungen sind in erster Linie pharmazeutisch verwendbare, nicht-toxische Salze, wie diejenigen von Verbindungen der Formel I mit sauren Gruppen, z.B. mit einer freien Carboxyl- und Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer sauren Gruppe, z.B. einer freien Carboxylgruppe, und einer basischen Gruppe, z.B. einer Aminogruppe, können auch in Form von inneren Salzen, d.h. in zwitterionischer Form, vorliegen, oder es kann ein Teil des Moleküls als inneres Salz und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel I, worin die funktionellen Gruppen in freier, d.h. nicht in geschützter Form, vorliegen, und ihre pharmazeutisch verwendbaren, nicht-toxischen Salze sind wertvolle, antibiotisch wirksame Substanzen, die insbesondere als antibakterielle Antibiotika verwendet werden können. Beispielsweise sind sie in vitro gegen grampositive und gramnegative Kokken, z.B. Staphylococcus aureus, Streptokokken, z.B. Streptococcus pyogenes und Streptococcus pneumoniae, und Neisseria spp., sowie gegen Haemophilus influenzae in minimalen Konzentrationen von ca. 0.01 µg/ml bis ca. 32 µg/ml, gegen Enterobacteriaceae, z.B. Escherichia coli, Klebsiella, Salmonella, Serratia, Enterobacter und Proteus spp. in minimalen Konzentrationen von ca. 0,005 µg/ml bis ca. 128 µg/ml und gegen grampositive Anaerobier, z.B. Clostridium, bzw. gramnegative Anaerobier, z.B. Bacteroides, in minimalen Konzentrationen von ca. 0,005 µg/ml bis ca. 64 µg/ml wirksam. In vivo, bei subcutaner Applikation an der Maus, sind die neuen Verbindungen beispielsweise gegen systemische Infektionen, welche durch grampositive Keime, z.B. Staphylococcus aureus, oder Enterobacteriaceae, z.B. Escherichia coli, hervorgerufen werden, in Minimaldosen von ca. 0,4 mg/kg bis ca. 100 mg/kg wirksam.

In dem folgenden Versuchsbericht wird anhand von ausgewählten Verbindungen die Wirksamkeit von Verbindungen der Formel I gezeigt:

Versuchsbericht

I. Getestete Verbindungen:

Die antibiotische Wirksamkeit der folgenden Verbindungen wurde getestet:

1. 7β-[2-(5-Amino-1-methyl-1H-1,2,4-triazol-3-yl)-acetamino]-3-acetoxymethyl-3-cephem-4-carbonsäure, Natriumsalz (Beispiel 1)

2. 7β-[2-(5-Amino-1-methyl-1H-1,2,4-triazol-3-yl)-2-Z-methoxy-imino-acetamino]-3-acetoxymethyl-3-cephem-4-carbonsäure, Natriumsalz (Beispiel 3)

3. 7β-[2-(5-Amino-1-methyl-1H-1,2,4-triazol-3-yl)-2-Z-methoxyimino-acetamino]-3-(1-methyltetrazol-5-yl-thiomethyl)-3-cephem-4-carbonsäure, Natriumsalz (Beispiel 4)

4. 7β-[2-(5-Amino-1-methyl-1H-1,2,4-triazol-3-yl)-2-Z-methoxyimino-acetamino]-3-(4-carbamoylpyridiniomethyl)-3-cephem-4-carboxylat (Beispiel 5)

5. 7β-[2-(5-Dimethylaminomethylidenamino-1-methyl-1H-1,2,4-triazol-3-yl)-2-Z-methoxyiminoacetamino]-3-(4-carbamoylpyridiniomethyl)-3-cephem-4-carboxylat (Beispiel 5)

6. 7β-[2-(5-Amino-1-methyl-1H-1,2,4-triazol-3-yl)-2-Z-methoxyimino-acetamino]-3-cephem-4-carbonsäure, Natriumsalz (Beispiel 10).

II. Experimentelles:

A. Die antibiotische Aktivität der Testverbindungen in vitro wurde durch die Agarverdünnungsmethode nach Ericsson, H.M., und Sherris, S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen (MIC = minimum inhibitory concentrations) werden in Mikrogramm pro Milliliter (µg/ml) für die geprüften Verbindungen in der Tabelle I angegeben.

B. Die chemotherapeutische Wirksamkeit in vivo gegen systemische Infektionen in weiblichen SPF, $MF_2$ Mäusen wurde nach der Methode von Zak., O., et al., 1979, Drugs Exptl. Clin. Res. 5, 45-59, ermittelt. Die gefundenen $ED_{50}$-Werte in Milligramm Substanz pro Kilogramm Maus (mg/kg) für eine Anzahl von Mikroorganismen werden für die subcutan (s.c.) applizierten Testverbindungen in der Tabelle 2 angegeben.

III. Versuchsergebnisse:

Tabelle 1: Antibiotische Wirksamkeit (in vitro)

| Testverbindung MIC (µg/ml) | 1 | 2 | 3 | 4 | 5 | 6 |
|---|---|---|---|---|---|---|
| Staphylococcus aureus 2999i$^+$p$^+$ | 8 | 8 | 4 | 8 | 8 | 32 |
| Streptococcus pyogenes Aronson | 1 | 0,5 | 0,5 | 1 | 0,5 | 32 |
| Escherichia coli 205 | 32 | 0,1 | 0,05 | 0,2 | 32 | 0,1 |
| Klebsiella pneumoniae 327 | 16 | 0,1 | 0,05 | 0,2 | 32 | 0,1 |
| Serratia marcescens 344 | | 1 | 0,1 | 0,5 | 128 | 0,5 |
| Proteus mirabilis 774 | 128 | 0,05 | 0,1 | 0,2 | 32 | 0,1 |
| Proteus rettgeri 856 | 32 | 0,01 | 0,005 | 0,1 | 8 | 0,02 |
| Pseudomonas aeruginosa ATCC 12055 | | 128 | 128 | 1 | 128 | |
| Clostridium perfringens 194 | 16 | 0,5 | 0,005 | 0,2 | 2 | 8 |
| Bacteroides fragilis LO 1 | | 8 | 8 | 16 | 32 | 4 |
| Neisseria meningitidis 1316 | 2 | 0,02 | 0,1 | 0,5 | 8 | 0,5 |
| Haemophilus influenzae NCTC 4560 | 4 | 1 | 0,5 | 8 | 128 | 2 |

Tabelle 2: Chemotherapeutische Wirksamkeit (in vivo)

| Testverbindung $ED_{50}$ (mg/kg, s.c.) | 1 | 2 | 3 | 4 | 5 | 6 |
|---|---|---|---|---|---|---|
| Staphylococcus aureus 10B | | 10 | > 30 | 28 | 55 | |
| Escherichia coli 205 | > 30 | 1 | 0,4 | 1,7 | >30 | 2,5 |
| Proteus morganii 2359 | > 30 | | > 30 | 50 | 70 | |

Die neuen Verbindungen können deshalb z.B. in Form von antibiotisch wirksamen Präparaten zur Behandlung von Infektionen, die durch grampositive und gramnegative Bakterien und Kokken verursacht werden, Verwendung finden.

Verbindungen der Formel I, worin die funktionellen Gruppen geschützt sind, werden als Zwischenprodukte zur Herstellung von Verbindungen der Formel I verwendet, worin die funktionellen Gruppen in freier Form vorliegen.

Die vorliegende Erfindung betrifft in erster Linie Verbindungen der Formel I, worin n 0 oder 1 ist, $R_1$ Wasserstoff, Niederalkoxy, Phenylniederalkoxy, Halogen, Niederalkanoyloxy, Niederalkansulfonyloxy, Arensulfonyloxy, Heterocyclylthio, worin Heterocyclyl über ein Ringkohlenstoffatom an die Thiogruppe gebundenes, unsubstituiertes oder durch Niederalkyl, das Diniederalkylamino, Carboxy oder Sulfo als Substituenten enthalten kann, substituiertes Thiadiazolyl, Oxadiazolyl, Triazolyl oder Tetrazolyl bedeutet, oder einen Rest $-CH_2-R_2$ darstellt, worin $R_2$ Wasserstoff, Hydroxy, Carboxy, Niederalkoxycarbonyl, Niederalkanoyloxy, Carbamoyloxy, N-Niederalkylcarbamoyloxy, Heterocyclylthio, worin Heterocyclyl einen über ein Ringkohlenstoffatom an die Thiogruppe gebundenen monoheterocyclischer Rest mit 1 bis 4 Ringstickstoffatomen und gegebenenfalls einem weiteren Ringheteroatom der Gruppe

Sauerstoff und Schwefel darstellt, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell gesättigt und/oder unsubstituiert oder durch Niederalkyl, durch Carboxy, Sulfo, Diniederalkylamino, Niederalkanoylamino, Hydroxy oder Halogen substituiertes Niederalkyl, ferner durch Cycloalkyl, Phenyl, Benzyl, Carboxy, Carbamoyl oder N-Niederalkylcarbamoyl substituiert sein können, oder Ammonio, wie Triniederalkylammonio oder unsubstituiertes oder durch Niederalkyl, Hydroxyniederalkyl, Carboxyniederalkyl, Sulfoniederalkyl, Carboxy, Carbamoyl, N-Niederalkylcarbamoyl,Halogen,Sulfo,Niederalkanoyl,Hydroxy oder Cyano substituiertes Pyrazolio, Triazolio, Pyridinio, Pyrimidinio, Pyridazinio, Thiazolinio oder Chinolinio, bedeutet, $R_3$ Carboxyl oder in physiologisch spaltbarer Form verestertes Carboxyl darstellt, $R_4$ für Wasserstoff oder Niederalkoxy steht, A eine Gruppe $-CH_2-$, $-C(=O)-$ oder $-C(=N-O-R_5)-$ ist, worin $R_5$ Wasserstoff, Niederalkyl, Cycloalkyl, durch Phenyl, Niederalkoxy, Carboxy, Amino oder Diniederalkylamino substituiertes Niederalkyl oder Cycloalkyl, ferner Cycloalkenyl, Niederalkenyl, Carbamoyl oder N-Niederalkylcarbamoyl bedeutet, $R_6$ Wasserstoff, Niederalkyl, durch Hydroxy, Niederalkoxy, Carboxy, Niederalkoxycarbonyl, Amino, Niederalkylamino, Diniederalkylamino, Carbamoyl, Halogen, Sulfo, Niederalkylthio oder Aminosulfinyl substituiertes Niederalkyl, ferner Niederalkenyl, Cycloalkyl, Cycloalkenyl, Phenyl, Phenylniederalkyl, Niederalkylsulfonyl, unsubstituiertes oder durch Niederalkyl oder Halogen substituiertes Phenylsulfonyl oder Phenylniederalkylsulfonyl, Niederalkanoyl, Niederalkoxycarbonyl, Carbamoyl oder N-Niederalkylcarbamoyl darstellt und Am Amino oder Diniederalkylamino-niederalkylidenamino ist, sowie Salze, insbesondere pharmazeutisch verwendbare Salze von solchen Verbindungen der Formel I, die salzbildende Gruppen aufweisen.

Die vorliegende Erfindung betrifft vorzugsweise Verbindungen der Formel I, worin n 0 oder 1 ist, $R_1$ Wasserstoff, Niederalkoxy, z.B. Methoxy, Phenylniederalkoxy, z.B. Benzyloxy, Halogen, z.B. Chlor, Heterocyclylthio, worin Heterocyclyl über ein Ringkohlenstoffatom an die Thiogruppe gebundenes, unsubstituiertes oder durch Niederalkyl, z.B. Methyl, substituiertes Thiadiazolyl, z.B. 1,3,4-Thiadiazol-2-yl, oder Oxadiazolyl, z.B. 1,3,4-Oxadiazol-2-yl, bedeutet, oder einen Rest $-CH_2-R_2$ darstellt, worin $R_2$ Wasserstoff, Niederalkanoyloxy, z.B. Acetyloxy, Carbamoyloxy, N-Niederalkylcarbamoyloxy, z.B. N-Methylcarbamoyloxy, unsubstituiertes oder durch Niederalkyl, z.B. Methyl, Carboxyniederalkyl, z.B. Carboxymethyl oder 2-Carboxyäthyl, Sulfoniederalkyl, z.B. Sulfomethyl oder 2-Sulfoäthyl, Diniederalkylaminoniederalkyl, z.B. 2-Dimethylaminoäthyl, oder Niederalkanoylamino, z.B. Acetylamino, substituiertes Tetrazol-5-ylthio, unsubstituiertes oder durch Niederalkyl, z.B. Methyl, oder Carboxyniederalkyl, z.B. Carboxymethyl oder 2-Carboxyäthyl, substituiertes 1,3,4-Thiadiazol-2-ylthio, unsubstituiertes oder durch Niederalkyl, z.B. Methyl, substituiertes 1,3,4-Oxadiazol-2-ylthio, unsubstituiertes oder durch Niederalkyl, z.B. Methyl, oder Carboxyniederalkyl, z.B. Carboxymethyl, substituiertes 1,2,4-Triazol-3-ylthio, unsubstituiertes oder durch Hydroxy substituiertes Pyridazin-6-ylthio, oder unsubstituiertes oder durch Hydroxy substituiertes Pyrimidin-2-ylthio, oder Triniederalkylammonio, z.B. Trimethyl- oder Triäthylammonio, durch Niederalkyl, z.B. Methyl, N-substituiertes 1,2,3-Triazolio, durch Niederalkyl, z.B. Methyl, oder Carboxyniederalkyl, z.B. Carboxymethyl, N-substituiertes Pyrazolio, oder unsubstituiertes oder durch Niederalkyl, z.B. Methyl, Carboxy, Carbamoyl, Hydroxyniederalkyl, z.B. Hydroxymethyl, Carboxyniederalkyl, z.B. Carboxymethyl oder 2-Carboxyäthyl, Sulfoniederalkyl, z.B. Sulfomethyl, Halogen, z.B. Brom oder Chlor, oder Hydroxy substituiertes Pyridinio, bedeutet, $R_3$ Carboxyl oder in physiologisch spaltbarer Form verestertes Carboxyl, wie Niederalkanoyloxymethoxycarbonyl, z.B. Pivaloyloxy- oder Acetyloxymethoxycarbonyl, 2-Phthalidyloxycarbonyl oder Niederalkoxycarbonyloxymethoxycarbonyl, z.B. Aethoxycarbonyloxymethoxycarbonyl, darstellt, $R_4$ Wasserstoff oder, vorzugsweise wenn -A- eine Gruppe $-CH_2-$ ist, Methoxy bedeutet, A eine Gruppe $-CH_2-$, -C(=O)- oder $-C(=N-O-R_5)-$ ist, worin $R_5$ Wasserstoff, Niederalkyl, z.B. Methyl, Phenylniederalkyl, z.B. Benzyl, Carboxyniederalkyl, z.B. 2-Carboxy-2-propyl, Carboxycycloalkyl, z.B. 1-Carboxy-1-cyclopropyl, Carbamoyl, N-Niederalkylcarbamoyl, z.B. N-Methylcarbamoyl, oder Niederalkenyl, z.B. Allyl, bedeutet, und worin die Gruppe $-C(=N-O-R_5)-$ vorzugsweise in der syn-( oder Z-) Konfiguration vorliegt, wobei -A- mit der 3-Stellung des 1,2,4-Triazolringes verknüpft ist, $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. Dimethylaminoäthyl, Niederalkoxycarbonyl, z.B. Methoxy- oder Aethoxycarbonyl, Carbamoyl, Niederalkenyl, z.B. Allyl, Phenyl oder Phenylniederalkyl, z.B. Benzyl oder Diphenylmethyl, oder Niederalkylsulfonyl, z.B. Methylsulfonyl, darstellt, und Am Amino ist, sowie Salze, insbesondere pharmazeutisch verwendbare Salze von solchen Verbindungen der Formel I, die salzbildende Gruppen aufweisen.

Besonders hervorzuheben sind Verbindungen der Formel I, worin n für 0 steht, $R_1$ Wasserstoff, Niederalkoxy, z.B. Methoxy, oder einen Rest $-CH_2-R_2$ darstellt, worin $R_2$ Niederalkanoyloxy, z.B. Acetyloxy, in 1-Stellung durch Niederalkyl, z.B. Methyl, Carboxyniederalkyl, z.B. Carboxymethyl oder 2-Carboxyäthyl, Sulfoniederalkyl, z.B. Sulfomethyl oder 2-Sulfoäthyl, oder Diniederalkylaminoniederalkyl, z.B. 2-Dimethylaminoäthyl, substituiertes 1H-Tetrazol-5-ylthio, in 2-Stellung durch Niederalkyl, z.B. Methyl, oder Carboxyniederalkyl, z.B. Carboxymethyl, substituiertes 1,3,4-Thiadiazol-5-ylthio, 2-Niederalkylpyrazolio, z.B. 2-Methylpyrazolio, 3-Niederalkyl-1-(1,2,3-triazolio), z.B. 3-Methyl-1-(1,2,3-triazolio), Carboxypyridinio, z.B. 3- oder 4-Carboxypyridinio, Carbamoylpyridinio, z.B. 3- oder 4-Carbamoylpyridinio, Hydroxyniederalkylpyridinio, z.B. 4-Hydroxymethylpyridinio, Carboxyniederalkylpyridinio, z.B. 3-Carboxymethylpyridinio, oder Halogenpyridinio, z.B. 3- oder 4-Brompyridinio, bedeutet, $R_3$ Carboxyl

oder Niederalkanoyloxymethoxycarbonyl, z.B. Pivaloyloxymethoxycarbonyl, oder Niederalkoxycarbonyloxymethoxycarbonyl, z.B. Aethoxycarbonyloxymethoxycarbonyl, darstellt, $R_4$ für Wasserstoff oder, vorzugsweise wenn A eine Gruppe $-CH_2-$ ist, für Methoxy steht, A eine Gruppe $-CH_2-$ oder $-C(=N-O-R_5)-$ ist, worin $R_5$ Wasserstoff, Niederalkyl, z.B. Methyl, Carboxyniederalkyl, z.B. 2-Carboxy-2-propyl, Carbamoyl oder N-Niederalkylcarbamoyl, z.B. N-Methylcarbamoyl, bedeutet und worin die Gruppe $-C(=N-O-R_5)-$ in syn-(oder Z-) Konfiguration vorliegt, wobei -A- mit der 3-Stellung des 1,2,4-Triazolringes verknüpft ist, $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. 2-Dimethylaminoäthyl, oder Phenylniederalkyl, z.B. Benzyl oder Diphenylmethyl, darstellt, und Am Amino ist, sowie Salze, insbesondere pharmazeutisch verwendbare Salze von solchen Verbindungen der Formel I, die salzbildende Gruppen aufweisen.

Die Erfindung betrifft insbesondere die in den Beispielen beschriebenen Verbindungen der Formel I, sowie Salze, besonders die pharmazeutisch verwendbaren Salze von solchen Verbindungen mit salzbildenden Gruppen.

Die Verbindungen der vorliegenden Erfindung werden nach an sich bekannten Verfahren erhalten.

Verbindungen der Formel I und Salze von solchen Verbindungen, die salzbildende Gruppen aufweisen, werden beispielsweise hergestellt, indem man

a) eine Verbindung der Formel

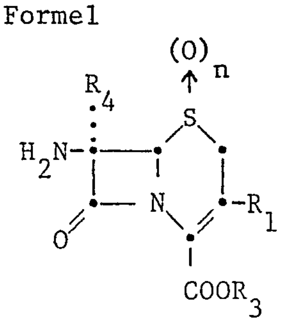

(II),

worin die 7β-Aminogruppe gegebenenfalls durch eine die Acylierungsreaktion erlaubende Gruppe substituiert ist und gegebenenfalls vorhandene funktionelle Gruppen geschützt sein können, durch Umsetzung mit einem den Acylrest einer Carbonsäure der Formel

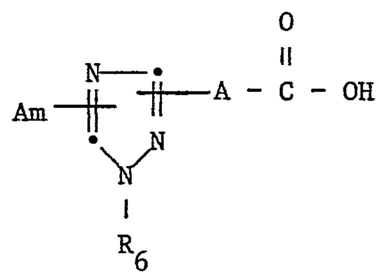

(III)

einführenden Acylierungsmittel, worin vorhandene funktionelle Gruppen gegebenenfalls geschützt sind, acyliert oder

b) zur Herstellung einer Verbindung der Formel I, worin $R_1$ den Rest der Formel $-CH_2-R_2$ darstellt, worin $R_2$ Ammonio oder Heterocyclylthio ist, eine Verbindung der Formel

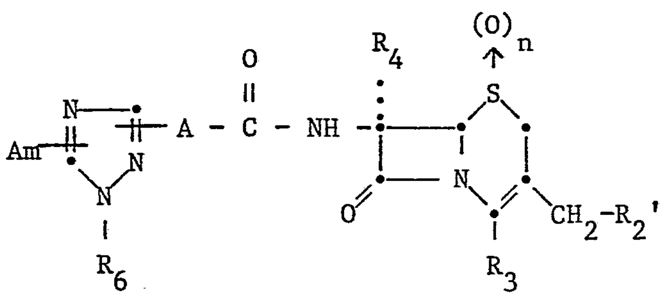

(IV),

worin $R_2'$ einen durch nucleophile Reaktion ersetzbaren Rest darstellt und worin gegebenenfalls vorhandene funktionelle Gruppen in geschützter Form vorliegen können, mit einem tertiären Amin oder einem Mercaptan der Formel $R_2$-H behandelt, oder

c) zur Herstellung einer Verbindung der Formel I, worin $R_4$ Niederalkoxy bedeutet, in eine Verbindung der Formel

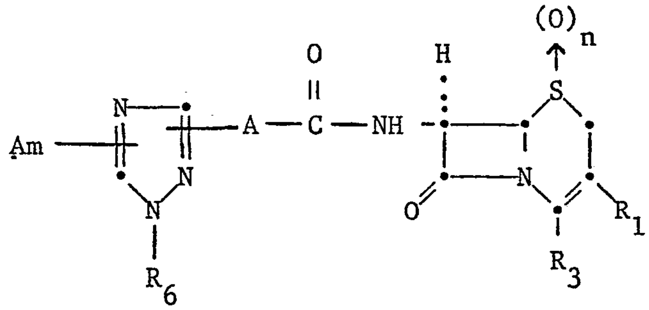 (V),

worin funktionelle Gruppen vorzugsweise geschützt sind, in 7α-Stellung eine Niederalkoxygruppe einführt, oder

d) eine 2-Cephemverbindung der Formel

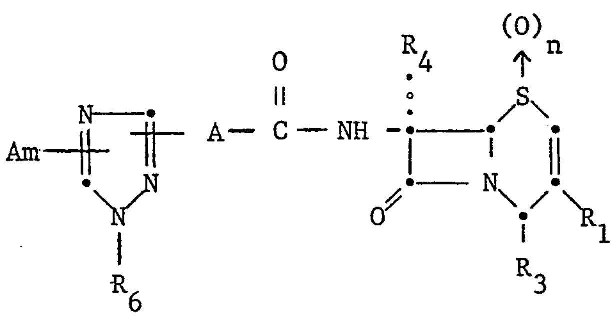 (VI),

worin gegebenenfalls vorhandene funktionelle Gruppen in geschützter Form vorliegen können, zur entsprechenden 3-Cephemverbindung isomerisiert, oder

e) zur Herstellung einer Verbindung der Formel I, worin A eine Gruppe $-C(=N-O-R_5)-$ ist, eine Verbindung der Formel

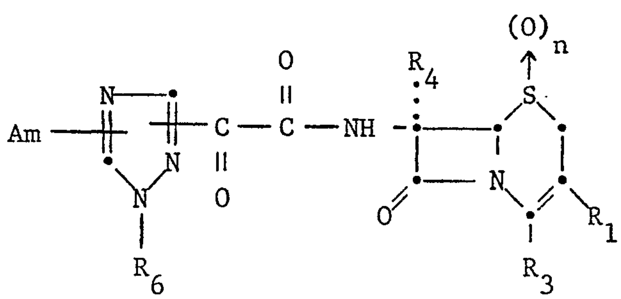 (VII),

worin gegebenenfalls vorhandene funktionelle Gruppen in geschützter Form vorliegen können, mit einem Hydroxylamin der Formel $H_2N-O-R_5$ umsetzt, oder

f) zur Herstellung einer Verbindung der Formel I, worin A eine Gruppe $-C(=N-O-R_5)-$ ist, worin $R_5$ gegebenenfalls substituiertes Niederalkyl oder Cycloalkyl, Cycloalkenyl, Niederalkenyl oder gegebenenfalls N-substituiertes Carbamoyl bedeutet, eine Verbindung der Formel

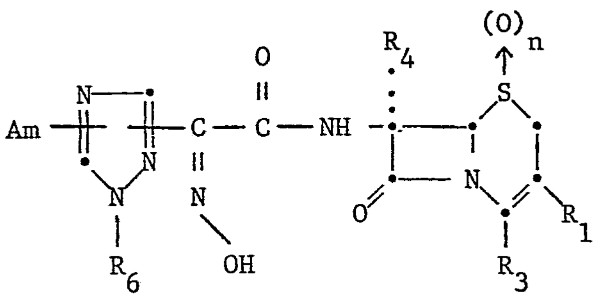

(VIII),

worin gegebenenfalls vorhandene funktionelle Gruppen in geschützter Form vorliegen können, mit einem den Rest $R_5$ einführenden Mittel behandelt, oder

g) zur Herstellung einer Verbindung der Formel I, worin A eine Gruppe $-C(=N-O-R_5)-$ ist, worin $R_5$ Wasserstoff bedeutet, eine Verbindung der Formel

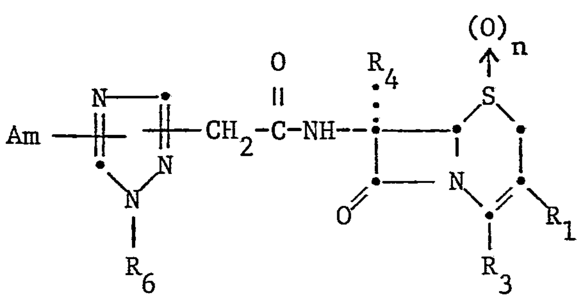

(IX) ,

worin gegebenenfalls vorhandene funktionelle Gruppen in geschützter Form vorliegen können, mit einem Nitrosierungsmittel behandelt, oder

h) zur Herstellung einer Verbindung der Formel I, worin $R_3$ eine geschützte Carboxylgruppe darstellt, in einer Verbindung der Formel

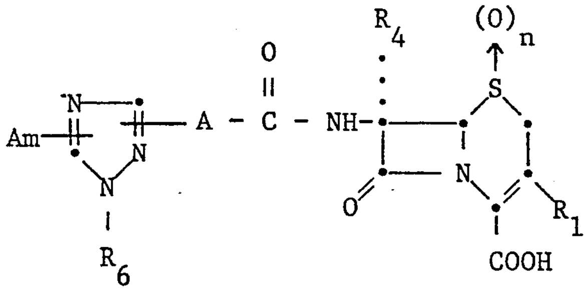

(X ),

worin gegebenenfalls vorhandene funktionelle Gruppen in geschützter Form vorliegen können, die 4-Carboxylgruppe in eine geschützte Carboxylgruppe überführt, oder

i) zur Herstellung einer Verbindung der Formel I, worin A für eine Gruppe $-CH_2-$ und Am für Amino steht, eine Verbindung der Formel

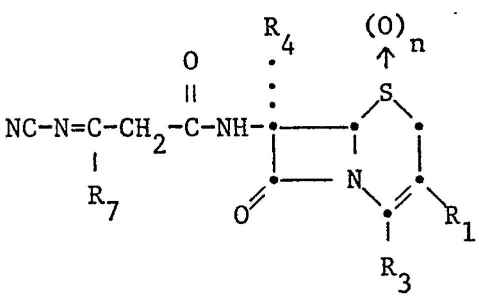

(XII) ,

worin $R_7$ für veräthertes Hydroxy steht und worin gegebenenfalls vorhandene funktionelle Gruppen in Resten $R_1$ und/oder $R_3$ in geschützer Form vorliegen können, mit einem Hydrazin der Formel $R_6$-NH-$NH_2$ (XI) umsetzt, oder

j) zur Herstellung einer Verbindung der Formel I, worin $R_6$ einen gegebenenfalls substituierten Kohlenwasserstoffrest, organisches Sulfonyl oder Acyl darstellt, in eine Verbindung der Formel

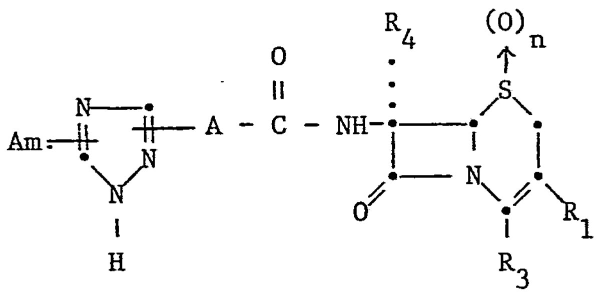

(XIII) ,

worin funktionelle Gruppen erforderlichenfalls in geschützter Form vorliegen, den Rest $R_6$ einführt, und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin n 0 bedeutet, in eine Verbindung der Formel I überführt, worin n 1 bedeutet, oder eine Verbindung der Formel I, worin n 1 bedeutet, in eine Verbindung der Formel I überführt, worin n 0 bedeutet, und/oder in einer Verbindung der Formel I in geschützter Form vorliegende funktionelle Gruppen in die freien funktionellen Gruppen überführt und/oder ein erhältliches Salz in die freie Verbindung (I) oder in ein anderes Salz überführt, und/oder eine erhältliche freie Verbindung (I) mit einer salzbildenden Gruppe in ein Salz überführt und/oder ein erhältliches Gemisch von isomeren Verbindungen der Formel I in die einzelnen Isomeren auftrennt.

Verfahren a) (Acylierung):

In einem Ausgangsmaterial der Formel II ist die 7β-Aminogruppe gegebenenfalls durch eine die Acylierung erlaubende Gruppe substituiert. Solche Gruppen sind beispielsweise organische Silyl- oder Stannylgruppen, ferner Ylidengruppen, die zusammen mit der Aminogruppe eine Schiff'sche Base bilden. Die genannten organischen Silyl- oder Stannylgruppen sind z.B. solche Gruppen, die auch mit einer Carboxylgruppe $R_3$ eine geschützte Carboxylgruppe zu bilden vermögen. Es sind dies in erster Linie Triniederalkylsilyl-, insbesondere Trimethylsilylgruppen.

Bei der Silylierung oder Stannylierung zum Schutz einer 4-Carboxylgruppe in einem Ausgangsmaterial der Formel II kann bei Verwendung eines Ueberschusses des Silylierungs- oder Stannylierungsmittels die 7β-Aminogruppe ebenfalls silyliert oder stannyliert werden. Die genannten Ylidengruppen sind in erster Linie 1-Aryl-niederalkyliden-, insbesondere 1-Arylmethylengruppen, worin Aryl besonders für einen carbocyclischen, in erster Linie monocyclischen Arylrest steht, z.B. für gegebenenfalls durch Niederalkyl, Hydroxy, Niederalkoxy und/oder Nitro substituiertes Phenyl.

Weitere in einem Ausgangsmaterial der Formel II vorhandene funktionelle Gruppen können z.B. durch die obengenannten Schutzgruppen geschützt sein. Vorzugsweise sind die an der Acylierungsreaktion nicht teilnehmenden funktionellen Gruppen, insbesondere gegebenenfalls vorhandene acylierbare Amino- und Hydroxygruppen, entsprechend geschützt.

In einem Acylrest der Säure der Formel III können vorhandene funktionelle Gruppen ausser der an der Reaktion teilnehmenden Carboxylgruppe in geschützter Form vorliegen. Eine freie Aminogruppe Am am Triazol-Ring kann auch in ionischer Form geschützt sein, d.h. das Ausgangsmaterial der Formel III kann in Form eines Säureadditionssalzes,

vorzugsweise mit einer starken anorganischen Säure, wie einer Halogenwasserstoffsäure, z.B. Salzsäure, oder Schwefelsäure, verwendet werden.

Acylierungsmittel, welche den Acylrest einer Carbonsäure der Formel III einführen, sind beispielsweise die Carbonsäure selbst oder ihre reaktionsfähigen funktionellen Derivate.

Falls eine freie Carbonsäure der Formel III zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, durchgeführt.

Zur Acylierung kann auch z.B. analog DT-OS 2.927.536, ein Salz einer Säure der Formel III, z.B. ein Alkalimetall-, wie Natriumsalz, oder ein tertiäres Aminsalz, z.B. ein Triäthylaminsalz, in Gegenwart eines am Phosphoratom trichlorierten Bis-(3-oxo-oxazolidin-1-yl)-phosphorans, worin der Oxazolidin-3-onring gegebenenfalls durch Niederalkylgruppen substituiert sein kann, eingesetzt werden.

Ein reaktionsfähiges funktionelles Derivat einer Carbonsäure der Formel III, worin vorhandene funktionelle Gruppen ausser der an der Reaktion beteiligten Carboxylgruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren wie Halogenwasserstoffsäuren, d.h. die entsprechenden Säurehalogenide, z.B. -chloride oder -bromide, ferner mit Stickstoffwassersäure, d.h. die entsprechenden Säureazide, mit

einer phosphorhaltigen Säure, z.B. Phosphorsäure, Diäthylphosphorsäure oder phosphoriger Säure, oder mit einer schwefelhaltigen Säure, z.B. Schwefelsäure,oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere zur Reaktion mit der Aminogruppe geeignete Derivate einer Säure der Formel III sind aktivierte Ester, beispielsweise Ester mit vinylogen Alkoholen, d.h. mit Enolen wie vinylogen Niederalkenolen oder Iminomethylesterhalogeniden, z.B. Dimethyliminomethylesterchlorid, hergestellt aus der Carbonsäure der Formel III und z.B. Dimethyl-(1-chlor-äthyliden)-iminium-chlorid der Formel $(CH_3)_2N^{\oplus}=C(Cl)CH_3Cl^{\ominus}$, das man seinerseits z.B. aus N,N-Dimethylacetamid und Phosgen erhalten kann, Arylester, z.B. durch Halogen, wie Chlor,und/oder Nitro, substituierte Phenylester, z.B. 4-Nitro-phenyl-, 2,4-Dinitrophenyl- oder Pentachlorphenylester, N-heteroaromatische Ester, wie N-Benztriazol-, z.B. 1-Benztriazolester, oder N-Diacylaminoester, wie N-Succinylimino- oder N-Phthalyliminoester.

Die Acylierung mit einem reaktionsfähigen funktionellen Derivat einer Carbonsäure der Formel III, z.B. einem entsprechenden Anhydrid, insbesondere einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B.

Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines 1,2-Niederalkylenoxids, wie Aethylenoxid oder Propylenoxid, durchgeführt.

Die obigen Acylierungen werden vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, einem Aether, z.B. Tetrahydrofuran, oder einem Nitril, z.B. Acetonitril, oder einer Mischung davon, wenn notwendig oder erwünscht, bei erniedrigter oder erhöhter Temperatur, z.B. in einem Temperaturbereich von etwa -40°C bis etwa +100°C, bevorzugt von etwa -10°C bis etwa +50°C, und/oder in einer Inertgas-, z.B. Stickstoffatmosphäre.

Die Acylierung kann auch durch Verwendung eines geeigneten Derivates der Säure der Formel III in Gegenwart einer geeigneten Acylase erfolgen. Solche Acylasen sind bekannt und können durch eine Anzahl von Mikroorganismen gebildet werden, z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung werden insbesondere Amide, Ester oder Thioester, wie Niederalkyl-, z.B. Methyl- oder Aethylester, der Carbonsäure der Formel III als entsprechende Derivate eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden

Medium, z.B. in einem Temperaturbereich von etwa +20°C bis etwa +40°C, bevorzugt bei etwa +37°C, durchgeführt.

Ein in der Acylierungsreaktion eingesetztes reaktionsfähiges funktionelles Derivat einer Säure der Formel III kann, wenn erwünscht, in situ gebildet werden. So kann man z.B. ein gemischtes Anhydrid herstellen, indem man eine Säure der Formel III, gegebenenfalls mit entsprechend geschützten funktionellen Gruppen, oder ein geeignetes Salz davon, z.B. ein Ammoniumsalz, welches z.B. mit einer organischen Base, wie Pyridin oder 4-Methylmorpholin gebildet wird, oder ein Metall-, z.B. Alkalimetallsalz, mit einem geeigneten Säurederivat, beispielsweise einem entsprechenden Säurehalogenid, einer gegebenenfalls substituierten Niederalkancarbonsäure, z.B. Trichloracetylchlorid, einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung in der Acylierungsreaktion verwenden.

Verfahren b) (Einführung einer Heterocyclylthio- oder Ammoniogruppe)

In einem Ausgangsmaterial der Formel IV, worin die 4-Carboxylgruppe sowie weitere funktionelle Gruppen gegebenenfalls geschützt sind, stellt der durch nucleophile Reaktion ersetzbare Rest $R_2'$ vorzugsweise eine veresterte Hydroxygruppe dar, insbesondere eine durch eine gegebenenfalls durch Oxo substituierte Niederalkancarbonsäure, z.B. Acetessig-, Ameisen- oder Essigsäure, Niederalkan- oder gegebenenfalls substituierte Benzolsulfonsäure, z.B. Methan-, Benzol-, p-Brombenzol- oder p-Toluolsulfonsäure, oder Halogenwasserstoffsäure, z.B. Brom- oder Jodwasserstoffsäure, veresterte Hydroxygruppe. Solche Reste $R_2'$ sind insbesondere Acetyloxy, Formyloxy, Methansulfonyloxy, p-Toluolsulfonyloxy oder Brom.

Die Reaktion mit einem tertiären Amin bzw. einem Mercaptan der Formel $R_2$-H kann unter neutralen Bedingungen in Gegenwart von Wasser und einem gegebenenfalls mit Wasser mischbaren organischen Lösungsmittel durchgeführt werden. Als mit Wasser mischbare organische Lösungsmittel können z.B. Niederalkanole, z.B. Methanol oder Aethanol, Niederalkanone, z.B. Aceton, Niederalkancarbonsäureamide, z.B. Dimethylformamid, oder Niederalkancarbonsäurenitrile, z.B. Acetonitril, verwendet werden. Ferner können, z.B. zur Erhöhung der Ausbeuten, dem Reaktionsgemisch geeignete Salze, wie Alkalimetall-, z.B. Natrium- und insbesondere Kaliumsalze, von anorganischen Säuren, wie Halogenwasserstoffsäure, z.B. Chlorwasserstoff- und insbesondere Jodwasserstoffsäure, sowie der Thiocyansäure, oder von organischen Säuren, wie Niederalkancarbonsäuren, z.B. Essigsäure, insbesondere Kaliumjodid und Kaliumthiocyanat, zugesetzt werden. Auch Salze von geeigneten Anionenaustauschern mit Säuren, z.B. Essigsäure, z.B. flüssige Ionenaustauscher in Salzform, z.B. Amberlite LA-1 (flüssige sekundäre Amine mit einem Molekulargewicht von 351-393; Oel-löslich und Wasser-unlöslich; mAeq./g = 2,5-2,7, z.B. in Acetatform) können für diesen Zweck verwendet werden. Man kann aber auch, insbesondere bei der Reaktion mit Mercaptan der Formel $R_2$-H, unter schwach basischen Bedingungen oder, insbesondere bei der Reaktion mit tertiären Aminen, unter schwach sauren Bedingungen, z.B. bei einem pH von etwa 6,5, arbeiten. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, und die schwachsauren Bedingungen durch Zugabe einer geeigneten organischen oder anorganischen Säure, beispielsweise Essigsäure, Chlorwasserstoffsäure, Phosphorsäure oder auch Schwefelsäure eingestellt werden. Die Reaktion wird bei Raumtemperatur oder, je nach Reaktionsfähigkeit, unter Kühlen oder leichtem Erwärmen durchgeführt.

Ammoniogruppen $R_2$ können vorteilhaft auch unter Verwendung eines Zwischenprodukts der Formel IV, in welchem $R_2'$ für eine substituierte, insbesondere für eine aromatische substituierte Carbonylthiogruppe und in erster Linie für die Benzoylthiogruppe steht, eingeführt werden. Ein solches Zwischenprodukt kann man z.B. durch Umsetzen eines Ausgangsmaterials der Formel IV, worin $R_2'$ eine veresterte Hydroxygruppe, insbesondere Niederalkanoyloxy, z.B. Acetyloxy, bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten. Das Zwischenprodukt wird dann mit der tertiären Aminbase, insbesondere einer entsprechenden heterocyclischen Base, wie einem gegebenenfalls substituierten Pyridin, umgesetzt, wobei man die gewünschte Ammonioverbindung erhält. Die Reaktion wird üblicherweise in Gegenwart eines geeigneten Entschwefelungsmittels, insbesondere eines Quecksilbersalzes, z.B. Quecksilber-II-perchlorat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatmosphäre, durchgeführt.

Verfahren c) (Alkoxylierung der 7α-Stellung):

In einer Verbindung der Formel V, worin alle funktionellen Gruppen in geschützter Form vorliegen, kann die 7α-Niederalkoxygruppe $R_4$ auf an sich bekannte Weise eingeführt werden, beispielsweise indem man das genannte Zwischenprodukt nacheinander mit einem Anion-bildenden Mittel, einem N-Halogenierungsmittel und einem Niederalkanol behandelt.

Ein geeignetes Anion-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethanolat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0°C bis etwa -80°C, und in Gegenwart eines geeigneten Lösungs- oder Verdünnungsmittels, z.B. eines Aethers, wie Tetrahydrofuran, bei Verwendung von Lithiummethanolat auch in Gegenwart von Methanol, und, wenn erwünscht, in einem geschlossenen Gefäss und/oder in einer Inertgas-, z.B. Stickstoffatmosphäre, vorgenommen.

Als N-halogenierendes Mittel verwendet man üblicherweise ein sterisch gehindertes, organisches Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethanolat, unter den Reaktionsbedingungen und ohne isoliert zu werden in eine 7-Acyliminocephemverbindung umgewandelt, und diese durch Zugabe des Niederalkanols in eine 7α-Niederalkoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter Zugabe einer Halogenwasserstoff-abspaltenden Base, wie eines geeigneten Alkalimetall-niederalkanolats, z.B. Lithium-tert.butanolat,wobei diese Reaktion üblicherweise unter den Bedingungen der Anion- und N-Halogenverbindung-bildenden Reaktion stattfindet, wobei man in Gegenwart von Methanol arbeiten und anstelle der Acyliminoverbindung direkt die 7α-Niederalkoxy-cephem-Verbindung erhalten kann. Man geht üblicherweise aus von einer Verbindung der Formel V, worin funktionelle Gruppen in geschützter Form vorliegen, setzt diese mit einem

Ueberschuss des Anion-bildenden Mittels, z.B. Lithiummethanolat oder Phenyllithium, in Gegenwart des Niederalkanols um, behandelt dann mit dem N-Halogenierungsmittel, z.B. tert.-Butylhypochlorit, und erhält so direkt die gewünschte Verbindung der Formel I, worin funktionelle Gruppen geschützt sind. Man kann auch das Niederalkanol nachträglich zugeben, wobei man die Dehydrohalogenierung und die Zugabe des Niederalkanols bei etwas höheren Temperaturen als die Anion- und N-Halogenverbindung-bildenden Reaktionen, z.B. bei etwa 0°C bis etwa -20°C, wenn notwendig, in einem geschlossenen Gefäss und/oder in einer Inertgas-, z.B. Stickstoffatmosphäre, durchführen kann.

Bei vorstehenden Reaktionen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Verfahren d) (Isomerisierung):

In einem 2-Cephem-Ausgangsmaterial der Formel VI weist die gegebenenfalls geschützte 4-Carboxylgruppe vorzugsweise die α-Konfiguration auf.

2-Cephem-Verbindungen der Formel VI können isomerisiert werden, indem man sie mit einem schwach-basischen Mittel behandelt und die entsprechende 3-Cephem-Verbindung isoliert. Geeignete Isomerisierungsmittel sind z.B. organische stickstoffhaltige Basen, insbesondere tertiäre heterocyclische Basen aromatischen Charakters, in erster Linie Basen des Pyridin-Typs, wie Pyridin, sowie Picoline, Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie Triniederalkylamine, z.B.

Trimethylamin oder Diisopropyläthylamin, N-Niederalkyl-azacycloalkane, z.B. N-Methyl-piperidin, oder N-Phenylniederalkyl-N,N-diniederalkylamine, z.B. N-Benzyl-N,N-dimethylamin, sowie Gemische von solchen basischen Mitteln, wie das Gemisch einer Base vom Pyridintyp und eines Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall- oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei der Isomerisierung von 2-Cephem-Verbindungen der Formel VI mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstoffatmosphäre, und/oder in einem geschlossenen Gefäss.

So erhältliche 3-Cephem-Verbindungen der Formel I lassen sich in an sich bekannter Weise, z.B. durch Adsorption und/oder Kristallisation, von gegebenenfalls noch vorhandenen 2-Cephem-Ausgangsstoffen abtrennen.

Die Isomerisierung von 2-Cephem-verbindungen der Formel VI zu entsprechenden 3-Cephem-verbindungen wird vorzugsweise durchgeführt, indem man diese in 1-Stellung oxidiert, wenn erwünscht, gegebenenfalls Isomerengemische der gebildeten 1-Oxide trennt, und die so

erhältlichen 1-Oxide der entsprechenden 3-Cephemverbindungen reduziert.

Als geeignete Oxidationsmittel für die Oxidation in 1-Stellung von 2-Cephem-verbindungen der Formel VI kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure anzuwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B.

Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber verwenden kann. Die Oxidation wird unter milden Bedingungen, z.B. bei Temperaturen von etwa -50°C bis etwa +100°C, vorzugsweise von etwa -10°C bis etwa -40°C, durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanidanionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphonigen, phosphinigen oder phosphorigen Säure, sowie diesen Phosphorsauerstoffverbindungen entsprechende Phosphor-Schwefelverbindungen, worin organische Reste in erster Linie aliphatische, aromatische oder araliphatische Reste, z.B. gegebenenfalls substituiertes

Niederalkyl, Phenyl oder Phenylniederalkyl darstellen, wie z.B. Triphenylphosphin, Tri-n-butylphosphin, Diphenylphosphonigsäuremethylester, Diphenylchlorphosphin, Phenyldichlorphosphin, Benzolphosphonigsäuredimethylester, Butanphosphonigsäuremethylester, Phosphorsäuretriphenylester, Phosphorigsäuretrimethylester, Phosphorigtrichlorid, Phosphorigtribromid, etc; reduzierende Halogensilanverbindungen, die mindestens ein an das Siliciumatom gebundendes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, wie Chlorsilan, Bromsilan, Di- oder Trichlorsilan, Di- oder Tribromsilan, Diphenylchlorsilan, oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefel enthaltende Silane, wie 1,3-Dithia-2,4-disilacyclobutane oder 1,3,5-Trithia-2,4,6-trisila-cyclohexane, deren Siliciumatome durch Kohlenwasserstoffreste, wie insbesondere Niederalkyl, substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylen-pyrrolidiniumchlorid; oder komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obengenannten Reduktionsmittel verwendet werden, welche selber nicht Lewissäure-Eigenschaften aufweisen, d.h. in erster Linie zusammen mit Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, Propansulton, 1,3-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

Verfahren e) (Bildung der gegebenenfalls substituierten Hydroxyiminogruppe):

In einem Ausgangsmaterial der Formel VII, worin die 4-Carboxylgruppe sowie weitere funktionelle Gruppen gegebenenfalls geschützt sind, kann die nicht zur Amidgruppierung gehörende Oxogruppe in der 7β-Acylamidogruppe durch Behandeln mit einem Hydroxylaminderivat der Formel $H_2N-O-R_5$ in eine gegebenenfalls O-substituierte Hydroxyiminogruppe übergeführt werden. Die Reaktion eines Ausgangsmaterials der Formel VII mit dem Hydroxylaminderivat wird in üblicher Weise durchgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, z.B. in einem Temperaturbereich von etwa -20°C bis etwa +100°C, bevorzugt von etwa 0°C bis etwa 30°C. gegebenenfalls in einer Inertgas-, wie Stickstoffatmosphäre, umsetzt. Das Hydroxylaminderivat kann auch in situ, z.B. aus einem ihrer Salze, wie einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triäthylamin oder Aethyldiisopropylamin, oder einer heterocyclischen tertiären Base, wie Pyridin, in Freiheit gesetzt werden.

Verfahren f) (Verätherung bzw. Carbamoylierung der Hydroxyiminogruppe)

In dem Ausgangsmaterial der Formel VIII sind verätherbare bzw. carbamoylierbare Gruppen, ausser der reagierenden Hydroxyiminogruppe, bevorzugt geschützt.

Die Verätherung der freien Hydroxygruppe wird mit einem den gewünschten, gegebenenfalls substituierten Niederalkyl-, Cycloalkyl-, Cycloalkenyl- oder Niederalkenylrest $R_5$ einführenden konventionellen Verätherungsmittel durchgeführt.

Geeignete Verätherungsmittel sind beispielsweise Diazoverbindungen, wie Diazoniederalkane oder -cycloalkane, z.B. Diazomethan, Diazoäthan oder Diazo-n-butan, die gegebenenfalls im Alkanteil substituiert sein können. Diese Reagentien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid. oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und je nach Diazoreagens unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure oder Halogen-schwefelsäure, z.B. Fluorschwefelsäure, oder starken organischen Sulfonsäuren, wie gegebenenfalls z.B. durch Halogen, wie Fluor, substituierten Niederalkansulfonsäuren, oder aromatischen Sulfonsäuren, wie z.B. gegebenenfalls, z.B. durch Niederalkyl, wie Methyl, Halogen, wie Brom, oder Nitro substituierten Benzolsulfonsäuren, z.B. Methansulfon-, Trifluormethansulfon- oder p-Toluolsulfonsäure, z.B. Diniederalkylsulfate, wie Dimethylsulfat, ferner Fluorsulfonsäureniederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten aliphatischen, cycloaliphatischen oder aromatischen Kohlenwasserstoffs, z.B. Methylenchlorid, eines Aethers, wie Dioxan oder Tetrahydrofuran, oder eines

Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-äthyl-amin (vorzugsweise zusammen mit Niederalkyl-halogensulfaten oder gegebenenfalls Halogen-substituierten Methansulfonsäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre gearbeitet wird.

Weitere Verätherungsmittel sind entsprechende Acetale, z.B. gem-Niederalkoxyniederalkane, wie 2,2-Dimethoxy-propan, die in Gegenwart einer starken organischen Sulfonsäure, wie p-Toluolsulfonsäure, und eines geeigneten Lösungsmittels, wie eines Diniederalkyl- oder Niederalkylensulfoxids, z.B. Dimethylsulfoxid, zur Anwendung gelangen, oder Orthoester, z.B. Orthoameisensäure-triniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende Tri-$R_5$-oxoniumsalze (sogenannte Meerweinsalze), Di-$R_5$-O-carbeniumsalze oder Di-$R_5$-haloniumsalze, worin $R_5$ den einzuführenden Rest darstellt, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxoniumhexafluorantimonat, -hexachlorantimonat, -hexafluorphosphat oder -tetrafluorborat, Dimethoxycarbeniumhexafluorphosphat oder Dimethylbromonium-hexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-$R_5$-3-Aryltriazenverbindungen, worin $R_5$ den einzuführenden Rest und Aryl vorzugsweise einen gegebenenfalls substituierten Phenylrest, z.B. Niederalkylphenyl, wie 4-Methylphenyl, bedeutet. Solche Triazenverbindungen sind z.B. 3-Aryl-1-niederalkyl-triazene, wie 3-(4-Methylphenyl)-1-methyl-triazen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

Die Einführung einer gegebenenfalls substituierten Carbamoylgruppe anstelle des Wasserstoffatoms geschieht auf an sich bekannte Weise durch Carbamoylierung. Zu diesem Zwecke wird eine Verbindung der Formel VIII, worin alle reaktionsfähigen Gruppen, die nicht carbamoyliert werden sollen oder die die Reagentien verbrauchen und zerstören würden, in geschützter Form vorliegen, gegebenenfalls stufenweise mit einem Carbamoylierungsmittel behandelt. Geeignete Carbamoylierungsmittel sind reaktionsfähige Derivate von gegebenenfalls substituierten Carbamidsäuren, insbesondere die entsprechenden Isocyanate oder die gemischten Anhydride, z.B. die entsprechenden

Carbamidsäurechloride, die auch in situ aus Phosgen und einem primären Amin hergestellt werden können.

Der Umsatz mit den Isocyanaten erfolgt in einem der üblichen inerten Lösungsmittel, beispielsweise einem gegebenenfalls halogenierten Kohlenwasserstoff, z.B. Benzol, Xylol oder Methylenchlorid, einem Aether, z.B. Diäthyläther, Dioxan oder Tetrahydrofuran, oder einem Ester, z.B. Essigsäureäthylester, gegebenenfalls in Gegenwart eines basischen Katalysators, beispielsweise eines tertiären Amins, z.B. Triäthylamin oder Pyridin, bei Raumtemperatur oder etwas erniedrigter oder erhöhter Temperatur, etwa zwischen -20°C und +50°C.

Zum Umsatz mit einem gemischten Anhydrid der Carbamidsäure wird die Alkoholgruppe mit Vorteil zunächst metalliert, beispielsweise mit einem Alkalimetallhydrid, z.B. Natrium- oder Lithiumhydrid. Die an der Hydroxygruppe metallierte Verbindung der Formel VIII kann auch zunächst mit Phosgen und anschliessend mit Ammoniak oder einem primären Amin behandelt werden und auf diese Art stufenweise carbamoyliert werden. Diese Reaktionen finden ebenfalls in einem inerten Lösungsmittel und bei den oben angegebenen Temperaturen statt.

Verfahren g) (Bildung der unsubstituierten Hydroxyiminogruppe):

In einem Ausgangsmaterial der Formel IX sind die funktionellen Gruppen vorzugsweise geschützt. Geeignete Nitrosierungsmittel sind salpetrige Säure und davon abgeleitete Derivate, wie Nitrosylhalogenide, z.B. Nitrosylchlorid oder Nitrosylbromid, Salze der salpetrigen Säure, wie Alkalimetallsalze, z.B. Natrium- oder Kaliumnitrit, oder insbesondere Ester der salpetrigen Säure, wie entsprechende Niederalkylester, z.B. Butyl-, Pentyl- oder insbesondere Isoamylnitrit. Verwendet man als Nitrosierungsmittel ein Salz der salpetrigen Säure, so wird die Reaktion vorzugsweise in Gegenwart einer starken anorganischen oder organischen Säure, z.B. Chlorwasserstoffsäure, Schwefelsäure, Ameisensäure oder Essigsäure, durchgeführt. Bei Verwendung eines Esters der salpetrigen Säure wird die Reaktion vorzugsweise in Gegenwart einer starken Base, wie eines Alkalimetallalkanolates, durchgeführt.

Die Nitrosierung wird in einem geeigneten Lösungsmittel, wie Wasser, einer Carbonsäure, z.B. Essigsäure, einem Niederalkanol, z.B. Methanol oder Aethanol, einem Aether, z.B. Dioxan oder Tetrahydrofuran, oder einem Kohlenwasserstoff, z.B. Hexan oder Benzol, oder in Mischungen davon, wenn notwendig unter Kühlen oder Erwärmen, insbesondere in einem Temperaturbereich von etwa -15°C bis etwa Raumtemperatur, und/oder in einer Inertgasatmosphäre durchgeführt.

Verfahren h) (Ueberführung der freien in die geschützte Carboxylgruppe $R_3$):

Die Ueberführung der freien Carboxylgruppe in einer Verbindung der Formel X in eine geschützte, insbesondere in eine veresterte Carboxylgruppe, die unter schonenden chemischen Bedingungen oder unter physiologischen Bedingungen leicht spaltbar ist, erfolgt nach an sich bekannten Methoden, beispielsweise indem man eine Verbindung der Formel X , worin andere funktionelle Gruppen, wie Amino-, Hydroxy- oder Sulfogruppen, gegebenenfalls in geschützter Form vorliegen, oder ein bezüglich der zu schützenden Carboxylgruppe reaktionsfähiges funktionelles Derivat davon oder ein Salz davon, mit einem entsprechenden Alkohol oder einem reaktionsfähigen funktionellen Derivat davon, verestert.

Geeignete reaktionsfähige funktionelle Derivate der zu schützenden Carboxylgruppe sind Anhydride, insbesondere gemischte Anhydride, und aktivierte Ester.

Die Veresterung der freien Carboxylgruppe mit dem gewünschten Alkohol wird in Gegenwart eines Kondensationsmittels durchgeführt.

Uebliche Kondensationsmittel sind Carbodiimide, beispielsweise N,N'-Diäthyl, N,N'-Dipropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazolinium-3'-sulfonat und N-tert.-Butyl-5-methyl-isoxazoliumperchlorat, oder eine Acylaminoverbindung, z.B. 2-Aethoxy-1-äthoxycarbonyl-1,2-dihydrochinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Gemischte Anhydride sind z.B. diejenigen mit anorganischen Säuren, wie Halogenwasserstoffsäuren, d.h. die entsprechenden Säurehalogenide, z.B. -chloride oder-bromide, ferner mit Stickstoffwasserstoffsäure, d.h. die entsprechenden Säureazide, mit einer phosphorhaltigen Säure, z.B. Phosphorsäure, Diäthylphosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen Sulfonsäuren, z.B. p-Toluolsulfonsäure.

Zur Reaktion mit dem Alkohol geeignete aktivierte Ester sind z.B. Ester mit vinlyogen Alkoholen (d.h. Enolen), wie vinylogen Niederalkenolen, oder Iminomethylesterhalogeniden, wie Dimethyliminiomethylesterchlorid (hergestellt aus der Carbonsäure und Dimethylchlormethyliden-iminium-chlorid der Formel $[(CH_3)_2N{=}CHCl]^{\oplus}Cl^{\ominus}$ oder

Arylester, wie 4-Nitrophenyl- oder 2,4-Dinitrophenylester, heteroaromatische Ester, wie Benztriazol-, z.B. 1-Benztriazolester, oder Diacyliminoester, wie Succinylimino- oder Phthaliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei -40°C bis etwa +100°C, bevorzugt bei -10°C bis +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäure mit entsprechend geschützten funktionellen Gruppen oder eines gecigneten Salzes davon, wie eines Ammoniumsalzes, z.B. mit einem organischen Amin, wie Piperidin oder 4-Methylmorpholin, oder

eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, mit einem Halbester eines Kohlensäurehalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Verfahren i) (Bildung des 1,2,4-Triazolringes):

In einer Ausgangsverbindung der Formel XII sind funktionelle Gruppen, insbesondere freie Carboxyl-, Sulfo- und Hydroxygruppen, vorzugsweise geschützt. Veräthertes Hydroxy $R_7$ steht insbesondere für Niederalkoxy, z.B. Methoxy, Aethoxy, Propoxy oder Butoxy, ferner auch für gegebenenfalls substituiertes Aralkoxy, insbesondere Benzyloxy, z.B. Benzyloxy oder p-Methoxybenzyloxy.

Die Reaktion wird bevorzugt in einem inerten Lösungsmittel, wie einem Aether, z.B. Diäthyläther, Dioxan oder Tetrahydrofuran, einem aliphatischen oder aromatischen Kohlenwasserstoff, wie Hexan, Benzol oder Toluol, einem Nitril, wie Acetonitril, oder dergleichen oder in Mischungen davon, gegebenenfalls unter Kühlen oder Erwärmen, d.h. bei Temperaturen zwischen etwa -20° und etwa +110°C, bevorzugt zwischen etwa -10° und +20°C, und/oder in einer Inertgasatmosphäre durchgeführt.

Verfahren j) (Einführung des Restes $R_6$):

Die Einführung des Restes $R_6$ in Verbindungen der Formel XIII erfolgt beispielsweise durch Umsetzen mit Verbindungen der Formel $R_6$-X, worin X für reaktionsfähiges verestertes Hydroxy steht.

Reaktionsfähiges verestertes Hydroxy X ist beispielsweise mit Halogenwasserstoffsäuren, organischen Sulfonsäuren, wie Niederalkan- oder gegebenenfalls substituierten Benzolsulfonsäuren, Niederalkancarbonsäuren oder Phosphinsäuren verestertes Hydroxy und bedeutet in erster Linie Halogen, z.B. Chlor, Brom oder Jod, Sulfonyloxy, z.B. Methan-, Benzol-, p-Toluol- oder p-Brombenzolsulfonyloxy, Niederalkanoyloxy. z.B. Acetoxy, oder Phosphinoyloxy, z.B. Dimethyl- oder Diphenylphosphinoyloxy. Verbindungen der Formel $R_6$-X können aber auch Ester der Schwefelsäure, Phosphorsäure oder einer Phosphonsäure, z.B. Aethylphosphonsäure, sein, worin alle freien Hydroxygruppen mit einem Alkohol der Formel $R_6$-OH veräthert sind.

Weitere in den Ausgangsverbindungen der Formel XIII vorhandene funktionelle Gruppen sind vorzugsweise geschützt.

Die Einführung eines gegebenenfalls substituierten Kohlenwasserstoffrestes $R_6$, z.B. eines entsprechenden Niederalkyl- oder eines Niederalkenyl-, Cycloalkyl- oder Phenylniederalkylrestes, erfolgt mit Verbindungen der Formel $R_6$-X, worin X bevorzugt für Halogen oder Sulfonyloxy steht. Solche Verbindungen sind z.B. gegebenenfalls substituierte Niederalkylhalogenide oder -sulfonate, z.B. Methyljodid, Aethylbromid, Bromessigsäureäthylester oder 3-Brompropionsäuremethylester, Niederalkenylhalogenide, z.B. Allylbromid, Cycloalkylhalogenide oder -sulfonate, z.B. Cyclohexylbromid oder -mesylat, oder Phenylniederalkylhalogenide, z.B. Benzyl- oder Diphenylmethylbromid. Die Reaktion wird in Gegenwart eines basischen Kondensationsmittels, beispielsweise eines Alkali- oder Erdalkalimetallhydroxids, -carbonats oder -hydrogencarbonats, z.B. Natriumhydroxid, -carbonat, oder -hydrogencarbonat oder Calciumcarbonat, eines Alkalimetall-niederalkanolats, z.B. Natriummethanolat, Natriumäthanolat oder Kalium-tert.-butanolat, oder eines tertiären Amins, z.B. Triäthylamin, Diisopropyläthylamin, Pyridin oder Chinolin, in einem inerten Lösungsmittel, wie einem Niederalkanol, z.B. Methanol, Aethanol oder tert.-Butanol, einem Amid, z.B. Dimethylformamid, oder in überschüssiger Amin-Base bei Raumtemperatur oder erhöhter oder erniedrigter Temperatur, z.B. bei etwa -20° bis etwa +80°C, durchgeführt.

Zur Einführung eines gegebenenfalls substituierten Kohlenwasserstoffrestes $R_6$, insbesondere eines entsprechenden Niederalkyl- oder eines Phenylniederalkylrestes, sind weiterhin Phosphorsäuren der Formel $O{=}P(OR_6)_3$, Phosphonsäuren der Formel $O{=}P(OR_6)_2(Y)$ oder Phosphinsäuren der Formel $O{=}P(OR_6)(Y)_2$ geeignet, worin Y z.B. für Niederalkyl, z.B. Methyl oder Aethyl,oder Phenyl steht. Die Kondensation erfolgt gegebenenfalls in Anwesenheit einer Base, wie eines Triniederalkylamins, z.B. Triäthyl- oder Tri-n-butylamin, und bei erhöhter Temperatur, z.B. bei etwa +50° bis etwa +150°C, wobei man in einem inerten Lösungsmittel oder bevorzugt ohne Zugabe eines Lösungsmittels arbeitet.

Die Einführung eines organischen Sulfonyl-, Aroyl- oder Niederalkanoyl-Restes $R_6$ erfolgt in an sich bekannter Weise durch Acylierung mit einer Verbindung der Formel $R_6$-X, worin $R_6$ für organisches Sulfonyl, Aroyl oder Niederalkanoyl steht. Geeignete Acylierungsmittel sind beispielsweise reaktionsfähige funktionelle Derivate der entsprechenden Aren- bzw. Niederalkansulfonsäuren oder -carbonsäuren, wie Anhydride davon, z.B. die symmetrischen Anhydride oder die gemischten Anhydride mit Halogenwasserstoffsäuren, z.B. Säurechloride. Beispiele für solche Acylierungsmittel sind Niederalkansulfonylchloride, z.B. Methansulfonylchlorid, gegebenenfalls substituierte Benzolsulfonylchloride, z.B. Benzolsulfonylchlorid oder 4-Toluolsulfonylchlorid, Niederalkancarbonsäurechloride oder -anhydride, z.B. Acetylchlorid, Propionylchlorid oder Acetanhydrid, oder Arencarbonsäurehalogenide, z.B. Benzoylchlorid. Die Reaktion kann erforderlichenfalls in Gegenwart eines basischen Kondensationsmittels, wie eines der oben genannten, in einem inerten Lösungsmittel, wie einem aromatischen Kohlenwasserstoff, z.B. Benzol oder Toluol, einem Aether, z.B. Dioxan, Tetrahydrofuran oder Dimethoxyäthan, oder einem Amid, z.B. Dimethylformamid, bei Raumtemperatur oder erhöhter Temperatur, z.B. bei etwa 20° bis etwa 100°C, durchgeführt werden.

Durch Umsetzen von Verbindungen der Formel XIII mit Halogenameisensäureniederalkylestern, z.B. mit Chlorameisensäureäthylester, bzw. mit substituierten Carbaminsäurehalogeniden, z.B. Diäthylcarbaminsäurechlorid, kann man Verbindungen der Formel I herstellen, worin $R_6$ für Niederalkoxycarbonyl oder substituiertes Carbamoyl steht. Die Umsetzung kann unter den gleichen Reaktionsbedingungen erfolgen wie bei der Einführung eines Aroyl- oder Niederalkanoylrestes $R_6$ beschrieben.

Die Einführung einer N-mononiederalkylierten Carbamoylgruppe kann auch erfolgen, indem man eine Verbindung der Formel XIII mit einem Isocyanat der Formel $R_6'$ -NCO umsetzt, worin $R_6'$ für Niederalkyl steht. Die Reaktion wird in einem inerten Lösungsmittel, beispielsweise in einem tertiären Amin, wie Pyridin, bei Raumtemperatur oder vorzugsweise bei erhöhter Temperatur, z.B. bei Siedetemperatur, durchgeführt.

Weiterhin kann man eine unsubstituierte Carbamoylgruppe einführen, indem man eine Verbindung der Formel XIII mit Cyansäure, die vorzugsweise <u>in situ</u> aus Kalium-cyanat und einer organischen Säure, welche gleichzeitig als Lösungsmittel dienen kann, z.B. Essigsäure, erzeugt wird, bei Raumtemperatur oder erniedrigter Temperatur, z.B. bei etwa -30° bis etwa +20°C, umsetzt, oder indem man eine Verbindung der Formel XIII in wässriger Lösung in Gegenwart eines basischen Kondensationsmittels, insbesondere eines Alkalimetallhydroxids, z.B. Natriumhydroxid, mit einem Halogencyan, z.B. Bromcyan, behandelt, wobei man die Reaktionstemperatur vorzugsweise bei etwa -20° bis +20°C hält.

Man kann die Acylierung, insbesondere die Aroylierung, Niederalkanoylierung und Niederalkoxycarbonylierung, aber auch zweistufig durchführen, indem man eine, vorzugsweise <u>in situ</u> gebildete Verbindung der Formel I, worin $R_6$ für Triniederalkyl-, z.B. Trimethylsilyl, steht, bei Raumtemperatur oder etwas erniedrigter Temperatur, z.B. bei etwa -20° bis etwa +20°C, mit dem Acylierungsmittel umsetzt, wobei als

Lösungsmittel z.B. aromatische Kohlenwasserstoffe, wie Benzol oder Toluol, Verwendung finden können. Die Triniederalkylsilyl-Zwischenverbindungen werden beispielsweise erhalten, indem man eine Verbindung der Formel XIII mit einem Hexaniederalkylsilazan, z.B. Hexamethylsilazan, Triniederalkyl-aminosilan, z.B. Trimethyl-aminosilan, oder in Gegenwart einer Base, wie eines tertiären Amins, z.B. Triäthylamin, mit einem Triniederalkyl-halogensilan, z.B. Trimethylsilylchlorid, umsetzt.

Die erfindungsgemäss erhältlichen Verbindungen der Formel I können, unabhängig von ihrem Herstellungsweg, in an sich bekannter Weise in andere Verbindungen der Formel I übergeführt werden.

Bildung des 1-Oxids: Eine Verbindung der Formel I, worin n für 0 steht, kann durch Oxidation, z.B. nach einer der unter Verfahren d) beschriebenen Oxidationsmethoden, in eine Verbindung der Formel I, worin n für 1 steht, überführt werden.

Reduktion des 1-Oxids: Eine Verbindung der Formel I, worin n für 1 steht, kann durch Reduktion, z.B. nach einer der unter Verfahren d) beschriebenen Reduktionsmethoden, in eine Verbindung der Formel I, worin n für 0 steht, überführt werden.

Ueberführung einer freien in eine substituierte Aminogruppe Am: Verbindungen der Formel I, worin Am für Amino steht, können in an sich bekannter Weise in andere Verbindungen der Formel I überführt werden, worin Am substituiertes Amino, wie insbesondere Diniederalkylamino-niederalkylidenamino, bedeutet. So können entsprechende Diniederalkylamino-niederalkylidenamino-Verbindungen z.B. hergestellt werden, indem man in Aminotriazolyl-Verbindungen der Formel I die freie Aminogruppe durch Umsetzen mit einem Niederalkancarbonsäurehalogenid, z.B. Acetylchlorid, oder auch Ameisensäure in Gegenwart von Acetanhydrid, in eine Niederalkanoylamino-, z.B. Acetyl-oder Formylamino-Gruppe umwandelt, diese mit einem Halogenierungsmittel, z.B. Phosphorpentachlorid, umsetzt und das entstandene Imidoesterhalogenid mit einem Diniederalkylamin, z.B. Dimethylamin oder Diäthylamin, behandelt.

Abspaltung von Schutzgruppen: In einer erhaltenen Verbindung der Formel I, worin eine oder mehrere funktionelle Gruppen geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

Eine geschützte Carboxylgruppe, insbesondere eine geschützte Carboxylgruppe $R_3$, setzt man in an sich bekannter und je nach Art der Schutzgruppe in verschiedenartiger Weise, vorzugsweise mittels Solvolyse oder Reduktion, frei.

So kann man z.B. eine gegebenenfalls in 2-Stellung durch eine Silylgruppe oder in 1-Stellung durch Niederalkoxy oder Niederalkylthio substituierte Niederalkoxycarbonylgruppe, eine Polycycloalkoxycarbonyl- oder eine Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators, gespalten werden.

Ferner kann man bestimmte substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem

Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer Carbonsäure oder α-Hydroxycarbonsäure, wie insbesondere Essigsäure, Ameisensäure, Glycolsäure, Diphenylglycolsäure, Milchsäure, Mandelsäure, p-Chlormandelsäure, Weinsäure und dergleichen, oder eines Alkohols oder Thiols, wobei man vorzugsweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Jodniederalkoxycarbonylgruppe) oder eine Aroylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Einesubstituierte Silyläthoxycarbonylgruppe kann auch durch Behandeln mit einem Salz der Fluorwasserstoffsäure, das Fluoridanion liefert, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraalkylammoniumfluorid oder Trialkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in die freie Carboxylgruppe übergeführt werden. Eine Polyhalogenaryloxycarbonylgruppe, wie die Pentachlorphenyloxycarbonylgruppe, wird unter milden basischen Bedingungen, wie durch verdünnte Natronlauge oder organische Basen in Gegenwart von Wasser, zur freien Carboxylgruppe verseift.

Eine z.B. durch Silylierung oder Stannylierung geschützte Carboxylgruppe kann in üblicher Weise, z.B. durch Behandeln mit Wasser oder einem Alkohol, freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink, in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitrobenzyloxycarbonylamino auch durch Behandeln mit einem Alkalimetall-, z.B. Natriumdithionat, gespalten werden. Gegebenenfalls substituiertes Diphenylmethoxycarbonylamino, tert.-Niederalkoxycarbonylamino oder 2-trisubstituiertes Silyloxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 1-Acyl-niederalk-1-en-2-yl-amino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base oder mit einem Thiolatsalz, wie einem Alkalimetallthiolat, des Thioharnstoffs und anschliessende Solvolyse, wie Alkoholyse oder Hydrolyse, des entstandenen Kondensationsproduktes freigesetzt werden. Eine durch 2-substituiertes Silyläthoxycarbonyl geschützte Aminogruppe kann auch durch Behandeln mit einem Fluoridanion liefernden

Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat, oder Monomethylphosphonsäure oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20° bis 25°C, oder auch unter Kühlen oder Erwärmen, durchgeführt.

Eine durch eine geeignete Acylgruppe, eine organische Silyl- oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse gespalten, während eine durch tert.-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt wird.

Eine geschützte, insbesondere veresterte Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle-Katalysator.

Salzbildung: Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionaustauschreagens. Innere Salze von Verbindunden der Formel I, welche z.B. eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

<u>Ausgangsstoffe</u>: Die im Verfahren zur Herstellung der Verbindungen der vorliegenden Erfindung verwendeten Ausgangsstoffe sind bekannt oder, falls neu, können in an sich bekannter Weise hergestellt werden.

Die Ausgangsverbindungen der Formel II sowie entsprechende Verbindungen mit geschützten funktionellen Gruppen sind bekannt.

Mit Ausnahme der 5-Amino-1,2,4-triazol-3-ylessigsäure (Russ. Patent 320496) sind Ausgangsverbindungen der Formel III bisher nicht bekannt geworden. Neue Verbindungen der Formel III können in an sich bekannter Weise hergestellt werden. Die Herstellungsweise wird anhand des folgenden Reaktionsschemas illustriert:

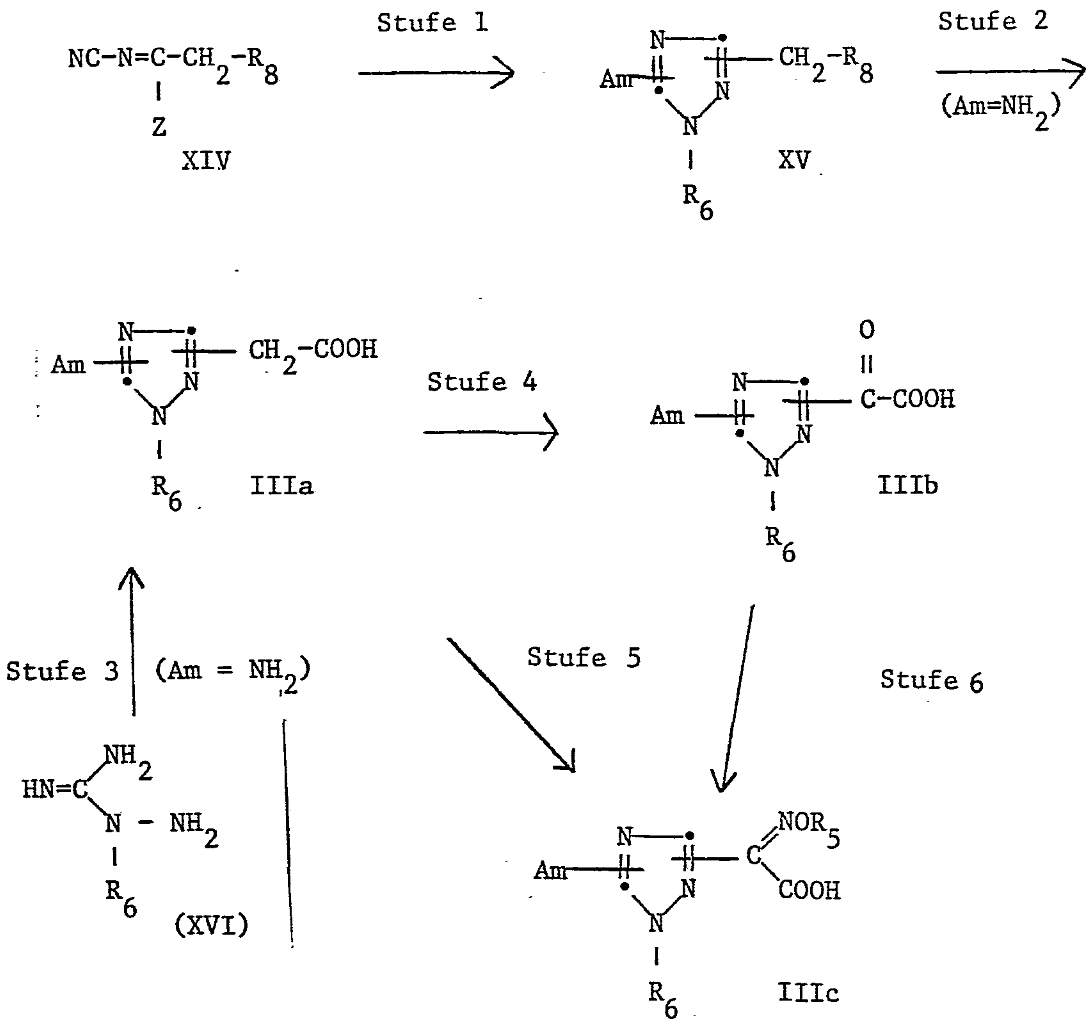


<u>Stufe 1:</u> In Verbindungen der Formel XIV steht Z für veräthertes Hydroxy, insbesondere Niederalkoxy, z.B. Methoxy oder Aethoxy, ferner auch für gegebenenfalls substituiertes Aralkoxy, z.B. Benzyloxy oder p-Methoxybenzyloxy, und $R_8$ für Wasserstoff, Cyano oder eine geschützte Carboxygruppe. Eine geschützte Carboxygruppe ist insbesondere eine unter schonenden chemischen Bedingungen leicht abspaltbare veresterte Carboxygruppe. Geeignete Schutzgruppen sind beispielsweise die bei einer geschützten Carboxygruppe $R_3$ aufgeführten, insbesondere die gegebenenfalls substituierten Niederalkylgruppen.

Die Verbindungen der Formel XIV sind bekannt oder können nach an sich bekannten Verfahren, beispielsweise nach der von McCall (Synthesis <u>1980</u>, 123) beschriebenen Methode, hergestellt werden.

Die Verbindungen der Formel XV werden erhalten, indem man die Cyanimine der Formel XIV mit Hydrazinen der Formel $R_6$-NH-$NH_2$, beispielsweise nach der unter Verfahren i) beschriebenen Methode, umsetzt.

Bei der Ringschlussreaktion können als Reaktionsprodukte neben den 5-Amino-3-($R_8$-$CH_2$)-1-($R_6$)-triazolen auch die isomeren 3-Amino-5-($R_8$-$CH_2$)-1-($R_6$)-triazole gebildet werden. Das Isomerenverhältnis hängt von den Reaktionsbedingungen, z.B. Temperatur oder Lösungsmittel, und von der Art der Substituenten $R_8$ und insbesondere $R_6$ ab. Die Isomeren können gewünschtenfalls in üblicher Weise, beispielsweise durch fraktionierte Kristallisation, Chromatographie, etc., getrennt werden. Die Verbindungen der Formel XV können als Isomerengemisch oder als reine Isomere in die weiteren Reaktionsschritte eingesetzt werden.

Stufe 2: In Verbindungen der Formel IIIa liegen die Aminogruppe Am und die Carboxylgruppe gegebenenfalls in geschützter Form vor, Die Verbindungen der Formel IIIa können hergestellt werden, indem man in Verbindungen der Formel XV die freie Aminogruppe gegebenenfalls in eine geschützte Aminogruppe überführt und in Verbindungen der Formel XV, worin $R_8$ Wasserstoff oder Cyano ist, Wasserstoff oder Cyano durch Carboxy ersetzt.
Die Einführung der Aminoschutzgruppe erfolgt nach einem der üblichen Verfahren, z.B. Acylierung, Silylierung oder Alkylierung. Eine geschützte Aminogruppe weist eine der oben beschriebenen Schutzgruppen auf, beispielsweise eine Formyl-, tert.-Butoxycarbonyl-. Benzyloxycarbonyl- oder 2,2,2-Trichloräthoxycarbonyl-Gruppe.

In einer Verbindung der Formel XV, worin $R_8$ Wasserstoff oder Cyano bedeutet, wird Wasserstoff bzw. Cyano in an sich bekannter Weise durch Carboxy ersetzt, beispielsweise durch Carboxylierung bzw. alkalische oder saure Hydrolyse. Die Carboxylierung von Verbindungen der Formel XV, worin $R_8$ Wasserstoff ist, erfolgt beispielsweise, in dem man sie metalliert und mit Kohlendioxid behandelt. Als Metallierungsmittel

kommen insbesondere Organolithiumverbindungen, wie Niederalkyllithium, z.B. Methyl-, Aethyl- oder Butyllithium, Aryllithium, z.B. Phenyllithium, und Lithiumamide, wie Lithiumdiniederalkylamide, z.B. Lithiumdiisopropylamid, in Frage. Die Metallierung wird in einem inerten Lösungsmittel, wie einem Aether, z.B. Diäthyläther, Dioxan oder Tetrahydrofuran, oder einem Kohlenwasserstoff, z.B. Hexan oder Benzol, oder einem tertiären Amin oder Diamin, z.B. Tetramethyläthylendiamin, bei erniedrigten Temperaturen, z.B. bei etwa 0°C bis etwa -80°C, durchgeführt. Anschliessend wird bei der gleichen Temperatur Kohlendioxid eingeleitet, oder das Metallierungsgemisch wird auf Trockeneis gegossen. Wenn man statt Kohlendioxid einen Halogenameisensäureester benutzt, erhält man einen Ester einer Säure der Formel IIIa.

Carbonsäuren der Formel IIIa sind weiterhin erhältlich, indem man Nitrile der Formel XV ($R_8$: Cyano) einer alkalischen oder, bevorzugt, sauren Hydrolyse unterwirft. Bei der alkalischen Hydrolyse arbeitet man vorzugsweise in Gegenwart eines Alkali- oder Erdalkalimetallhydroxids, z.B. Natrium-, Kalium-, Calcium- oder Bariumhydroxid, in siedender wässriger oder, zur Erzielung höherer Temperaturen, z.B. von 100° bis 150°C, in alkoholischer Lösung, z.B. in Amylalkohol oder Glyzerin. Die saure Hydrolyse wird vorzugsweise durch Erhitzen, z.B. auf 80° bis 120°C, mit konzentrierten Halogenwasserstoffsäuren, z.B. Chlor- oder Bromwasserstoffsäure, oder mit hochprozentiger, z.B. 50% bis 75% freier Säure enthaltender Schwefelsäure durchgeführt, wobei man zur besseren Löslichkeit des Ausgangsprodukts einen Löslichkeitsvermittler, z.B. Essigsäure, zugeben kann.

Stufe 3: 5-Amino-triazol-3-ylessigsäuren der Formel IIIa können auch erhalten werden, indem man ein Aminoguanidin der Formel XVI oder ein Salz davon mit Malonsäure umsetzt.

Salze von Verbindungen der Formel XVI sind insbesondere Salze mit Mineralsäuren, wie Halogenwasserstoffsäuren, Schwefelsäure oder Salpetersäure, oder mit Kohlensäure. Solche Salze sind beispielsweise Hydrochloride, Hydrobromide, Hydrogensulfate, Nitrate oder Hydrogencarbonate.

Die Umsetzung erfolgt z.B. in wässeriger Lösung, gegebenenfalls unter Zusatz von katalytischen Mengen eines sauren Kondensationsmittels, z.B. Salpetersäure oder Schwefelsäure, bei erhöhter Temperatur, z.B. bei Siedetemperatur oder in einem Temperaturbereich von etwa 120° bis 180°C, wobei die Reaktion im zuletzt genannten Fall in einem geschlossenen Gefäss durchgeführt wird. Man kann aber auch in einem organischen Lösungsmittel arbeiten, wobei ein solches Lösungsmittel mit Wasser vorzugsweise ein azetropes Gemisch bildet, z.B. Toluol oder Xylol, und das während der Reaktion gebildete Wasser durch Erhitzen auf die Siedetemperatur des verwendeten Lösungsmittel azeotrop abdestillieren.

Stufe 4: Verbindungen der Formel IIIb, worin eine Aminogruppe Am geschützt ist und die Carboxylgruppe gegebenenfalls in geschützter Form vorliegt, werden z.B. erhalten, indem man entsprechend geschützte Derivate von Verbindungen der Formel IIIa mit einem geeigneten Oxidationsmittel, z.B. Selendioxid, behandelt. Die Reaktion wird in einem inerten Lösungsmittel, wie einem Aether, z.B. Dioxan oder Tetrahydrofuran, einem Amid, z.B. Dimethylformamid, oder einem tertiären Amin, z.B. Pyridin, gegebenenfalls bei erniedrigter oder erhöhter Temperatur, z.B. bei etwa 0° bis etwa 100°C, bevorzugt bei Zimmertemperatur bis zu etwa 95°C, durchführt.

Stufe 5: Die Ueberführung von Verbindungen der Formel IIIa in Verbindungen der Formel IIIc, worin eine Aminogruppe Am in geschützter Form und die Carboxygruppe gegebenenfalls in geschützter Form vorliegen, erfolgt beispielsweise nach einem der unter Verfahren g) genannten Methoden mit einem der dort genannten Nitrosierungsmittel.

Stufe 6: Die Umsetzung einer Verbindung der Formel IIIb, worin eine Aminogruppe Am geschützt und die Carboxygruppe gegebenenfalls geschützt ist, mit einem Hydroxylamin der Formel $H_2N-OR_5$ kann z.B. nach einem der unter Verfahren e) beschriebenen Methoden durchgeführt werden.

In verfahrensgemäss erhältlichen Verbindungen der Formel IIIa, IIIb und IIIc können gegebenenfalls vorhandene Schutzgruppen in an sich bekannter Weise abgespalten werden. So können beispielsweise Aminoschutzgruppen, wie oben beschrieben, und Carboxyschutzgruppen, wie unter Verfahren h) beschrieben, abgespalten werden.

Reaktionsfähige funktionelle Derivate von Carbonsäuren der Formel III, die zur Acylierung geeignet sind, werden auf an sich bekannte Weise, beispielsweise wie unter Verfahren a) beschrieben, gegebenenfalls in situ, hergestellt.

Ferner kann in Verbindungen der Formel IIIc, worin $R_5$ Wasserstoff ist, die Hydroxyiminogruppe analog Verfahren f) veräthert oder carbamoyliert werden.

In Verbindungen der Formel IIIa, IIIb oder IIIc, worin $R_6$ Wasserstoff ist, kann, beispielsweise nach einem der unter Verfahren j) beschriebenen Methoden, ein gegebenenfalls substituierter Kohlenwasserstoff- oder Acylrest $R_6$ eingeführt werden. Je nach Art der Substituenten am Triazolring und des Restes $R_6$ und in Abhängigkeit von den gewählten Reaktionsbedingungen (Temperatur, Lösungsmittel, basisches Kondensationsmittel, etc.) können dabei 5—Amino—1-($R_6$)-triazol-3-ylessigsäuren oder 3—Amino—1-($R_6$)-triazol-5-ylessigsäuren oder Gemische derselben entstehen. Gemische können in üblicher Weise, z.B. durch fraktionierte Kristallisation, Chromatographie, etc., aufgetrennt werden.

Weiterhin kann in Verbindungen der Formeln IIIa, IIIb und IIIc, ferner auch XV, eine Aminogruppe Am in an sich bekannter Weise, beispielsweise wie oben beschrieben, in eine substituierte Aminogruppe, z.B. in Diniederalkylamino-niederalkylidenamino, überführt werden.

Ein erhältliches Gemisch von isomeren Verbindungen der Formel III kann, beispielsweise wie bei den Verbindungen der Formel I beschrieben, in die einzelnen Isomeren aufgetrennt werden.

Verbindungen der Formel IV sind bekannt oder können in an sich bekannter Weise erhalten werden, beispielsweise indem man von den entsprechenden 7β-Amino-3-($R_2'$-$CH_2$)-3-cephem-4-carbonsäure-Verbindungen ausgeht und die Aminogruppe, z.B. durch Behandeln mit einem den Acylrest einer Säure der Formel III einführenden Acylierungsmittel, z.B. nach einer der unter Verfahren a) beschriebenen Methoden, acyliert.

Ausgangsstoffe der Formel VI können u.a. als Nebenprodukte bei der Herstellung von Verbindungen vom Typ der Formel I erhalten werden, z.B. wenn diese unter basischen Bedingungen gebildet werden. Sie brauchen nicht in reiner Form vorzuliegen, sondern können z.B. auch im Gemisch mit entsprechenden Verbindungen der Formel I eingesetzt werden.

Ausgangsstoffe der Formel XII können beispielsweise hergestellt werden, indem man 7β-Amino-cephem-Verbindungen der Formel II mit einem den Cyanacetyl-Rest einführenden Mittel behandelt, z.B. mit Cyanessigsäure nach einer der unter Verfahren a) beschriebenen Methoden, die entstandene 7β-Cyanacetylamino-Verbindung der Formel

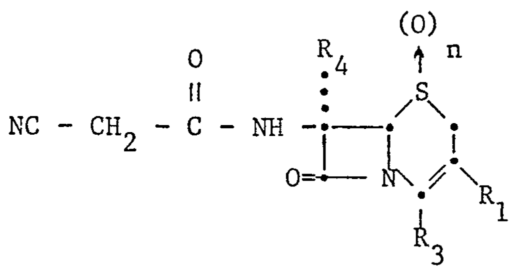

(XIX)

mit Chlorwasserstoff und einem Niederalkanol, z.B. Aethanol, in das Iminoester-Hydrochlorid der Formel

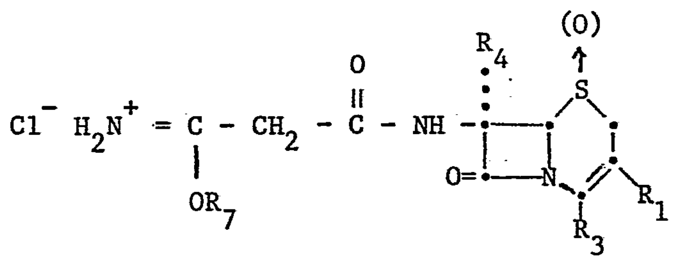

(XX)

umwandelt und dieses mit Cyanamid behandelt.

Verbindungen der Formeln V, VII bis X und XIII fallen unter die allgemeine Formel I und können gemäss einem der Verfahren a), b), d), oder i) erhalten werden.

Neue Ausgangsverbindungen, sowie Zwischenprodukte und Verfahren zu ihrer Herstellung sind ebenfalls Gegenstand der vorliegenden Erfindung.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in Form von parenteral, z.B. intramuskulär oder intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Gewisse Verbindungen der Formel I, z.B. solche, worin $R_3$ eine unter physiologischen Bedingungen leicht spaltbare, veresterte Carboxylgruppe ist, können auch oral, z.B. in Form von Kapseln, verabreicht werden. Diese enthalten den Wirkstoff, gegebenenfalls zusammen mit geeigneten Trägerstoffen, in Form eines Granulats und zwar in Dosen von etwa 0,2 bis 0,5 g pro Dosiseinheit.

Je nach der Art der Infektion und Zustand des infizierten Organismus verwendet man tägliche Dosen von etwa 0,5 g bis etwa 5 g parenteral (z.B. i.m. oder i.v.) zur Behandlung von Warmblütern von etwa 70 kg Gewicht.

Die folgenden Beispiele dienen zur Illustration der Erfindung; Temperaturen werden in Celsiusgraden angegeben.

In den Beispielen werden folgende Abkürzungen verwendet:
BOC: tert.-Butyloxycarbonyl
F.: Schmelzpunkt
Dünnschichtchromatographie (DC): Opti-UPC$_{12}$-Platten der Fa. Antec sind mit Silicagel beschichtete Glasplatten, wobei das Silicagel mit Dodecyltrichlorsilan behandelt wurde (zur Chromatographie mit umgekehrter Phase).

Beispiel 1:

a) Natriumsalz der 7β-[2-(5-Amino-1-methyl-1H-1,2,4-triazol-3-yl)-acetamino]-3-acetoxymethyl-3-cephem-4-carbonsäure

Eine Lösung von 1,07 g (1,50 mMol) 7β-[2-(5-tert.Butoxycarbonylamino-1-methyl-1H-1,2,4-triazol-3-yl)-acetamino]-3-acetoxymethyl-3-cephem-4-carbonsäure-diphenylmethylester in 2,5 ml Methylenchlorid und 0,83 ml Anisol wird bei Raumtemperatur mit 10,75 ml Trifluoressigsäure versetzt und während 30 Min. gerührt und anschliessend in 300 ml Diäthyläther/Hexan 1:2 eingerührt. Der Niederschlag, bestehend aus dem Trifluoracetat der Titelverbindung, wird durch Filtration isoliert, in Methanol gelöst und mit einer methanolischen ca. 3N Natrium-2-äthylhexanoat-lösung auf pH 7,0 gestellt. Nach Zugabe von Diäthyläther wird der gebildete Niederschlag abgenutscht und chromatographiert ($UPC_{12}$-Platten, Wasser/Acetonitril 6:1). Man erhält die einheitliche Titelverbindung vom Rf-Wert 0,50 ($UPC_{12}$-Platten, Wasser/Acetonitril 6:1). IR-Spektrum (Nujol): charakteristische Absorptionsbanden bei 3450; 1785; 1610; 1385; 1235 $cm^{-1}$. UV-Spektrum (Aethylalkohol) $\lambda_{max}$ ($\varepsilon$): 263 (6800) nm.

Das Ausgangsmaterial kann wie folgt hergestellt werden:

aa) 5-Amino-1-methyl-1H-1,2,4-triazol-3-yl-essigsäureäthylester

Eine Lösung von 35,5 g (0,193 Mol) 3-Cyanoimino-3-äthoxy-propionsäureäthylester in 65 ml Acetonitril wird bei 0° mit 13,1 ml Methylhydrazin versetzt und während 4 Stunden bei dieser Temperatur gerührt. Der entstandene Niederschlag wird filtriert und mit Diäthyläther gewaschen. Das Filtrat wird eingeengt und mit Diäthyläther versetzt, wobei weiteres Produkt ausfällt. Man erhält die einheitliche Titelverbindung vom Rf.-Wert 0,70 (Silicagel, Chloroform/Methanol/Eisessig 85:12:3). F.: 115-6°. IR-Spektrum ($CH_2Cl_2$): charakteristische Absorptionsbanden bei 3475; 3380; 1735; 1630 $cm^{-1}$.

ab) <u>1-Methyl-5-(N,N-di-tert.butoxycarbonylamino)-1H-1,2,4-triazol-3-yl-essigsäureäthylester</u>

Eine Suspension von 2,09 g (10,87 mMol) 5-Amino-1-methyl-1H-1,2,4-triazol-3-yl-essigsäureäthylester und 5,2 g BOC-Anhydrid wird bei 60° (Badtemperatur) während 3 Stunden gerührt. Man lässt das Reaktionsgemisch abkühlen, verdünnt mit 20 ml Aethylacetat, wäscht mit gesättigter Natriumchloridlösung, trocknet über Magnesiumsulfat und engt ein. Der Rückstand wird aus Aethylacetat/Hexan gefällt. Man erhält die Titelverbindung vom Rf-Wert 0,60 (Silicagel, Chloroform/Methanol 95:5). F. 98-100°. IR-Spektrum ($CH_2Cl_2$): charakteristische Absorptionsbanden bei 3400; 3100; 1785; 1745; 1660; 1125 $cm^{-1}$.

Man kann die Titelverbindung auch wie folgt erhalten:

ab1) Eine Lösung von 5 ml 20%igem Phosgen in Toluol und 8 ml Tetrahydrofuran wird bei -20° mit einer Suspension von 0,92 g (5,0 mMol) 5-Amino-1-methyl-1H-1,2,4-triazol-3-yl-essigsäureäthylester und 0,8 ml Triäthylamin in 10 ml Tetrahydrofuran versetzt und während 10 Min. gerührt. Nach Zugabe von 10 ml tert.Butylalkohol und 2 ml Triäthylamin wird das Gemisch langsam auf Raumtemperatur erwärmt, mit Diäthyläther verdünnt, mit Wasser gewaschen, über Natriumsulfat getrocknet und eingeengt. Der Rückstand wird aus Aethylacetat/Hexan gefällt. Man erhält die Titelverbindung vom Rf-Wert 0,60 (Silicagel, Chloroform/Methanol 95:5).

Die Titelverbindung kann man ebenfalls wie folgt erhalten:

ab2) Eine Suspension von 0,184 g (1,0 mMol) 5-Amino-1-methyl-1H-1,2,4-triazol-3-yl-essigsäureäthylester, 1 ml tert.Butylalkohol und 0,262 g BOC-Anhydrid wird während 16 Stunden bei 55° (Badtemperatur) gerührt. Das Reaktionsgemisch wird abgekühlt, mit Aethylacetat verdünnt, mit gesättigter Natriumchloridlösung gewaschen, über Magnesiumsulfat getrocknet und eingeengt. Der Rückstand wird aus Aethylacetat/Hexan gefällt. Man erhält die Titelverbindung vom Rf-Wert 0,60 (Silicagel, Chloroform/Methanol 95:5).

ac) 1-Methyl-5-tert.butoxycarbonylamino-1H-1,2,4-triazol-3-yl-essigsäure

Eine Lösung von 4,0 g 1-Methyl-5-(N,N-di-tert.butoxycarbonylamino)-1H-1,2,4-triazol-3-yl-essigsäureäthylester in 110 ml 95% Aethylalkohol wird mit einer Lösung von 5,475 g Kaliumhydroxyd in 45 ml Wasser versetzt und während 1,5 Stunden bei 50° gerührt. Das Reaktionsgemisch wird im Vakuum eingeengt, mit Wasser verdünnt, mit 4 N Salzsäure auf pH 8,5 eingestellt und mit Aethylacetat extrahiert. Die wässrige Phase wird dann mit 4 N Salzsäure auf pH 3,0 eingestellt, der ausgefallene Niederschlag wird filtriert, mit Diäthyläther gewaschen und getrocknet. Man erhält die Titelverbindung vom Rf-Wert 0,50 (Silicagel, sek.Butanol/Eisessig/Wasser 67:10:23). F. = 140-142°. IR-Spektrum ($CH_2Cl_2$): charakteristische Absorptionsbanden bei 3200; 3080; 1735; 1710; 1155 $cm^{-1}$.

ad) 7β-[2-(5-tert.Butoxycarbonylamino-1-methyl-1H-1,2,4-triazol-3-yl)-acetamino]-3-acetoxymethyl-3-cephem-4-carbonsäure-diphenylmethylester

Eine Lösung von 0,50 g (1,27 mMol) 1-Methyl-5-tert.butoxycarbonylamino-1H-1,2,4-triazol-3-yl-essigsäure und 0,823 g (1,88 mMol) 7β-Amino-3-acetoxymethyl-3-cephem-4-carbonsäure-diphenylmethylester in 15 ml trockenem Tetrahydrofuran wird bei 0° mit 0,21 g 1-Hydroxybenzotriazol und 0,60 g N,N'-Dicyclohexylcarbodiimid in 2 ml trockenem Tetrahydrofuran versetzt, während 2,5 Stunden bei 0° und weitere 6 Stunden bei Raumtemperatur gerührt. Nach Filtrieren des Reaktionsgemisches wird das Filtrat mit 50 ml Aethylacetat versetzt und nacheinander mit Phosphatpufferlösung pH 8,0, und wässriger Natriumchloridlösung gewaschen, über Magnesiumsulfat getrocknet und im Vakuum eingeengt. Der Rückstand wird mit Diäthyläther/Hexan verrieben. Man erhält die Titelverbindung vom Rf-Wert 0,60 (Silicagel, Aethylacetat). IR-Spektrum ($CH_2Cl_2$): charakteristische Absorptionsbanden bei 3360; 1785; 1715; 1220; 1155 $cm^{-1}$.

b) Das Natriumsalz der 7β-[2-(5-Amino-1-methyl-1H-1,2,4-triazol-3-yl)-acetamino]-3-acetoxymethyl-3-cephem-4-carbonsäure kann auch wie folgt hergestellt werden:

1,56 g (1,0 mMol) 5-Amino-1-methyl-1H-1,2,4-triazol-3-yl-essigsäure und 0,5 ml Aethylacetat werden bei 0° zu einer gerührten Suspension eines Vilsmeier-Reagenses (dargestellt aus 0,1 ml (0,12 mMol) Oxalylchlorid und 0,09 ml N,N-Dimethylformamid in 3 ml Aethylacetat) gegeben. Das Gemisch wird bei 0° während 45 Min. gerührt und anschliessend auf -10° abgekühlt (Lösung 1). Andererseits wird eine Mischung von 0,27 g (1,0 mMol) 7β-Amino-3-acetoxymethyl-3-cephem-4-carbonsäure, 11 ml N,O-Bis-(trimethylsilyl)-acetamid und 40 ml Aethylacetat während 20 Min. bei Raumtemperatur gerührt und anschliessend auf -10° abgekühlt (Lösung 2). Lösung 2 und Lösung 1 werden vereinigt. Die Mischung wird während 2 Stunden gerührt und anschliessend auf gesättigte Natriumbicarbonatlösung gegossen. Die wässrige Phase wird abgetrennt und eingeengt. Nach Chromatographie des Rückstandes an $UPC_{12}$-Silicagel mit Wasser erhält man die Titelverbindung vom Rf-Wert 0,50 ($UPC_{12}$-Platten, Wasser/Acetonitril 6:1).

Das Ausgangsmaterial kann wie folgt hergestellt werden:

ba) 5-Amino-1-methyl-1H-1,2,4-triazol-3-yl-acetonitril

Eine Lösung von 4,76 g (34,75 mMol) 3-Cyanoimino-3-äthoxy-propionitril in 10 ml Acetonitril wird bei 0° mit 2,2 ml Methylhydrazin versetzt und während 1 Stunde bei dieser Temperatur gerührt. Der entstandene Niederschlag wird gewaschen. Das Filtrat wird eingeengt und mit Aethylalkohol versetzt, wobei weiteres Produkt ausfällt. Man erhält die einheitliche Titelverbindung vom Rf-Wert 0,33 (Silicagel/Chloroform/Methanol/Eisessig 85:12:3) IR-Spektrum (KBr) charakteristische Absorptionsbanden bei 3375; 1637; 1575 $cm^{-1}$.

bb) 5-Amino-1-methyl-1H-1,2,4-triazol-3-yl-essigsäure

Eine Lösung von 0,49 g (3,59 mMol) 5-Amino-1-methyl-1H-1,2,4-triazol-3-yl-acetonitril in 7,4 ml 50% Schwefelsäure wird während 16 Stunden bei 100° gerührt. Das Reaktionsgemisch wird abgekühlt, auf Eis gegossen, mit konz. Ammoniak-Lösung auf pH 5.5 eingestellt und mit Aethylalkohol versetzt. Der Niederschlag wird abgenutscht, mit Aethylalkohol gewaschen und getrocknet. Nach Chromatographie des Rohproduktes ($UPC_{12}$-Platten, Wasser/Acetonitril 4:1) erhält man die einheitliche Titelverbindung vom Rf-Wert 0,80 ($UPC_{12}$-Platten, Wasser/Acetonitril 6:1). IR.-Spektrum (Nujol): charakteristische Absorptionsbanden bei 3450; 3375; 1600; 1537 $cm^{-1}$.

Beispiel 2:

a) 7β-[2-(5-Amino-1-methyl-1H-1,2,4-triazol-3-yl)-2-Z-methoxyimino-acetamino]-3-acetoxymethyl-3-cephem-4-carbonsäure-hydrochlorid

1,99 g (10,0 mMol) 2-(5-Amino-1-methyl-1H-1,2,4-triazol-3-yl)-2-methoxyimino-essigsäure und 40 ml Methylenchlorid werden bei -5° zu einer gerührten Suspension eines Vilsmeier-Reagenses (dargestellt aus 1,0 ml Oxalylchlorid und 0,9 ml N,N-Dimethylformamid in 10 ml Methylenchlorid) gegeben. Das Gemisch wird während 20 Min. bei 0° gerührt und anschliessend auf -10° abgekühlt (Lösung 1). Andererseits wird eine Mischung von 2,7 g (10,0 mMol) 7β-Amino-3-acetoxymethyl-3-cephem-4-carbonsäure, 11 ml N,O-Bis-(trimethylsilyl)-acetamid und 50 ml Methylenchlorid während 30 Min. bei Raumtemperatur gerührt und anschliessend auf -10° abgekühlt (Lösung 2). Lösung 2 und Lösung 1 werden vereinigt. Die Mischung wird bei 0° während 2,5 Stunden gerührt und anschliessend auf gesättigte Natriumbicarbonatlösung gegossen. Die wässrige Phase wird abgetrennt, mit wässriger Salzsäure sauer gestellt, mit festem Natriumchlorid gesättigt und mit Aethylacetat/Aceton 4:1 extrahiert. Die organischen Extrakte werden über Natriumsulfat getrocknet und im Vakuum eingeengt. Nach Chromatographie des

Rückstandes an XAD-2-Harz(Wasser) erhält man die Titelverbindung vom Rf-Wert 0,30 ($UPC_{12}$-Platten, Wasser/Acetonitril 6:1). UV-Spektrum (Wasser)$\lambda_{max}$ ($\varepsilon$): 235 (11700); 260 (12700)nm.

Die Ausgangsverbindung kann wie folgt hergestellt werden:

b) <u>2-[5-(N-Benzyloxycarbonylamino)-1-methyl-1H-1,2,4-triazol-3-yl]-essigsäureäthylester</u>

Zu einem Gemisch von 10,0 g (54,35 mMol) 5-Amino-1-methyl-1H-1,2,4-triazol-3-yl-essigsäureäthylester und 5,8 g Natriumcarbonat in 54 ml Wasser werden bei 0° 2,9 ml Chlorameisensäurebenzylester gegeben und während 4 Stunden bei 0° gerührt. Das Gemisch wird mit Wasser verdünnt und mit Aethylacetat extrahiert. Die organischen Extrakte werden mit gesättigter Natriumchloridlösung gewaschen, über Magnesiumsulfat getrocknet und eingeengt. Der Rückstand wird chromatographiert (Silicagel, Toluol, steigende Anteile Aethylacetat). Man erhält die einheitliche Titelverbindung vom Rf-Wert 0,25 (Silicagel, Toluol/Aethylacetat 1:1). IR-Spektrum (Film): charakteristische Absorptionsbanden bei 3150; 1735; 1570 $cm^{-1}$.

c) <u>2-[5-(N-Benzyloxycarbonyl)-1-methyl-1H-1,2,4-triazyl-3-yl]-2-oxo-essigsäureäthylester</u>

Eine Suspension von 16,4 g (51,5 mMol) 2-[5-(N-Benzyloxycarbonylamino)-1-methyl-1H-1,2,4-triazol-3-yl]-essigsäureäthylester und 15,4 g Selendioxid in 235 ml Dioxan wird 2,5 Stunden bei 95° gerührt. Nach Filtrieren des Reaktionsgemisches wird das Filtrat im Vakuum eingeengt. Der Rückstand enthält die Titelverbindung vom Rf-Wert 0,35 (Silicagel,Aethylacetat). IR-Spektrum (Film): charakteristische Absorptionsbanden bei 3300; 1737; 1715 $cm^{-1}$.

d) 2-(5-Amino-1-methyl-1H-1,2,4-triazol-3-yl)-2-methoxyimino-essigsäureäthylester

Eine Suspension von 22,65 g (68,20 mMol) 2-[5-(N-Benzyloxycarbonylamino)-1-methyl-1H-1,2,4-triazol-3-yl]-2-oxo-essigsäureäthylester, 6,30 g Methoxyamin-Hydrochlorid und 6,8 ml Pyridin in 215 ml 95% Aethanol wird 3,5 Stunden bei Raumtemperatur gerührt. Das Reaktionsgemisch wird im Vakuum eingeengt. Der Rückstand wird in Aethylacetat gelöst und die Lösung nacheinander mit verdünnter Salzsäure, Wasser und wässriger Natriumchloridlösung extrahiert, über Magnesiumsulfat getrocknet und im Vakuum eingeengt. Der Rückstand wird mit Diäthyläther verrieben. Man erhält die Titelverbindung vom Rf-Wert 0,25 (Silicagel, Chloroform/Methanol 95:5). IR-Spektrum (Nujol): charakteristische Absorptionsbanden bei 3350; 1735; 1645; 1045 $cm^{-1}$.

e) 2-(5-Amino-1-methyl-1H-1,2,4-triazol-3-yl)-2-methoxyimino-essigsäure

Eine Lösung von 11,96 g (52,64 mMol) 2-(5-Amino-1-methyl-1H-1,2,4-triazol-3-yl)-2-methoxyimino-essigsäureäthylester in 350 ml 95% Aethanol wird mit 17,25 g Kaliumhydroxid und 140 ml Wasser versetzt und während 3 Stunden bei 40° gerührt. Das Reaktionsgemisch wird im Vakuum eingeengt, der Rückstand in Wasser gelöst, die Lösung mit verdünnter Salzsäure auf pH 8,5 gestellt und mit Aethylacetat extrahiert. Die wässrige Phase wird abgetrennt, mit verdünnter Salzsäure auf pH 4,0 gestellt und das Produkt mit Aethanol ausgefällt. Man erhält die Titelverbindung vom Rf-Wert 0,20 (Silicagel, s-Butanol/Eisessig/Wasser 67:10:23). IR-Spektrum (Nujol): charakteristische Absorptionsbanden bei 3550; 3325; 1660; 1125 $cm^{-1}$.

Beispiel 3:

a) Natriumsalz der 7β-[2-(5-Amino-1-methyl-1H-1,2,4-triazol-3-yl)-2-Z-methoxyimino-acetamino]-3-acetoxymethyl-3-cephem-4-carbonsäure

Aus 5,1 g (8,2 mMol) 7β-[2-(5-Amino-1-methyl-1H-1,2,4-triazol-3-yl)-2-Z-methoxyimino-acetamino]-3-acetoxymethyl-3-cephem-4-carbonsäure-diphenylmethylester in 13 ml Methylenchlorid, 4 ml Anisol und 60 ml Trifluoressigsäure erhält man analog Beispiel 1a) die Titelverbindung vom Rf-Wert 0,20 ($UPC_{12}$-Platten, Wasser/Acetonitril 95:5). UV-Spektrum ($H_2O$): $\lambda_{max}$ ($\varepsilon$) 235 (11700)nm. IR-Spektrum (Nujol): charakteristische Absorptionsbanden bei 3350; 1765; 1605; 1235; 1045 $cm^{-1}$.

Das Ausgangsmaterial kann wie folgt hergestellt werden:

b) 7β-[2-(5-Amino-1-methyl-1H-1,2,4-triazol-3-yl)-2-Z-methoxyimino-acetamino]-3-acetoxymethyl-3-cephem-4-carbonsäure-diphenylmethylester

1,99 g (10,0 mMol) 2-(5-Amino-1-methyl-1H-1,2,4-triazol-3-yl)-2-methoxyiminoessigsäure und 40 ml Methylenchlorid werden bei -5° zu einer gerührten Suspension eines Vilsmeier-Reagenses (dargestellt aus 1,0 ml Oxalylchlorid und 0,9 ml N,N-Dimethylformamid in 10 ml Methylenchlorid) gegeben. Das Gemisch wird während 20 Min. bei 0° gerührt und anschliessend auf -10° abgekühlt. Nach Zugabe von 4,40 g (10,0 mMol) 7β-Amino-3-acetoxymethyl-3-cephem-4-carbonsäure-diphenylmethylester wird während 2,5 Stunden bei 0° gerührt, dann mit 50 ml Methylenchlorid verdünnt und nacheinander mit Wasser, Phosphatpuffer pH 8,0 und wässriger Natriumchloridlösung gewaschen, über Magnesiumsulfat getrocknet und im Vakuum eingeengt. Der Rückstand enthält die Titelverbindung vom Rf-Wert 0,60 (Silicagel, Chloroform/Methanol/Eisessig 85:12:3).

Beispiel 4:

Natriumsalz der 7β-[2-(5-Amino-1-methyl-1H-1,2,4-triazol-3-yl)-2-Z-methoxyimino-acetamino]-3-(1-methyltetrazol-5-yl-thiomethyl)-3-cephem-4-carbonsäure.

Eine Lösung von 0,475 g (1,0 mMol) Natrium-7β-[2-(5-amino-1-methyl-1H-1,2,4-triazol-3-yl)-2-Z-methoxyimino-acetamino]-3-acetoxymethyl-3-cephem-4-carboxylat und 0,207 g (1,5 mMol) Natrium-1-methyl-tetrazol-5-yl-thiolat in 4,5 ml Wasser werden während 5 Stunden bei

70° gerührt, dann mit Wasser verdünnt und mit Aethylacetat extrahiert. Die wässrige Phase wird eingeengt und chromatographiert ($UPC_{12}$-Platten, Wasser/Acetonitril 95:5). Man erhält die Titelverbindung vom Rf-Wert 0,25 ($UPC_{12}$-Platten, Wasser/Acetonitril 95:5).
IR - Spektrum (Nujol): charakteristische Absorptionsbanden bei 3400; 1762; 1640; 1595; 1045 $cm^{-1}$.

Beispiel 5:

7β-[2(5-Amino-1-methyl-1H-1,2,4-triazol-3-yl)-2-Z-methoxyimino-acetamino]-3-(4-carbamoylpyridiniomethyl)-3-cephem-4-carboxylat und 7β-[2-(5-Dimethylaminomethylidenamino-1-methyl-1H-1,2,4-triazol-3-yl)-2-Z-methoxyimino-acetamino]-3-(4-carbamoylpyridiniomethyl)-3-cephem-4-carboxylat.

1,99 g (10 mMol) 2-(5-Amino-1-methyl-1H-1,2,4-triazol-3-yl)-2-methoxyimino-essigsäure werden bei -5° zu einer gerührten Suspension eines Vilsmeyer-Reagenzes (dargestellt aus 1,0 ml Oxalylchlorid und 0,9 ml N,N-Dimethylformamid in 25 ml Methylenchlorid) gegeben. Das Gemisch wird während 20 Minuten bei 0° gerührt und anschliessend auf -10° abgekühlt (Lösung 1). Andererseits wird eine Mischung von 4,08 g (10 mMol) 7β-Amino-3-(4-carbamoylpyridiniomethyl)-3-cephem-4-carbonsäure, 10,5 ml N,O-Bis-(trimethylsilyl)-acetamid und 40 ml Methylenchlorid während 30 Minuten bei Raumtemperatur gerührt und anschliessend auf -20° abgekühlt (Lösung 2). Lösung 1 und Lösung 2 werden vereinigt. Die Mischung wird bei 0° während 3 Stunden gerührt und anschliessend auf 1 N Natriumbicarbonatlösung gegossen. Die wässrige Phase wird abgetrennt, mit wässriger Salzsäure sauer gestellt und chromatographiert (XAD-2 Harz, Wasser/Aethanol Gemische). Man erhält die einheitliche Titelverbindung 7β-[2-(5-Amino-1-methyl-1H-1,2,4-triazol-3-yl)-2-Z-methoxyimino-acetamino]-3-(4-carbamoylpyridiniomethyl)-3-cephem-4-carboxylat vom Rf-Wert 0,65 ($UPC_{12}$-Platten, Wasser/Acetonitril 6:1), IR - Spektrum (KBr): charakteristische Absorptionsbanden bei 3425; 1775; 1620; 1385 $cm^{-1}$; und die einheitliche Titelverbindung 7β-[2-(5-Dimethylaminomethylidenamino-1-methyl-1H-1,2,4-triazol-3-yl)-2-Z-methoxyimino-acetamino]-3-(4-carbamoyl-pyridiniomethyl)-3-cephem-4-carboxylat vom Rf-Wert 0,30 ($UPC_{12}$-Platten, Wasser/Acetonitril 6:1). IR—Spektrum (KBr): charakteristische Absorptionsbanden bei 1775; 1615; 1537 $cm^{-1}$.

Beispiel 6:

7β-[2-(5-Amino-1-methyl-1H-1,2,4-triazol-3-yl)-2-Z-methoxyimino-acetamino]-3-(4-carbamoylpyridiniomethyl)-3-cephem-4-carboxylat kann man auch wie folgt herstellen:

Eine Lösung von 0,950 g (2,0 mMol) Natrium-7β-[2-(5-amino-1-methyl-1H-1,2,4-triazol-3-yl)-2-Z-methoxyimino-acetamino-3-acetoxymethyl-3-cephem-4-carboxylat in 2 ml Wasser wird zu einer 80° warmen Lösung von 0,34 g Isonicotinsäureamid und 3,42 g Natriumjodid in 0,75 ml Wasser gegeben und während 2,5 Stunden bei 75° gerührt. Nach Abkühlen auf Raumtemperatur wird das Gemisch auf 250 ml Aceton gegossen, der gebildete Niederschlag filtriert und getrocknet. Nach Chromatographie des Rohproduktes an einer XAD-2 Säule mit Wasser/Methanol Gemischen erhält man die einheitliche Titelverbindung vom Rf-Wert 0,65 ($UPC_{12}$-Platten, Wasser/Acetonitril 6:1). IR—Spektrum (KBr): charakteristische Absorptionsbanden bei 1775; 1620; 1045 $cm^{-1}$.

Beispiel 7:

7β-[2-(5-Amino-1-methyl-1H-1,2,4-triazol-3-yl)-2-Z-methoxyimino-acetamino)-3-(4-hydroxymethylpyridiniomethyl)-3-cephem-4-carboxylat.

Ausgehend von 0,475 g (1,0 mMol) Natrium 7β-[2-(5-Amino-1-methyl-1H-1,2,4-triazol-3-yl)-2-Z-methoxyimino-acetamino)-3-acetoxymethyl-3-cephem-4-carboxylat, 0,12 g (1,1 mMol) 4-Hydroxymethylpyridin und 1,75 g Natriumjodid in 2 ml Wasser erhält man analog Beispiel 6 die Titelverbindung vom Rf-Wert 0,55 ($UPC_{12}$-Platten, Wasser/Acetonitril 6:1); IR—Spektrum (Nujol): charakteristische Absorptionsbanden bei 1765; 1620; 1040 $cm^{-1}$.

Beispiel 8:

a) Natriumsalz der 7β-[2-(5-Amino-1-methyl-1H-1,2,4-triazol-3-yl)-2-hydroxyimino-acetamino]-3-acetoxymethyl-3-cephem-4-carbonsäure.

Eine Lösung von 2,77 g (4,45 mMol) 7β-[2-(5-tert.-Butoxycarbonylamino-1-methyl-1H-1,2,4-triazol-3-yl)-2-tetrahydropyranyloxyimino-acetamino]-3-acetoxymethyl-3-cephem-4-carbonsäure in 10 ml Methylenchlorid wird bei Raumtemperatur mit 49 ml Trifluoressigsäure versetzt, während 60 Minuten bei gleicher Temperatur gerührt und anschliessend in 600 ml Diäthyläther/Hexan (1:2) eingerührt. Der Niederschlag, bestehend aus dem Trifluoracetat der Titelverbindung, wird durch Filtration isoliert, in Methanol gelöst und mit einer methanolischen Natrium-2-äthylhexanoatlösung (ca. 3 N) auf pH 7,0 gestellt. Nach Zugabe von Diäthyläther wird der gebildete Niederschlag abgenutscht und chromatographiert ($UPC_{12}$-Silicagel, Wasser). Man erhält die einheitliche Titelverbindung vom Rf-Wert 0,65 ($UPC_{12}$-Platten, Wasser/Acetonitril 9:1); IR—Spektrum (Nujol): charakteristische Absorptionsbanden bei 3425; 1767; 1735; 1600 $cm^{-1}$.

Die Ausgangsverbindung kann wie folgt hergestellt werden:

b) 2-(5-Amino-1-methyl-1H-1,2,4-triazol-3-yl)-2-tetrahydropyranyl-oxyimino-essigsäureäthylester

Eine Lösung von 10,0 g (30,0 mMol) 2-[5-(N-Benzyloxycarbonylamino)-1-methyl-1H-1,2,4-triazol-3-yl]-2-oxo-essigsäureäthylester und 5,30 g Tetrahydropyranyloxyamin in 80 ml 95 %igem Aethanol wird während 2 Stunden bei Raumtemperatur und weitere 2 Stunden bei 60° gerührt. Das Reaktionsgemisch wird im Vakuum eingeengt. Nach Chromatographie des Rückstandes an Silicagel (Chloroform-steigende Anteile Methanol) erhält man die Titelverbindung vom Rf-Wert 0,40 (Silicagel, Chloroform/Methanol 9:1). NMR—Spektrum (60 MHz, $CDCl_3$): charakteristische Signale bei 1,46 (t); 3,70 (s); 4,70 (q); 5,80 (s) ppm.

c) 2-[5-(N,N-Di-tert.-butoxycarbonylamino)-1-methyl-1H-1,2,4-triazol-3-yl)-2-tetrahydropyranyloxyimino-essigsäureäthylester.

Eine Suspension von 4,30 g (14,6 mMol) 2-(5-Amino-1-methyl-1H-1,2,4-triazol-3-yl)-2-tetrahydropyranyloxyimino-essigsäureäthylester, 8,30 g BOC-Anhydrid und 0,40 g 4-(N,N-Dimethylamino)-pyridin wird während 3,5 Stunden bei Raumtemperatur gerührt, mit Aethylacetat verdünnt, mit wässriger Natriumchloridlösung extrahiert, über Magnesiumsulfat getrocknet und im Vakuum eingeengt. Man erhält die Titelverbindung vom Rf-Wert 0,85 (Silicagel, Chloroform/Methanol 95:5). IR—Spektrum (Methylenchlorid): charakteristische Absorptionsbanden bei 1790; 1735; 1600 $cm^{-1}$.

d) 2-(5-tert.-Butoxycarbonylamino-1-methyl-1H-1,2,4-triazol-3-yl)-2-tetrahydropyranyloxyiminoessigsäure.

Ausgehend von 8,30 g (16,7 mMol) 2-[5-(N,N-Di-tert.-butoxycarbonylamino)-1-methyl-1H-1,2,4-triazol-3-yl)-2-tetrahydropyranyloxyimino-essigsäureäthylester und 5,43 g Kaliumhydroxid in 45 ml Wasser und 110 ml 95 %igem Aethanol erhält man analog Beispiel 2e die Titelverbindung vom Rf-Wert 0,40 (Silicagel, sek.-Butanol/Eisessig/Wasser 67:10:23) IR—Spektrum (Nujol): charakteristische Absorptionsbanden bei 3500; 1740 $cm^{-1}$.

e) 7β-[2-(5-tert.-Butoxycarbonylamino-1-methyl-1H-1,2,4-triazol-3-yl)-2-tetrahydropyranyloxyimino-acetamino]-3-acetoxymethyl-3-cephem-4-carbonsäure.

2,70 g (7,32 mMol) 2-(5-tert.-Butoxycarbonylamino-1-methyl-1H-1,2,4-triazol-3-yl)-2-tetrahydropyranyloxyimino-essigsäure und 0,97 ml N-Methylmorpholin werden bei -5° zu einer gerührten Suspension eines Vilsmeier-Reagenses (dargestellt aus 0,73 ml Oxalylchlorid und 0,65 ml N,N-Dimethylformamid in 27 ml Methylenchlorid) gegeben. Das Gemisch wird während 30 Minuten bei 0° gerührt und anschliessend auf -10° abgekühlt (Lösung 1). Andererseits

wird eine Mischung von 2,43 g (8,91 mMol) 7β-Amino-3-acetoxymethyl-3-cephem-4-carbonsäure, 8,1 ml N,O-Bis-(trimethylsilyl)-acetamid und 1,54 ml N-Methylmorpholin in 40 ml Methylenchlorid während 30 Minuten bei Raumtemperatur gerührt und anschliessend auf -10° abgekühlt (Lösung 2). Lösung 1 und Lösung 2 werden vereinigt. Die Mischung wird bei 0° während 3 Stunden gerührt, im Vakuum eingeengt, mit Aethylacetat verdünnt und nacheinander mit Wasser, Phosphatpuffer pH 8,0, wässriger Salzsäure und wässriger Natriumchloridlösung extrahiert, über Magnesiumsulfat getrocknet und im Vakuum eingeengt. Der Rückstand enthält die Titelverbindung vom Rf-Wert 0,60 (Silicagel, sek.-Butanol/Eisessig/Wasser 67:10:23). IR—Spektrum (Nujol): charakteristische Absorptionsbanden bei 3350; 1740; 1620 $cm^{-1}$.

Beispiel 9:

a) Natriumsalz der 7β-[2-(5-Amino-1H-1,2,4-triazol-3-yl)-2-Z-methoxyimino-acetamino]-3-acetoxymethyl-3-cephem-4-carbonsäure.

Ausgehend von 0,85 g (1,2 mMol) 7β-[2-(5-tert.-Butoxycarbonylamino-1H-1,2,4-triazol-3-yl)-2-Z-methoxyimino-acetamino]-3-acetoxymethyl-3-cephem-4-carbonsäure-diphenylmethylester in 2 ml Methylenchlorid, 0,63 ml Anisol und 8,6 ml Trifluoressigsäure erhält man analog Beispiel 1a die Titelverbindung vom Rf-Wert 0,60 ($UPC_{12}$-Platten, Wasser/Acetonitril 6:1). IR—Spektrum (Nujol): charakteristische Absorptionsbanden bei 3300; 1765; 1600; 1045 $cm^{-1}$.

Das Ausgangsmaterial kann wie folgt hergestellt werden:

b) 2-(5-Amino-1-tert.-butoxycarbonyl-1H-1,2,4-triazol-3-yl)-essigsäureäthylester.

Ausgehend von 74,0 g (401,7 mMol) 3-Cyanoimino-3-äthoxy-propionsäureäthylester und 107,3 g tert.-Butoxycarbonyl-hydrazid in 370 ml Acetonitril erhält man analog Beispiel 1aa die Titelverbindung vom

Rf-Wert 0,60 (Silicagel, Aethylacetat) F.: 117 - 118°. IR-Spektrum (Methylenchlorid): charakteristische Absorptionsbanden bei 1735; 1625 $cm^{-1}$.

c) 2-[5-(N,N-Di-tert.-butoxycarbonylamino)-1-tert.-butoxycarbonyl-1H-1,2,4-triazol-3-yl]-essigsäureäthylester

Ausgehend von 17,70 g (65,5 mMol) 2-(5-Amino-1-tert.-butoxycarbonyl-amino-1H-1,2,4-triazol-3-yl)-essigsäureäthylester, 28,6 g BOC-Anhydrid und 650 mg 4-(N,N-Dimethylamino)-pyridin erhält man analog Beispiel 8c die Titelverbindung vom Rf-Wert 0,65 (Silicagel, Toluol/Aethylacetat 1:1). IR-Spektrum (Methylenchlorid): charakteristische Absorptionsbanden bei 1800; 1760; 1735 $cm^{-1}$.

d) 2-[5-(N,N-Di-tert.-butoxycarbonylamino)-1-tert.-butoxycarbonyl-1H-1,2,4-triazol-3-yl]-2-oxo-essigsäureäthylester.

Ausgehend von 22,0 g (44,4 mMol) 2-[5-(N,N-Di-tert.-butoxycarbonyl-amino)-1-tert.-butoxycarbonyl-1H-1,2,4-triazol-3-yl]-essigsäureäthylester und 13,8 g Selendioxid in 190 ml Dioxan erhält man analog Beispiel 2c die Titelverbindung vom Rf-Wert 0,25 (Silicagel, Toluol/Aethylacetat 4:1). IR-Spektrum (Methylenchlorid): charakteristische Absorptionsbanden bei 1765; 1735; 1715 $cm^{-1}$.

e) 2-(5-tert.-Butoxycarbonylamino-1-tert.-butoxycarbonyl-1H-1,2,4-triazol-3-yl)-2-methoxyimino-essigsäureäthylester.

Ausgehend von 26,4 g (54,5 mMol) 2-[5-(N,N-Di-tert.-butoxycarbonyl-amino)-1-tert.-butoxycarbonyl-1H-1,2,4-triazol-3-yl]-2-oxo-essigsäureäthylester und 6,90 g Methoxyamin-Hydrochlorid und 6,5 ml Pyridin in 265 ml 95 %igem Aethanol erhält man analog Beispiel 2d die Titelverbindung vom Rf-Wert 0,45 (Silicagel, Toluol/Aethylacetat 1:1). IR-Spektrum (Methylenchlorid): charakteristische Absorptionsbanden bei 1735; 1050 $cm^{-1}$.

f) 2-(5-tert.-Butoxycarbonylamino-1H-1,2,4-triazol-3-yl)-2-methoxy-imino-essigsäure.

Ausgehend von 1,20 g (2,90 mMol) 2-(5-tert.-Butoxycarbonylamino-1-tert.-butoxycarbonyl-1H-1,2,4-triazol-3-yl)-2-methoxyimino-essigsäureäthylester und 1,25 g Kaliumhydroxid in 11 ml Wasser und 24 ml 95 %igem Aethanol erhält man analog Beispiel 2e die Titelverbindung vom Rf-Wert 0,20 (Silicagel, sek.-Butanol/Eisessig/Wasser 67:10:23), IR-Spektrum (Nujol): charakteristische Absorptionsbanden bei 3315; 1710; 1050 $cm^{-1}$.

g) 7β-[2-(5-tert.-Butoxycarbonylamino-1H-1,2,4-triazol-3-yl)-2-Z-methoxyimino-acetamino]-3-acetoxymethyl-3-cephem-4-carbonsäure-diphenylmethylester.

Ausgehend von 0,806 g (2,82 mMol) 2-(5-tert.-Butoxycarbonylamino-1H-1,2,4-triazol-3-yl)-2-methoxyimino-essigsäure und 1,20 g (2,73 mMol) 7β-Amino-3-acetoxymethyl-3-cephem-4-carbonsäure-diphenylmethylester in 14 ml Methylenchlorid erhält man analog Beispiel 3b die Titelverbindung vom Rf-Wert 0,65 (Silicagel, Chloroform/Methanol/Eisessig 85:12:3), IR-Spektrum (Methylenchlorid): charakteristische Absorptionsbanden bei 1765; 1735 $cm^{-1}$.

Beispiel 10:

Natriumsalz der 7β-[2-(5-Amino-1-methyl-1H-1,2,4-triazol-3-yl)-2-Z-methoxyimino-acetamino]-3-cephem-4-carbonsäure

Ausgehend von 2,0 g (10,0 mMol) 2-(5-Amino-1-methyl-1H-1,2,4-triazol-3-yl)-2-methoxyimino-essigsäure und 1,05 g 7β-Amino-3-cephem-4-carbonsäure erhält man analog Beispiel 2a die Titelverbindung vom Rf-Wert 0,30 ($UPC_{12}$-Platten, Wasser/Acetonitril 95:5). IR-Spektrum (Nujol): charakteristische Absorptionsbanden bei 3300; 1755; 1600; 1045 $cm^{-1}$.

Beispiel 11:

7β-[2-(5-Amino-1-methyl-1H-1,2,4-triazol-3-yl)-2-Z-methoxyimino-acetamino]-3-cephem-4-carbonsäure-pivaloyloxymethylester.

Eine Mischung von 2,0 g (4,96 mMol) Natrium-7β-[2-(5-amino-1-methyl-1H-1,2,4-triazol-3-yl)-2-Z-methoxyimino-acetamino]-3-cephem-4-carboxylat in 25 ml N,N-Dimethylformamid wird bei 0° mit 1,12 ml Jodmethylpivalat versetzt und während 60 Minuten bei dieser Temperatur gerührt, dann in 140 ml Phosphatpuffer pH 7,0 gegossen und mit Aethylacetat/Aceton 4:1 extrahiert. Die organischen Extrakte werden mit wässriger Natriumchloridlösung gewaschen, über Magnesiumsulfat getrocknet und im Vakuum eingeengt. Nach Verreiben des Rückstandes mit Diäthyläther erhält man die Titelverbindung vom Rf-Wert 0,75 (Silicagel, sek.-Butanol/Eisessig/Wasser 67:10:23). IR -Spektrum (Nujol): charakteristische Absorptionsbanden bei 3300; 1765; 1735; 1050 $cm^{-1}$.

Beispiel 12:

7β-[2-(5-Amino-1-methyl-1H-1,2,4-triazol-3-yl)-2-Z-methoxyimino-acetamino]-3-acetoxymethyl-3-cephem-4-carbonsäure-diphenylmethylester

Eine Mischung von 1,0 g (1,41 mMol) 7β-[2-(5-Amino-1H-1,2,4-triazol-3-yl)-2-Z-methoxyimino-acetamino]-3-acetoxymethyl-3-cephem-4-carbonsäure-diphenylmethylester in 5 ml Methylenchlorid wird mit 1,0 ml N,O-Bis-(trimethylsilyl)-acetamid versetzt und während 60 Minuten bei Raumtemperatur gerührt, dann auf 0° abgekühlt und mit 0,25 ml Methyljodid versetzt. Das Gemisch wird während 4 Stunden bei 5° gerührt, mit Aethylacetat und Wasser verdünnt. Die organische Phase wird abgetrennt, mit wässriger Natriumchloridlösung gewaschen, über Magnesiumsulfat getrocknet und im Vakuum eingeengt. Nach Chromatographie des Rückstandes an Silicagel mit Chloroform-steigende Anteile Methanol erhält man die Titelverbindung vom Rf-Wert 0,65 (Silicagel, Chloroform/Methanol/Eisessig 85:12:3).

Beispiel 13:

a) Dinatriumsalz von 7β-[2-(5-Amino-1-carboxymethyl-1H-1,2,4-triazol-3-yl)-2-Z-methoxyimino-acetamino]-3-acetoxymethyl-3-cephem-4-carbonsäure.

Eine Mischung von 1,5 g (2,71 mMol) 7β-[2-(5-Amino-1-tert.-butoxycarbonylmethyl-1H-1,2,4-triazol-3-yl)-2-Z-methoxyimino-acetamino]-3-acetoxymethyl-3-cephem-4-carbonsäure in 4 ml Methylenchlorid wird mit 15 ml Trifluoressigsäure versetzt, während 60 Minuten gerührt und dann in 200 ml Diäthyläther/Hexan 1:2 eingerührt. Der Niederschlag wird durch Filtration isoliert, in Methanol gelöst und mit einer methanolischen Natrium-2-äthylhexanoatlösung (ca. 3 N) auf pH 7,0 gestellt. Nach Zugabe von Diäthyläther wird der gebildete Niederschlag abgenutscht und chromatographiert ($UPC_{12}$-Silicagel, Wasser). Man erhält die Titelverbindung vom Rf-Wert 0,60 ($UPC_{12}$-Platten, Wasser/Acetonitril 9:1). IR-Spektrum (Nujol): charakteristische Absorptionsbanden bei 3430; 1765; 1620; 1045 $cm^{-1}$.

Das Ausgangsmaterial wird wie folgt hergestellt:

b) 2-(5-N-Formylamino-1-tert.-butoxycarbonylmethyl-1H-1,2,4-triazol-3-yl)-essigsäureäthylester

Eine Suspension von 3,18 g Natriumhydrid in 10 ml N,N-Dimethylformamid wird mit einer Lösung von 12,0 g (60 mMol) 2-(5-N-Formylamino-1H-1,2,4-triazol-3-yl)-essigsäureäthylester versetzt, während 2 Stunden bei 40° erwärmt, dann auf 0° abgekühlt, mit einer Lösung von 11 ml Bromessigsäure-tert.-butylester in 12 ml N,N-Dimethylformamid versetzt, auf Raumtemperatur aufgewärmt und während 4 Stunden bei dieser Temperatur gerührt. Das Gemisch wird im Vakuum eingeengt, mit Aethylacetat verdünnt, nacheinander mit Wasser, verdünnter Salzsäure und wässriger Natriumchloridlösung gewaschen, über Magnesiumsulfat getrocknet und im Vakuum eingeengt. Nach Verreiben des Rohproduktes mit Diäthyläther erhält man die Titelverbindung vom Rf-Wert 0,65 (Silicagel, Chloroform/Methanol 9:1). IR-Spektrum

(Methylenchlorid): charakteristische Absorptionsbanden bei 3400; 1745; 1710; 1157 $cm^{-1}$.

c) 2-(5-N-Formylamino-1-tert.-butoxycarbonylmethyl-1H-1,2,4-triazol-3-yl)-2-oxo-essigsäureäthylester.

Ausgehend von 3,12 g (10 mMol) 2-(5-N-Formylamino-1-tert.-butoxycarbonylmethyl-1H-1,2,4-triazol-3-yl)-essigsäureäthylester und 3,0 g Selendioxid in 50 ml Dioxan erhält man analog Beispiel 2c die Titelverbindung vom Rf-Wert 0,80 (Silicagel, Chloroform/Methanol/Eisessig 85:12:3). IR—Spektrum (Methylenchlorid): charakteristische Absorptionsbanden bei 3380; 1735; 1700; 1145 $cm^{-1}$.

d) 2-(5-N-Formylamino-1-tert.-butoxycarbonylmethyl-1H-1,2,4-triazol-3-yl)-2-methoxyimino-essigsäureäthylester.

Ausgehend von 1,47 g (4,5 mMol) 2-(5-N-Formylamino-1-tert.-butoxycarbonylmethyl-1H-1,2,4-triazol-3-yl)-2-oxo-essigsäureäthylester, 0,5 g Methoxyamin-Hydrochlorid und 0,55 ml Pyridin in 20 ml 95 %igem Aethanol erhält man analog Beispiel 2d dieTitelverbindung vom Rf-Wert 0,70 (Silicagel, Chloroform/Methanol 9:1); IR—Spektrum (Methylenchlorid): charakteristische Absorptionsbanden bei 3400; 1735; 1700; 1055 $cm^{-1}$.

e) 2-(5-Amino-1-tert.-butoxycarbonylmethyl-1H-1,2,4-triazol-3-yl)-2-methoxyimino-essigsäure

Ausgehend von 2,13 g (6,0 mMol) 2-(5-N-Formylamino-1-tert.-butoxycarbonylmethyl-1H-1,2,4-triazol-3-yl)-2-methoxyimino-essigsäureäthylester und 1,97 g Kaliumhydroxid in 16 ml Wasser und 40 ml 95 %igem Aethanol erhält man analog Beispiel 2e die Titelverbindung vom Rf-Wert 0,20 (Silicagel, sek.-Butanol/Eisessig/Wasser 67:10:23).

IR—Spektrum (Nujol): charakteristische Absorptionsbanden bei 3400; 1735; 1145; 1040 $cm^{-1}$.

f) 7β-[2-(5-Amino-1-tert.-butoxycarbonylmethyl-1H-1,2,4-triazol-3-yl)-2-Z-methoxyimino-acetamino]-3-acetoxymethyl-3-cephem-4-carbonsäure.

Ausgehend von 1,87 g (6,25 mMol) 2-(5-Amino-1-tert.-butoxycarbonylmethyl-1H-1,2,4-triazol-3-yl)-2-methoxyimino-essigsäure und 1,62 g (6,0 mMol) 7β-Amino-3-acetoxymethyl-3-cephem-4-carbonsäure in 40 ml Methylenchlorid erhält man analog Beispiel 2a die Titelverbindung vom Rf-Wert 0,55 (Silicagel, sek.-Butanol/Eisessig/Wasser 67:10:23). IR—Spektrum (Nujol): charakteristische Absorptionsbanden bei 1765; 1145; 1045 $cm^{-1}$.

Beispiel 14:

a) Natriumsalz der 7β-[2-(5-Amino-1-methyl-1H-1,2,4-triazol-3-yl)-2-hydroxyimino-acetamino]-3-acetoxymethyl-3-cephem-4-carbonsäure

Ausgehend von 0,705 g (1,0 mMol) 7β-[2-(5-tert.-Butoxycarbonylamino-1-methyl-1H-1,2,4-triazol-3-yl)-2-hydroxyimino-acetamino]-3-acetoxymethyl-3-cephem-4-carbonsäure-diphenylmethylester in 1,4 ml Methylenchlorid 0,55 ml Anisol und 7,2 ml Trifluoressigsäure erhält man analog Beispiel 1a die Titelverbindung vom Rf-Wert 0,65 ($UPC_{12}$-Platten, Wasser/Acetonitril 9:1) und mit den gleichen IR-Daten wie in Beispiel 8a beschrieben.

Die Ausgangsverbindung kann wie folgt hergestellt werden:

b) 2-[5-(N,N-Di-tert.-butoxycarbonylamino)-1-methyl-1H-1,2,4-triazol-3-yl]-2-oxo-essigsäureäthylester

Ausgehend von 11,52 g (300 mMol) 2-[5-(N,N-Di-tert.-butoxycarbonyl-

amino)-1-methyl-1H-1,2,4-triazol-3-yl]-essigsäureäthylester und 8,30 g Selendioxid in 150 ml Dioxan erhält man analog Beispiel 2c die Titelverbindung vom Rf-Wert 0,55 (Silicagel, Chloroform/Methanol). IR—Spektrum (Methylenchlorid): charakteristische Absorptionsbanden bei 1805; 1735; 1715 $cm^{-1}$.

c) <u>2-(5-tert.-Butoxycarbonylamino-1-methyl-1H-1,2,4-triazol-3-yl)-2-hydroxyimino-essigsäureäthylester.</u>

Ausgehend von 39,8 g (100 mMol) 2-[5-(N,N-Di-tert.-butoxycarbonyl-amino)-1-methyl-1H-1,2,4-triazol-3-yl]-2-oxo-essigsäureäthylester, 13,9 g Hydroxylamin-Hydrochlorid und 17,7 ml Pyridin in 400 ml 95 %igem Aethanol erhält man analog Beispiel 2d die Titelverbindung vom Rf-Wert 0,25 (Silicagel, Chloroform/Methanol 95:5) IR—Spektrum (Methylenchlorid): charakteristische Absorptionsbanden bei 3540; 1740 $cm^{-1}$.

d) <u>2-(5-tert.-Butoxycarbonylamino-1-methyl-1H-1,2,4-triazol-3-yl)-2-hydroxyimino-essigsäure.</u>

Eine Lösung von 7,1 g (170 mMol) 2-(5-tert.-Butoxycarbonylamino-1-methyl-1H-1,2,4-triazol-3-yl)-2-hydroxyimino-essigsäureäthylester in 200 ml Methanol wird mit 25,5 ml 2 N Natriumlauge versetzt und während 3,5 Stunden bei 60° gerührt. Das Reaktionsgemisch wird im Vakuum eingeengt, mit Wasser verdünnt, mit 4 N Salzsäure auf pH 8,5 eingestellt und mit Aethylacetat extrahiert. Die wässrige Phase wird dann mit 2 N Salzsäure auf pH 3,0 eingestellt, mit Aethylacetat extrahiert, über Magnesiumsulfat getrocknet und im Vakuum eingeengt. Nach Verreiben des Rückstandes mit Diäthyläther erhält man die Titelverbindung vom Rf-Wert 0,20 (Silicagel, sek.-Butanol/Eisessig/Wasser 67:10:23). IR—Spektrum (Nujol): charakteristische Absorptionsbanden bei 3400; 1735 $cm^{-1}$.

e) 7β-[2-(5-tert.-Butoxycarbonylamino-1-methyl-1H-1,2,4-triazol-3-yl)-2-hydroxyimino-acetamino]-3-acetoxymethyl-3-cephem-4-carbonsäure-diphenylmethylester.

Ausgehend von 1,34 g (5,0 mMol) 2-(5-tert.-Butoxycarbonylamino-1-methyl-1H-1,2,4-triazol-3-yl)-2-hydroxyimino-essigsäure und 2,20 g (5,0 mMol) 7β-Amino-3-cephem-4-carbonsäure-diphenylmethylester in 25 ml Methylenchlorid erhält man analog Beispiel 3b die Titelverbindung vom Rf-Wert 0,65 (Silicagel, Chloroform/Methanol/Eisessig 85:12:3). IR-Spektrum (Methylenchlorid): charakteristische Absorptionsbanden bei 3400; 1765; 1735 $cm^{-1}$.

Die Titelverbindung kann auch wie folgt hergestellt werden:

Eine Lösung von 0,71 g (1,0 mMol) 7β-[2-(5-tert.-Butoxycarbonylamino)-1-methyl-1H-1,2,4-triazol-3-yl)-acetamino]-3-acetoxymethyl-3-cephem-4-carbonsäure-diphenylmethylester in 5,5 ml Eisessig wird bei 5 - 10° mit 76 mg Natriumnitrit versetzt, während 30 Minuten bei dieser Temperatur gerührt, mit Aethylacetat verdünnt und nacheinander mit Wasser, wässriger Natriumbicarbonat und wässriger Natriumchloridlösung gewaschen. Man trocknet über Magnesiumsulfat und engt im Vakuum ein. Der Rückstand enthält die Titelverbindung vom Rf-Wert 0,65 (Silicagel, Chloroform/Methanol/Eisessig 85:12:3).

Beispiel 15:

7β-[2-(5-Amino-1-methyl-1H-1,2,4-triazol-3-yl)-2-Z-methoxyimino-acetamino]-3-acetoxymethyl-3-cephem-4-carbonsäure-diphenylmethylester.

Eine Lösung von 0,461 g (1,05 mMol) 7β-[2-(5-tert.Butoxycarbonylamino-1-methyl-1H-1,2,4-triazol-3-yl)-2-hydroxyimino-acetamino]-3-acetoxymethyl-3-cephem-4-carbonsäure-diphenylmethylester in 20 ml

Methanol wird tropfenweise solange mit einer kalten Lösung (0°) von Diazomethan in Diäthyläther versetzt, bis die Umsetzung beendet ist, und bei 0° - 5° während 45 Minuten gerührt. Das Reaktionsgemisch wird im Vakuum eingeengt und der Rückstand durch Chromatographie an Silicagelplatten mit Chloroform/Methanol (9:1) gereinigt. Man erhält die Titelverbindung vom Rf-Wert 0,65 (Silicagel, Chloroform/Methanol/Eisessig 85:12:3). IR—Spektrum (Methylenchlorid): charakteristische Absorptionsbanden bei 3300; 1765; 1740; 1045 $cm^{-1}$. Das Produkt ist mit dem von Beispiel 12 identisch.

Beispiel 16:

a) Natriumsalz der 7β-[2-(5-Amino-1H-1,2,4-triazol-3-yl)-2-Z-methoxyimino-acetamino]-3-acetoxymethyl-3-cephem-4-carbonsäure

Ein Gemisch von 0,585 g Phosphorpentachlorid und 0,463 g (2,5 mMol) 2-(5-Amino-1H-1,2,4-triazol-3-yl)-2-methoxyimino-essigsäure in 10 ml Methylenchlorid wird während 30 Minuten bei -10° gerührt (Lösung 1). Andererseits wird eine Mischung von 0,544 g (2,0 mMol) 7β-Amino-3-acetoxymethyl-3-cephem-4-carbonsäure, 4,2 g Trimethylsilylacetamid und 10 ml Methylenchlorid während 15 Minuten bei Raumtemperatur gerührt und anschliessend auf -10° abgekühlt (Lösung 2). Lösung 1 und Lösung 2 werden vereinigt. Die Mischung wird bei 0° während 3 Stunden gerührt und anschliessend auf gesättigte Natriumbicarbonatlösung gegossen. Die wässrige Phase wird abgetrennt, mit wässriger Salzsäure auf pH 4,5 gestellt und chromatographiert (XAD-2-Harz, Wasser). Die das Produkt enthaltenden Fraktionen werden eingeengt, in Methanol gelöst und mit einer methanolischen Natrium-2-äthylhexanoatlösung (ca. 3 N) auf pH 7,0 gestellt. Nach Zugabe von Diäthyläther wird der gebildete Niederschlag abgenutscht und chromatographiert ($UPC_{12}$-Platten, Wasser/Acetonitril 6:1). Man erhält die Titelverbindung vom Rf-Wert 0,60 ($UPC_{12}$-Platten, Wasser/Acetonitril 6:1).

Das Produkt ist mit dem von Beispiel 9 identisch.

Das Ausgangsmaterial kann wie folgt hergestellt werden:

b) 2-(5-Amino-1H-1,2,4-triazol-3-yl)-essigsäureäthylester.

Ausgehend von 48,5 g (0,263 Mol) 3-Cyanoimino-3-äthoxy-propionsäureäthylester und 10 ml 95 %igem Hydrazin in 88 ml Acetonitril erhält man analog Beispiel 1aa die Titelverbindung vom Rf-Wert 0,25 (Silicagel, Chloroform/Methanol 95:5) F. 151 - 153°. IR-Spektrum (Nujol): charakteristische Absorptionsbanden bei 3350; 1735; 1655 $cm^{-1}$.

c) 2-(5-N-Formylamino-1H-1,2,4-triazol-3-yl)-essigsäureäthylester.

25 ml Essigsäureanhydrid werden zu 130 ml Ameisensäure zugetropft und die Lösung wird während 30 Minuten bei 40° gerührt, auf Raumtemperatur abgekühlt und nach Zugabe von 10,8 g (63,55 mMol) 5-Amino-1H-1,2,4-triazol-3-yl-essigsäureäthylester während 4 Stunden bei Raumtemperatur gerührt. Das Reaktionsgemisch wird im Vakuum eingeengt und der Rückstand mit Diäthyläther verrieben. Man erhält die Titelverbindung vom Rf-Wert 0,65 (Silicagel, Chloroform/Methanol/Eisessig 85:12:3) F. 208 - 210°. IR-Spektrum (Nujol): charakteristische Absorptionsbanden bei 3290; 1732; 1685; 1595 $cm^{-1}$.

d) 2-(5-N-Formylamino-1H-1,2,4-triazol-3-yl)-2-oxo-essigsäureäthylester

Ausgehend von 8,70 g (43,9 mMol) 2-(5-N-Formylamino-1H-1,2,4-triazol-3-yl)-essigsäureäthylester und 12,2 g Selendioxid in 435 ml Dioxan erhält man analog Beispiel 2c die Titelverbindung vom Rf-Wert 0,45 (Silicagel; Chloroform/Methanol/Eisessig 85:12:3). IR-Spektrum (Nujol): charakteristische Absorptionsbanden bei 3250; 1735; 1710; 1675; 1600 $cm^{-1}$.

e) 2-(5-N-Formylamino-1H-1,2,4-triazol-3-yl)-2-methoxyimino-essigsäureäthylester

Ausgehend von 12,0 g (56,56 mMol) 2-(5-N-Formylamino-1H-1,2,4-triazol-3-yl)-2-oxo-essigsäureäthylester, 6,0 g Methoxyamin-Hydrochlorid und 6,5 ml Pyridin in 200 ml 95 %igem Aethanol erhält man analog Beispiel 2d die Titelverbindung vom Rf-Wert 0,45 (Silicagel, Chloroform/Methanol 9:1) F. 184 - 187°. IR.-Spektrum (Nujol): charakteristische Absorptionsbanden bei 3300; 1735; 1045 $cm^{-1}$.

f) 2-(5-Amino-1H-1,2,4-triazol-3-yl)-2-methoxyimino-essigsäure

Ein Gemisch von 4,6 g (19,0 mMol) 2-(5-N-Formylamino-1H-1,2,4-triazol-3-yl)-2-methoxyimino-essigsäureäthylester und 49 ml wässrige Salzsäure werden während 1 Stunde bei 100° gerührt, abgekühlt und mit Chloroform extrahiert. Die wässrigen Extrakte werden eingeengt, der Rückstand wird in Methanol gelöst, mit Propylenoxid versetzt und während 1 Stunde bei 0° gerührt. Der Niederschlag wird abgenutscht, mit Diäthyläther gewaschen und getrocknet. Man erhält die Titelverbindung vom Rf-Wert 0,15 (Silicagel, sek.-Butanol/Eisessig/Wasser 67:10:23). IR-Spektrum (Nujol): charakteristische Absorptionsbanden bei 3300; 1685; 1040 $cm^{-1}$.

Beispiel 17:

a) Natriumsalz der 7β-[2-(5-Amino-1-methylsulfonyl-1H-1,2,4-triazol-3-yl)-2-Z-methoxyimino-acetamino]-3-acetoxymethyl-3-cephem-4-carbonsäure

Ausgehend von 1,0 g (1,5 mMol) 7β-[2-(5-Amino-1-methylsulfonyl-1H-1,2,4-triazol-3-yl)-2-Z-methoxyimino-acetamino]-3-acetoxymethyl-3-cephem-4-carbonsäure-diphenylmethylester in 2,5 ml Methylenchlorid, 0,73 ml Anisol und 10 ml Trifluoressigsäure erhält man analog Beispiel 3a die Titelverbindung vom Rf-Wert 0,35 ($UPC_{12}$-Platten, Wasser/

Acetonitril 6:1). IR-Spektrum (Nujol): charakteristische Absorptionsbanden bei 3350; 1765; 1640; 1160 $cm^{-1}$.

Das Ausgangsmaterial kann wie folgt hergestellt werden:

b) <u>2-(5-Amino-1-methylsulfonyl-1H-1,2,4-triazol-3-yl)-2-methoxyimino-essigsäureäthylester</u>

Eine Lösung von 2,40 g (10,0 mMol) 2-(5-N-Formylamino-1H-1,2,4-triazol-3-yl)-2-methoxyimino-essigsäureäthylester (Beispiel 16e) in 15 ml Methylenchlorid wird mit 3 ml N,O-Bis-(trimethylsilyl)-acetamid versetzt, während 30 Minuten bei Raumtemperatur gerührt, anschliessend auf -10° abgekühlt und mit 0,24 ml Methansulfonylchlorid versetzt. Das Gemisch wird während 4 Stunden bei 0° und anschliessend während 16 Stunden bei Raumtemperatur gerührt, dann mit Aethylacetat verdünnt, nacheinander mit wässriger Natriumbicarbonatlösung, Wasser und wässriger Natriumchloridlösung gewaschen, über Magnesiumsulfat getrocknet und im Vakuum eingeengt. Nach Chromatographie des Rückstandes (Silicagel, Chloroform-steigende Anteile Methanol) erhält man die Titelverbindung vom Rf-Wert 0,60 (Silicagel, Chloroform/Methanol 9:1). IR-Spektrum (Methylenchlorid) charakteristische Absorptionsbanden bei 1735; 1155; 1045 $cm^{-1}$.

c) <u>2-(5-Amino-1-methylsulfonyl-1H-1,2,4-triazol-3-yl)-2-methoxyimino-essigsäure</u>

Ausgehend von 3,03 g (10,37 mMol) 2-(5-Amino-1-methylsulfonyl-1H-1,2,4-triazol-3-yl)-2-methoxyimino-essigsäureäthylester in 24 ml wässriger Salzsäure erhält man analog Beispiel 16f die Titelverbindung vom Rf-Wert 0,20 (Silicagel, sek.-Butanol/Eisessig/Wasser 67:10:23). IR-Spektrum (Nujol): charakteristische Absorptionsbanden bei 3330; 1160; 1045 $cm^{-1}$.

d) 7β-[2-(5-Amino-1-methylsulfonyl-1H-1,2,4-triazol-3-yl)-2-Z-methoxyimino-acetamino]-3-acetoxymethyl-3-cephem-4-carbonsäure-diphenylmethylester

Ausgehend von 1,05 g (4,0 mMol) 2-(5-Amino-1-methylsulfonyl-1H-1,2,4-triazol-3-yl)-2-methoxyimino-essigsäure und 1,76 g (4,0 mMol) 7β-Amino-3-acetoxymethyl-3-cephem-4-carbonsäure-diphenylmethylester in 20 ml Methylenchlorid erhält man analog Beispiel 3b die Titelverbindung vom Rf-Wert 0,50 (Silicagel, Chloroform/Methanol/Eisessig 85:12:3). IR-Spektrum (Nujol): charakteristische Absorptionsbanden bei 1765; 1155; 1045 $cm^{-1}$.

Beispiel 18:

a) Natriumsalz der 7β-[2-(5-Amino-1-phenyl-1H-1,2,4-triazol-3-yl)-2-Z-methoxyimino-acetamino]-3-cephem-4-carbonsäure

Ausgehend von 1,04 g (4,0 mMol) 2-(5-Amino-1-phenyl-1H-1,2,4-triazol-3-yl)-2-methoxyimino-essigsäure und 0,42 g 7β-Amino-3-cephem-4-carbonsäure erhält man analog Beispiel 2a die Titelverbindung vom Rf-Wert 0,50 ($UPC_{12}$-Platten, Wasser/Acetonitril 6:1). IR-Spektrum (Nujol): charakteristische Absorptionsbanden bei 3300; 1765; 1620; 1045 $cm^{-1}$.

Das Ausgangsmaterial kann wie folgt hergestellt werden:

b) 2-(5-Amino-1-phenyl-1H-1,2,4-triazol-3-yl)-essigsäureäthylester

Ausgehend von 20 g (108 mMol) 3-Cyanoimino-3-äthoxy-propionsäureäthylester und 15,3 g Phenylhydrazin in 40 ml Acetonitril erhält man analog Beispiel 1aa die Titelverbindung vom Rf-Wert o,80 (Silicagel, Chloroform/Methanol/Eisessig 85:12:3), F. 92 - 93°. IR-Spektrum (Methylenchlorid): charakteristische Absorptionsbanden bei 3460; 1740; 1620 $cm^{-1}$.

c) 2-(5-N,N-Di-tert.-butoxycarbonylamino-1-phenyl-1H-1,2,4-triazol-3-yl)-essigsäureäthylester und 2-(5-N-tert.-Butoxycarbonylamino-1-phenyl-1H-1,2,4-triazol-3-yl)-essigsäureäthylester

Ausgehend von 10 g (40,6 mMol) 2-(5-Amino-1-phenyl-1H-1,2,4-triazol-3-yl)-essigsäureäthylester, 26,6 g BOC-Anhydrid und 0,4 g N,N-Dimethylaminopyridin erhält man analog Beispiel 8c den 2-(5-N,N-Di-tert.-butoxycarbonylamino-1-phenyl-1H-1,2,4-triazol-3-yl)-essigsäureäthylester vom Rf-Wert 0,70 (Silicagel, Toluol/Aethylacetat 1:1); IR-Spektrum (Methylenchlorid): charakteristische Absorptionsbanden bei 1810; 1735 $cm^{-1}$ und den 2-(5-N-tert.-Butoxycarbonylamino-1-phenyl-1H-1,2,4-triazol-3-yl)-essigsäureäthylester vom Rf-Wert 0,40 (Silicagel, Toluol/Aethylacetat 1:1); IR-Spektrum (Methylenchlorid): charakteristische Absorptionsbanden bei 3400; 1735 $cm^{-1}$.

d) 2-(5-N-tert.- Butoxycarbonylamino-1-phenyl-1H-1,2,4-triazol-3-yl)-2-oxo-essigsäureäthylester

Ausgehend von 15,3 g (43,8 mMol) 2-(5-N-tert.-Butoxycarbonylamino-1-phenyl-1H-1,2,4-triazol-3-yl)-essigsäureäthylester und 12,9 g Selendioxid in 290 ml Dioxan erhält man analog Beispiel 2c die Titelverbindung vom Rf-Wert 0,20 (Silicagel, Toluol/Aethylacetat 1:1). IR-Spektrum (Methylenchlorid): charakteristische Absorptionsbanden bei: 1740; 1705 $cm^{-1}$.

e) 2-(5-Amino-1-phenyl-1H-1,2,4-triazol-3-yl)-2-methoxyimino-essigsäureäthylester

Ausgehend von 8,75 g (24,25 mMol) 2-(5-N-tert-Butoxycarbonylamino-1-phenyl-1H-1,2,4-triazol-3-yl)-2-Oxo-essigsäureäthylester, 2,55 g Methoxyamin-Hydrochlorid und 2,7 ml Pyridin in 90 ml 95 %igem Aethanol erhält man analog Beispiel 2d die Titelverbindung vom Rf-Wert

0,75 (Silicagel, Chloroform/Methanol/Eisessig 85:12:3). IR-Spektrum (Methylenchlroid): charakteristische Absorptionsbanden bei 3400; 1735; 1050 $cm^{-1}$.

f) 2-(5-Amino-1-phenyl-1H-1,2,4-triazol-3-yl)-2-methoxyiminoessigsäure

Ausgehend von 4,0 g (13,83 mMol) 2-(5-Amino-1-phenyl-1H-1,2,4-triazol-3-yl)-2-methoxyimino-essigsäureäthylester und 4,5 g Kaliumhydroxid in 35 ml Wasser und 85 ml 95 %igem Aethanol erhält man analog Beispiel 2e die Titelverbindung vom Rf-Wert 0,25 (Silicagel, sek.-Butanol/Eisessig/Wasser 67:10:23). IR-Spektrum (Nujol): charakteristische Absorptionsbanden bei 3500; 3330; 1600; 1035 $cm^{-1}$.

Beispiel 19: In analoger Weise, wie in den Beispielen 1 bis 18 beschrieben, kann man unter Verwendung der geeigneten Ausgangsstoffe die folgenden Verbindungen herstellen:

7β-[2-(5-Amino-1-methyl-1H-1,2,4-triazol-3-yl)-2-Z-methoxyimino-acetamino]-3-(1-carboxymethyletrazol-5-yl-thiomethyl)-3-cephem-4-carbonsäure, Dinatriumsalz; IR (Nujol): 3400; 1770;1605; 1045 $cm^{-1}$;

7β-[2-(5-Amino-1H-1,2,4-triazol-3-yl)-2-Z-methoxyimino]-3-(1-methyltetrazol-5-yl-thiomethyl)-3-cephem-4-carbonsäure, Natriumsalz; IR (Nujol): 3370; 1770 $cm^{-1}$;

7β-[2-(5-Amino-1H-1,2,4-triazol-3-yl)-2-Z-methoxyimino-acetamino]-3-(1-carboxymethyltetrazol-5-yl-thiomethyl)-3-cephem-4-carbonsäure, Dinatriumsalz; IR (Nujol): 1770; 1610 $cm^{-1}$;

7β-[2-(5-Amino-1H-1,2,4-triazol-3-yl)-2-Z-methoxyimino-acetamino]-3-(4-carbamoylpyridiniomethyl)-3-cephem-4-carboxylat; IR (Nujol): 3400; 1765; 1620; 1045 $cm^{-1}$;

7β-[2-(5-Amino-1H-1,2,4-triazol-3-yl)-2-Z-methoxyimino-acetamino]-3-cephem-4-carbonsäure, Natriumsalz; IR (Nujol): 3350; 1765; 1660; 1605 $cm^{-1}$;

7β-[2-(5-Amino-1-methyl-1H-1,2,4-triazol-3-yl)-2-hydroxyiminoacetamino]-3-(1-methyltetrazol-5-yl-thiomethyl)-3-cephem-4-carbonsäure, Natriumsalz; IR (Nujol): 3400; 1765 $cm^{-1}$;

7β-[2-(5-Amino-1-methyl-1H-1,2,4-triazol-3-yl)-2-hydroxyimino-acetamino]-3-(1-carboxymethyltetrazol-5-yl-thiomethyl)-3-cephem-4-carbonsäure, Dinatriumsalz; IR (Nujol): 3400; 1765; 1610 $cm^{-1}$;

7β-[2-(5-Amino-1-methyl-1H-1,2,4-triazol-3-yl)-2-hydroxyimino-acetamino]-3-(4-carbamoylpyridiniomethyl)-3-cephem-4-carboxylat IR (Nujol): 3425; 1770 $cm^{-1}$;

7β-[2-(5-Amino-1-methyl-1H-1,2,4-triazol-3-yl)-2-hydroxyimino-acetamino]-3-cephem-4-carbonsäure, Natriumsalz; IR (Nujol): 1770; 1655 $cm^{-1}$;

7β-[2-(5-Amino-1-methyl-1H-1,2,4-triazol-3-yl)-2-Z-(2-carboxy-2-propoxyimino)-acetamino]-3-(4-carbamoylpyridiniomethyl)-3-cephem-4-carboxylat, Natriumsalz; IR (Nujol): 3330; 1765; 1620 $cm^{-1}$;

7β-[2-(5-Amino-1H-1,2,4-triazol-3-yl)-2-Z-methoxyimino-acetamino]-3-(4-hydroxymethylpyridiniomethyl)-3-cephem-4-carboxylat; IR (Nujol): 3400; 1765; 1040 $cm^{-1}$;

7β-[2-(5-Amino-1H-1,2,4-triazol-3-yl)-2-Z-methoxyimino-acetamino]-3-cephem-4-carbonsäure-pivaloyloxymethylester; IR (Nujol): 1765; 1735 $cm^{-1}$;

4β-[2-(5-Amino-1H-1,2,4-triazol-3-yl)-2-Z-(2-carboxy-2-propoxyimino)-acetamino]-3-pyridiniomethyl-4-carboxylat, Natriumsalz; IR (Nujol): 1765; 1620 $cm^{-1}$;

7β-[2-(5-Amino-1H-1,2,4-triazol-3-yl)-2-hydroxyimino-acetamino]-3-(1-methyltetrazol-5-yl-thiomethyl)-3-cephem-4-carbonsäure, Natriumsalz; IR (Nujol): 3330; 1765 $cm^{-1}$;

7β-[2-(5-Amino-1H-1,2,4-triazol-3-yl)-2-hydroxyimino-acetamino]-3-(1-carboxymethyltetrazol-5-yl-thiomethyl)-3-cephem-4-carbonsäure, Dinatriumsalz; IR (Nujol): 3300; 1765; 1220 $cm^{-1}$;

7β-[2-(5-Amino-1-methylsulfonyl-1H-1,2,4-triazol-3-yl)-2-Z-methoxyimino-acetamino]-3-(1-methyltetrazol-5-yl-thiomethyl)-3-cephem-4-carbonsäure, Natriumsalz; IR (Nujol): 3330; 1765; 1160 $cm^{-1}$;
7β-[2-(5-Amino-1-methylsulfonyl-1H-1,2,4-triazol-3-yl)-2-Z-methoxy-imino-acetamino]-3-(4-carbamoylpyridiniomethyl)-3-cephem-4-carboxylat; IR (Nujol): 1765; 1620 $cm^{-1}$;
7β-[2-(5-Amino-1-methylsulfonyl-1H-1,2,4-triazol-3-yl)-2-Z-methoxy-imino-acetamino]-3-(1-carboxymethyltetrazol-5-yl-thiomethyl)-3-cephem-4-carbonsäure, Dinatriumsalz; IR (Nujol): 1765; 1620; 1045 $cm^{-1}$;
7β-[2-(5-Amino-1-methylsulfonyl-1H-1,2,4-triazol-3-yl)-2-hydroxy-imino-acetamino]-3-(1-carboxymethyltetrazol-5-yl-thiomethyl)-3-cephem-4-carbonsäure, Dinatriumsalz; IR (Nujol): 3350; 1767; 1625 $cm^{-1}$;
7β-[2-(5-Amino-1-phenyl-1H-1,2,4-triazol-3-yl)-2-Z-methoxyimino-acetamino]-3-(4-carbamoylpyridiniomethyl)-3-cephem-4-carboxylat; IR (Nujol): 1765; 1050 $cm^{-1}$;
7β-[2-(5-Amino-1-phenyl-1H-1,2,4-triazol-3-yl)-2-Z-methoxyimino-acetamino]-3-(1-methyltetrazol-5-yl-thiomethyl)-3-cephem-4-carbonsäure, Natriumsalz; IR(Nujol): 1765; 1615; 1045 $cm^{-1}$;
7β-[2-(5-Amino-1-phenyl-1H-1,2,4-triazol-3-yl)-2-Z-methoxyimino-acetamino]-3-acetoxymethyl-3-cephem-4-carbonsäure, Natriumsalz; IR (Nujol): 3330; 1765 $cm^{-1}$;

7β-[2-(5-Amino-1-phenyl-1H-1,2,4-triazol-3-yl)-2-hydroxyimino-acetamino]-2-(1-carboxymethyltetrazol-5-yl-thiomethyl)-3-cephem-4-carbonsäure, Dinatriumsalz; IR (Nujol): 3335; 1760; 1620 $cm^{-1}$;

7β-[2-(5-Amino-1-carboxymethyl-1H-1,2,4-triazol-3-yl)-2-Z-methoxy-imino-acetamino]-3-(1-methyltetrazol-5-yl-thiomethyl)-3-cephem-4-carbonsäure, Dinatriumsalz: IR (Nujol): 3350; 1765 $cm^{-1}$;

7β-[2-(5-Amino-1-carboxymethyl-1H-1,2,4-triazol-3-yl)-2-Z-methoxy-imino-acetamino)-3-(1-carboxymethyltetrazol-5-yl-thiomethyl)-3-cephem-4-carbonsäure, Trinatriumsalz; IR (Nujol): 3330; 1765 $cm^{-1}$;

7β-[2-(5-Amino-1-carboxymethyl-1H-1,2,4-triazol-3-yl)-2-Z-methoxyimino-acetamino]-3-(4-hydroxymethylpyridiniomethyl)-3-cephem-4-carboxylat, Natriumsalz; IR (Nujol); 3350; 1765; 1050 $cm^{-1}$;

7β-[2-(5-Amino-1-carboxymethyl-1H-1,2,4-triazol-3-yl)-2-hydroxyimino-acetamino]-3-acetoxymethyl-3-cephem-4-carbonsäure, Dinatriumsalz; IR (Nujol): 1765; 1735 $cm^{-1}$;

7β-[2-(5-Amino-1-carboxymethyl-1H-1,2,4-triazol-3-yl)-2-hydroxyimino-acetamino]-3-(1-methyltetrazol-5-yl-thiomethyl)-3-cephem-4-carbonsäure, Dinatriumsalz; IR (Nujol): 1765; 1620 $cm^{-1}$;

7β-[2-(5-Amino-1-acetyl-1H-1,2,4-triazol-3-yl)-2-hydroxyimino-acetamino]-3-acetoxymethyl-3-cephem-4-carbonsäure, Natriumsalz; IR (Nujol): 1765; 1735; 1630 $cm^{-1}$.

Beispiel 20: Trockenampullen oder Vials, enthaltend 0,5 g Wirksubstanz, z.B. 7β-[2-(5-Amino-1-methyl-1H-1,2,4-triazol-3-yl)-2-Z-methoxyimino-acetamino]-2-(4-carbamoylpyridiniomethyl)-3-cephem-4-carboxylat, können z.B. wie folgt hergestellt werden:

Zusammensetzung (für eine Ampulle oder Vial)

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml-Ampullen oder 5ml-Vials eingefüllt, und diese verschlossen und geprüft.

Patentansprüche (für alle benannten Länder ausser Oesterreich):

1. 7β-Aminotriazolylacetylamino-3-cephem-4-carbonsäureverbindungen der Formel

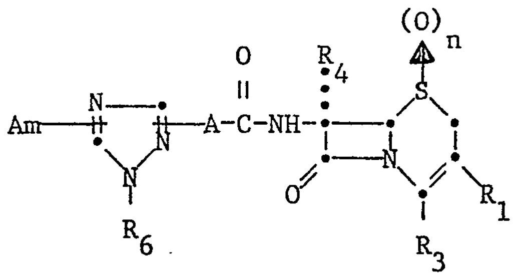

(I) ,

worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$ oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und Salze von solchen Verbindungen, die salzbildende Gruppen aufweisen.

2. Verbindungen der Formel I, worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$ oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes

Carbamoyl bedeutet, $R_6$ Wasserstoff, einen gegebenenfalls substituierten Kohlenwasserstoffrest oder Acyl darstellt und Am Amino ist, und Salze von solchen Verbindungen, die salzbildende Gruppen aufweisen.

3. Verbindungen der Formel I gemäss Anspruch 1, worin n 0 oder 1 ist, $R_1$ Wasserstoff, Niederalkoxy, Phenylniederalkoxy, Halogen, Niederalkanoyloxy, Niederalkansulfonyloxy, Arensulfonyloxy, Heterocyclylthio, worin Heterocyclyl über ein Ringkohlenstoffatom an die Thiogruppe gebundenes, unsubstituiertes oder durch Niederalkyl, das Diniederalkylamino, Carboxy oder Sulfo als Substituenten enthalten kann, substituiertes Thiadiazolyl, Oxadiazolyl, Triazolyl oder Tetrazolyl bedeutet, oder einen Rest $-CH_2-R_2$ darstellt, worin $R_2$ Wasserstoff, Hydroxy, Carboxy, Niederalkoxycarbonyl, Niederalkanoyloxy, Carbamoyloxy, N-Niederalkylcarbamoyloxy, Heterocyclylthio, worin Heterocyclyl einen über ein Ringkohlenstoffatom an die Thiogruppe gebundenen monoheterocyclischen Rest mit 1 bis 4 Ringstickstoffatomen und gegebenenfalls einem weiteren Ringheteroatom der Gruppe Sauerstoff und Schwefel darstellt, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell gesättigt und/oder unsubstituiert oder durch Niederalkyl, durch Carboxy, Sulfo, Diniederalkylamino, Niederalkanoylamino, Hydroxy oder Halogen substituiertes Niederalkyl, ferner durch Cycloalkyl, Phenyl, Benzyl, Carboxy, Carbamoyl oder N-Niederalkylcarbamoyl substituiert sein können, oder Ammonio, wie Triniederalkylammonio oder unsubstituiertes oder durch Niederalkyl, Hydroxyniederalkyl, Carboxyniederalkyl, Sulfoniederalkyl, Carboxy, Carbamoyl, N-Niederalkylcarbamoyl, Halogen, Sulfo, Niederalkanoyl, Hydroxy oder Cyano substituiertes Pyrazolio, Triazolio, Pyridinio, Pyrimidinio, Pyridazinio, Thiazolinio oder Chinolinio, bedeutet, $R_3$ Carboxyl oder in physiologisch spaltbarer Form verestertes Carboxyl darstellt, $R_4$ für Wasserstoff oder Niederalkoxy steht, A eine Gruppe $-CH_2-$,

-C(=O)- oder -C(=N-O-$R_5$)- ist, worin $R_5$ Wasserstoff, Niederalkyl, Cycloalkyl, durch Phenyl, Niederalkoxy, Carboxy, Amino oder Diniederalkylamino substituiertes Niederalkyl oder Cycloalkyl, ferner Cycloalkenyl, Niederalkenyl, Carbamoyl oder N-Niederalkylcarbamoyl bedeutet, $R_6$ Wasserstoff, Niederalkyl, durch Hydroxy, Niederalkoxy, Carboxy, Niederalkoxycarbonyl, Amino, Niederalkylamino, Diniederalkylamino, Carbamoyl, Halogen, Sulfo, Niederalkylthio, Cyano oder Aminosulfinyl substituiertes Niederalkyl, ferner Niederalkenyl, Cycloalkyl, Cycloalkenyl, Phenyl, Phenylniederalkyl, Niederalkylsulfonyl, unsubstituiertes oder durch Niederalkyl oder Halogen substituiertes Phenylsulfonyl oder Phenylniederalkylsulfonyl, Niederalkanoyl, Niederalkoxycarbonyl, Carbamoyl oder N-Niederalkylcarbamoyl darstellt und Am Amino oder Diniederalkylamino-niederalkylidenamino ist, sowie Salze, insbesondere pharmazeutisch verwendbare Salze von solchen Verbindungen der Formel I, die salzbildende Gruppen aufweisen.

4. Verbindungen der Formel I gemäss Anspruch 2, worin $R_6$ Wasserstoff, Niederalkyl, durch Hydroxy, Niederalkoxy, Carboxy, Niederalkoxycarbonyl, Amino, Niederalkylamino, Diniederalkylamino, Carbamoyl,Cyano, Halogen, Sulfo, Niederalkylthio oder Aminosulfinyl substituiertes Niederalkyl, ferner Niederalkenyl, Cycloalkyl, Cycloalkenyl, Phenyl, Phenylniederalkyl, Niederalkanoyl, Niederalkoxycarbonyl, Carbamoyl oder N-Niederalkylcarbamoyl darstellt, Am Amino ist und n, $R_1$, $R_3$, $R_4$ und A die in Anspruch 3 angegebenen Bedeutungen haben, sowie Salze, insbesondere pharmazeutisch verwendbare Salze von solchen Verbindungen der Formel I, die salzbildende Gruppen aufweisen.

5. Verbindungen der Formel I gemäss Anspruch 1, worin n 0 oder 1 ist, $R_1$ Wasserstoff, Niederalkoxy, z.B. Methoxy, Phenylniederalkoxy, z.B. Benzyloxy, Halogen, z.B. Chlor, Heterocyclylthio, worin Heterocyclyl über ein Ringkohlenstoffatom an die Thiogruppe gebundenes, unsubstituiertes oder durch Niederalkyl, z.B. Methyl, substituiertes Thiadiazolyl, z.B. 1,3,4-Thiadiazol-2-yl, oder Oxadiazolyl, z.B. 1,3,4-Oxadiazol-2-yl, bedeutet, oder einen Rest $-CH_2-R_2$ darstellt, worin

$R_2$ Wasserstoff, Niederalkanoyloxy, z.B. Acetyloxy, Carbamoyloxy, N-Niederalkylcarbamoyloxy, z.B. N-Methylcarbamoyloxy, unsubstituiertes oder durch Niederalkyl, z.B. Methyl, Carboxyniederalkyl, z.B. Carboxymethyl oder 2-Carboxyäthyl, Sulfoniederalkyl, z.B. Sulfomethyl oder 2-Sulfoäthyl, Diniederalkylaminoniederalkyl, z.B. 2-Dimethylaminoäthyl, oder Niederalkanoylamino, z.B. Acetylamino, substituiertes Tetrazol-5-ylthio, unsubstituiertes oder durch Niederalkyl, z.B. Methyl, oder Carboxyniederalkyl, z.B. Carboxymethyl oder 2-Carboxyäthyl, substituiertes 1,3,4-Thiadiazol-2-ylthio, unsubstituiertes oder durch Niederalkyl, z.B. Methyl, substituiertes 1,3,4-Oxadiazol-2-ylthio, unsubstituiertes oder durch Niederalkyl, z.B. Methyl, oder Carboxyniederalkyl, z.B. Carboxymethyl, substituiertes 1,2,4-Triazol-3-ylthio, unsubstituiertes oder durch Hydroxy substituiertes Pyridazin-6-ylthio, oder unsubstituiertes oder durch Hydroxy substituiertes Pyrimidin-2-ylthio, oder Triniederalkylammonio, z.B. Trimethyl- oder Triäthylammonio, durch Niederalkyl, z.B. Methyl, N-substituiertes 1,2,3-Triazolio, durch Niederalkyl, z.B. Methyl, oder Carboxyniederalkyl, z.B. Carboxymethyl, N-substituiertes Pyrazolio, oder unsubstituiertes oder durch Niederalkyl, z.B. Methyl, Carboxy, Carbamoyl, Hydroxyniederalkyl, z.B. Hydroxymethyl, Carboxyniederalkyl, z.B. Carboxymethyl oder 2-Carboxyäthyl, Sulfoniederalkyl, z.B. Sulfomethyl, Halogen, z.B. Brom oder Chlor, oder Hydroxy substituiertes Pyridinio, bedeutet, $R_3$ Carboxyl oder in physiologisch spaltbarer Form verestertes Carboxyl, wie Niederalkanoyloxymethoxycarbonyl, z.B. Pivaloyloxy- oder Acetyloxymethoxycarbonyl, 2-Phthalidyloxycarbonyl oder Niederalkoxycarbonyloxymethoxycarbonyl, z.B. Aethoxycarbonyloxymethoxycarbonyl, darstellt, $R_4$ Wasserstoff oder, vorzugsweise wenn -A- eine Gruppe $-CH_2-$ ist, Methoxy bedeutet, A eine Gruppe $-CH_2-$, -C(=O)- oder $-C(=N-O-R_5)-$ ist, worin $R_5$ Wasserstoff, Niederalkyl, z.B. Methyl, Phenylniederalkyl, z.B. Benzyl, Carboxyniederalkyl, z.B. 2-Carboxy-2-propyl, Carboxycycloalkyl, z.B. 1-Carboxy-1-cyclopropyl, Carbamoyl, N-Niederalkylcarbamoyl, z.B. N-Methylcarbamoyl, oder Niederalkenyl, z.B. Allyl, bedeutet, und worin die Gruppe $-C(=N-O-R_5)-$

vorzugsweise in der syn-(oder Z-) Konfiguration vorliegt, wobei -A- mit der 3-Stellung des 1,2,4-Triazolringes verknüpft ist, $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. Dimethylaminoäthyl, Niederalkoxycarbonyl, z.B. Methoxy- oder Aethoxycarbonyl, Carbamoyl, Niederalkenyl, z.B. Allyl, Phenyl, Phenylniederalkyl, z.B. Benzyl oder Diphenylmethyl, oder Niederalkylsulfonyl, z.B. Methylsulfonyl, darstellt und Am Amino ist, sowie Salze, insbesondere pharmazeutisch verwendbare Salze von solchen Verbindungen der Formel I, die salzbildende Gruppen aufweisen.

6. Verbindungen der Formel I gemäss Anspruch 2, worin $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. Dimethylaminoäthyl, Niederalkoxycarbonyl, z.B. Methoxy- oder Aethoxycarbonyl, Carbamoyl, Niederalkenyl, z.B. Allyl, Phenyl oder Phenylniederalkyl, z.B. Benzyl oder Diphenylmethyl, darstellt, und n, $R_1$, $R_3$, $R_4$, A und Am die in Anspruch 5 angegebenen Bedeutungen haben, sowie Salze, insbesondere pharmazeutisch verwendbare Salze von solchen Verbindungen der Formel I, die salzbildende Gruppen aufweisen.

7. Verbindungen der Formel I gemäss einem der Ansprüche 1 oder 2, worin n für 0 steht, $R_1$ Wasserstoff, Niederalkoxy, z.B. Methoxy, oder einen Rest $-CH_2-R_2$ darstellt, worin $R_2$ Niederalkanoyloxy, z.B. Acetyloxy, in 1-Stellung durch Niederalkyl, z.B. Methyl, Carboxyniederalkyl, z.B. Carboxymethyl oder 2-Carboxyäthyl, Sulfoniederalkyl, z.B. Sulfomethyl oder 2-Sulfoäthyl, oder Diniederalkylaminoniederalkyl, z.B. 2-Dimethylaminoäthyl, substituiertes 1H-Tetrazol-5-ylthio, in 2-Stellung durch Niederalkyl, z.B. Methyl, oder Carboxyniederalkyl, z.B. Carboxymethyl, substituiertes 1,3,4-Thiadiazol-5-ylthio, 2-Niederalkylpyrazolio, z.B. 2-Methylpyrazolio, 3-Niederalkyl-1-(1,2,3-triazolio), z.B. 3-Methyl-1-(1,2,3-triazolio), Carboxypyridinio, z.B. 3- oder 4-Carboxypyridinio, Carbamoylpyridinio, z.B. 3- oder 4-Carbamoylpyridinio, Hydroxyniederalkylpyridino, z.B. 4-Hydroxymethylpyridinio, Carboxyniederalkylpyridinio, z.B. 3-Carboxymethylpyridinio, oder Halogenpyridinio, z.B. 3- oder 4-Brompyridinio, bedeutet, $R_3$ Carboxyl oder Niederalkanoyloxymethoxycarbonyl, z.B. Pivaloyloxymethoxycarbonyl, oder Niederalkoxycarbonyloxymethoxycarbonyl, z.B. Aethoxycarbonyloxymethoxycarbonyl, darstellt, $R_4$ für Wasserstoff oder, vorzugsweise wenn A eine Gruppe $-CH_2-$ ist, für Methoxy steht, A eine Gruppe $-CH_2-$ oder $-C(=N-O-R_5)-$ ist, worin $R_5$ Wasserstoff, Niederalkyl, z.B. Methyl, Carboxyniederalkyl, z.B. 2-Carboxy-2-propyl, Carbamoyl oder N-Niederalkylcarbamoyl, z.B. N-Methylcarbamoyl, bedeutet und worin die Gruppe $-C(=N-O-R_5)-$ in syn-(oder Z-)Kontiguration vorliegt, wobei -A- mit der 3-Stellung des 1,2,4-Triazolringes verknüpft ist, $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. 2-Dimethylaminoäthyl, oder Phenylniederalkyl, z.B. Benzyl oder Diphenylmethyl, darstellt und Am Amino ist, sowie Salze, insbesondere pharmazeutisch verwendbare Salze von solchen Verbindungen der Formel I, die salzbildende Gruppen aufweisen.

8. 7β-[2-(5-Amino-1-methyl-1H-1,2,4-triazol-3-yl)-2-Z-methoxyimino-acetamino]-3-(4-hydroxymethylpyridiniomethyl)-3-cephem-4-carboxylat und pharmazeutisch verwendbare Salze davon gemäss Patentanspruch 1.

9. 7β-[2-(5-Amino-1-carboxymethyl-1H-1,2,4-triazol-3-yl)-2-Z-methoxyimino-acetamino]-3-acetoxymethyl-3-cephem-4-carbonsäure und pharmazeutisch verwendbare Salze davon gemäss Patentanspruch 1.

10. 7β-[2-(5-Amino-1-methyl-1H-1,2,4-triazol-3-yl)-2-Z-methoxyimino-acetamino]-3-cephem-4-carbonsäure-pivaloyloxymethylester und pharmazeutisch verwendbare Salze davon gemäss Patentanspruch 1.

11. 7β-[2-(5-Amino-1-methyl-1H-1,2,4-triazol-3-yl)-acetamino]-3-acetoxymethyl-3-cephem-4-carbonsäure und pharmazeutisch verwendbare Salze davon gemäss Patentanspruch 2.

12. 7β-[2-(5-Amino-1-methyl-1H-1,2,4-triazol-3-yl)-2-Z-methoxyimino-acetamino]-3-acetoxymethyl-3-cephem-4-carbonsäure und pharmazeutisch verwendbare Salze davon gemäss Patentanspruch 2.

13. 7β-[2-(5-Amino-1-methyl-1H-1,2,4-triazol-3-yl)-2-Z-methoxy-imino-acetamino]-3-(1-methyltetrazol-5-yl-thiomethyl)-3-cephem-4-carbonsäure und pharmazeutisch verwendbare Salze davon gemäss Patentanspruch 2.

14. 7β-[2-(5-Amino-1-methyl-1H-1,2,4-triazol-3-yl)-2-Z-methoxyimino-acetamino]-3-(4-carbamoylpyridiniomethyl)-3-cephem-4-carboxylat und pharmazeutisch verwendbare Salze davon gemäss Patentanspruch 2.

15. 7β-[2-(5-Amino-1-methyl-1H-1,2,4-triazol-3-yl)-2-Z-methoxyimino-acetamino]-3-cephem-4-carbonsäure und pharmazeutisch verwendbare Salze davon gemäss Patentanspruch 2.

16. 7β-[2-(5-Amino-1-methyl-1H-1,2,4-triazol-3-yl)-2-hydroxyimino-acetamino]-3-acetoxymethyl-3-cephem-4-carbonsäure und pharmazeutisch verwendbare Salze davon gemäss Patentanspruch 2.

17. 7β-[2-(5-Amino-1H-1,2,4-triazol-3-yl)-2-Z-methoxyimino-acetamino]-3-acetoxymethyl-3-cephem-4-carbonsäure und pharmazeutisch verwendbare Salze davon gemäss Patentanspruch 2.

18. Pharmazeutische Präparate enthaltend Verbindungen der Formel I gemäss Patentanspruch 1 oder pharmazeutisch verwendbare Salze von solchen Verbindungen mit salzbildenden Gruppen.

19. Pharmazeutische Präparate enthaltend Verbindungen der Formel I gemäss Patentanspruch 2 oder pharmazeutisch verwendbare Salze von solchen Verbindungen mit salzbildenden Gruppen.

20. Verbindungen der Formel I gemäss Patentanspruch 1 oder pharmazeutisch verwendbare Salze von solchen Verbindungen mit salzbildenden Gruppen zur Anwendung bei der Behandlung von bakteriellen Infektionen des menschlichen und tierischen Körpers.

21. Verbindungen der Formel I gemäss Patentanspruch 2 oder pharmazeutisch verwendbare Salze von solchen Verbindungen mit salzbildenden Gruppen zur Anwendung bei der Behandlung von bakteriellen Infektionen des menschlichen und tierischen Körpers.

22. Verwendung von Verbindungen der Formel I gemäss Patentanspruch 1 und pharmazeutisch verwendbarer Salze von solchen Verbindungen mit salzbildenden Gruppen zur Herstellung von pharmazeutischen Präparaten.

23. Verwendung von Verbindungen der Formel I gemäss Patentanspruch 2 und pharmazeutisch verwendbarer Salze von solchen Verbindungen mit salzbildenden Gruppen zur Herstellung von pharmazeutischen Präparaten.

24. Verwendung von Verbindungen der Formel I gemäss Patentanspruch 1 und pharmazeutisch verwendbarer Salze von solchen Verbindungen mit salzbildenden Gruppen als antibiotisch wirksame Verbindungen.

25. Verwendung von Verbindungen der Formel I gemäss Patentanspruch 2 und pharmazeutisch verwendbarer Salze von solchen Verbindungen mit salzbildenden Gruppen als antibiotisch wirksame Verbindungen.

26. Verfahren zur Herstellung von Verbindungen der Formel

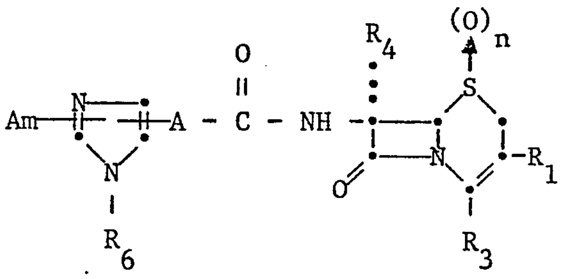

(I),

worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$ oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und Salzen von solchen Verbindungen der Formel I, die salzbildende Gruppen aufweisen, dadurch gekennzeichnet, dass man

a) eine Verbindung der Formel

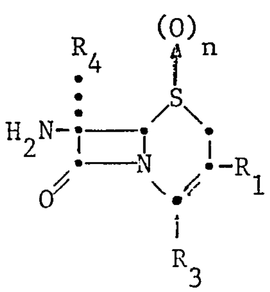

(II),

worin n, $R_1$, $R_3$ und $R_4$ die unter Formel I angegebenen Bedeutungen haben und worin die 7β-Aminogruppe gegebenenfalls durch eine die Acylierungsreaktion erlaubende Gruppe substituiert ist und gegebenenfalls vorhandene funktionelle Gruppen geschützt sein können, durch Umsetzung mit einem den Acylrest einer Carbonsäure der Formel

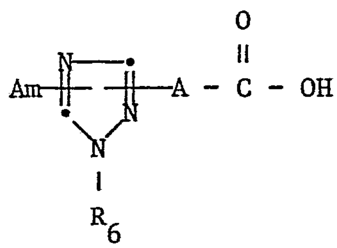

(III)

einführenden Acylierungsmittel, worin A, $R_6$ und Am die unter Formel I angegebenen Bedeutungen haben und vorhandene funktionelle Gruppen gegebenenfalls geschützt sind, acyliert oder

b) zur Herstellung einer Verbindung der Formel I, worin $R_1$ den Rest der Formel $-CH_2-R_2$ darstellt, worin $R_2$ Ammonio oder Heterocyclylthio ist, eine Verbindung der Formel

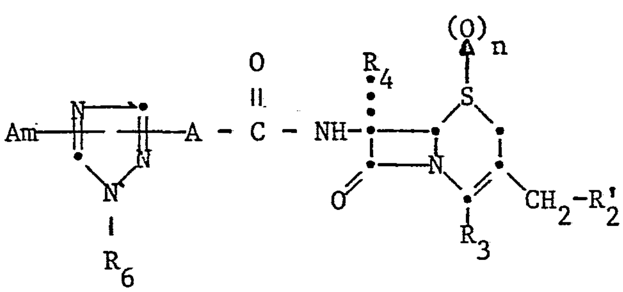

(IV),

worin n, $R_3$, $R_4$, A, $R_6$ und Am die unter Formel I angegebenen Bedeutungen haben, $R_2'$ einen durch nucleophile Reaktion ersetzbaren Rest darstellt und worin gegebenenfalls vorhandene funktionelle Gruppen in geschützter Form vorliegen können, mit einem tertiären Amin oder einem Mercaptan der Formel $R_2$-H behandelt, oder

c) zur Herstellung einer Verbindung der Formel I, worin $R_4$ Niederalkoxy bedeutet, in eine Verbindung der Formel

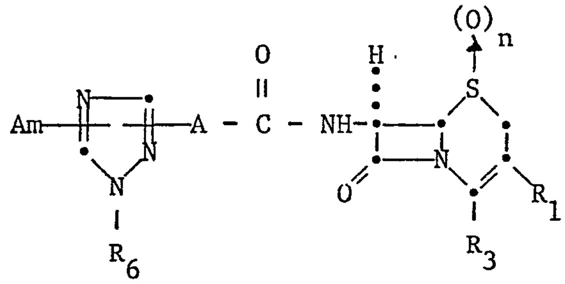

(V),

worin n, $R_1$, $R_3$, A, $R_6$ und Am die unter Formel I angegebenen Bedeutungen haben und worin funktionelle Gruppen vorzugsweise geschützt sind, in 7α-Stellung eine Niederalkoxygruppe einführt, oder

d) eine 2-Cephemverbindung der Formel

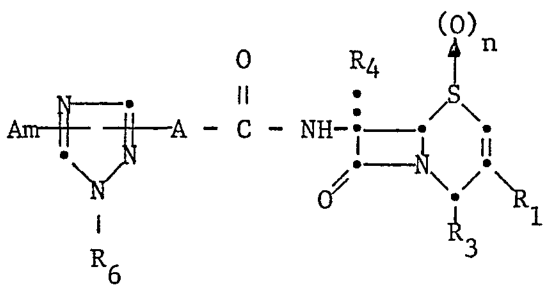

(VI),

worin n, $R_1$, $R_3$, $R_4$, A, $R_6$ und Am die unter Formel I angegebenen Bedeutungen haben und worin gegebenenfalls vorhandene funktionelle Gruppen in geschützter Form vorliegen können, zur entsprechenden 3-Cephemverbindung isomerisiert, oder

e) zur Herstellung einer Verbindung der Formel I, worin A eine Gruppe $-C(=N-O-R_5)-$ ist, eine Verbindung der Formel

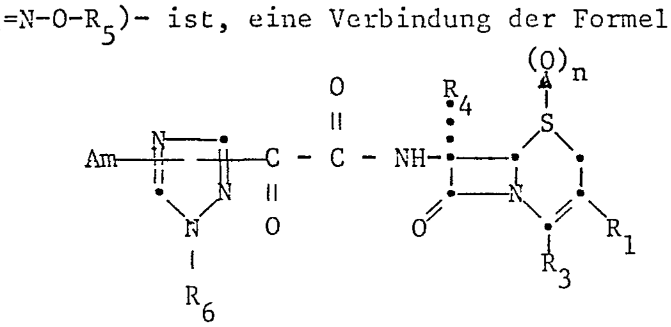

(VII),

worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Hydroxylamin der Formel $H_2N-O-R_5$ umsetzt, oder

f) zur Herstellung einer Verbindung der Formel I, worin A eine Gruppe $-C(=N-O-R_5)-$ ist, worin $R_5$ gegebenenfalls substituiertes Niederalkyl oder Cycloalkyl, Cycloalkenyl, Niederalkenyl oder gegebenenfalls N-substituiertes Carbamoyl bedeutet, eine Verbindung der Formel

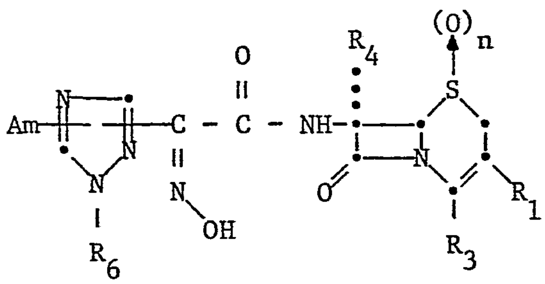
(VIII),

worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem den Rest $R_5$ einführenden Mittel behandelt, oder

g) zur Herstellung einer Verbindung der Formel I, worin A eine Gruppe $-C(=N-O-R_5)-$ ist, worin $R_5$ Wasserstoff bedeutet, eine Verbindung der Formel

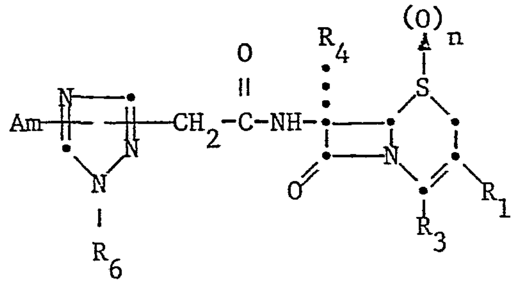
(IX),

worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Nitrosierungsmittel behandelt, oder

h) zur Herstellung einer Verbindung der Formel I, worin $R_3$ eine geschützte Carboxylgruppe darstellt, in einer Verbindung der Formel

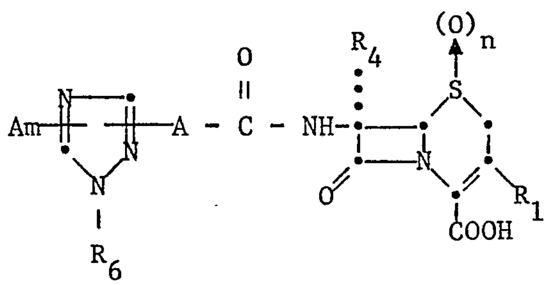

(X),

worin n, $R_1$, $R_4$, A, $R_6$ und Am die unter Formel I angegebenen Bedeutungen haben und worin gegebenenfalls vorhandene funktionelle Gruppen in geschützter Form vorliegen können, die 4-Carboxylgruppe in eine geschützte Carboxylgruppe überführt, oder

i) zur Herstellung einer Verbindung der Formel I, worin A für eine Gruppe $-CH_2-$ und Am für Amino steht, eine Verbindung der Formel

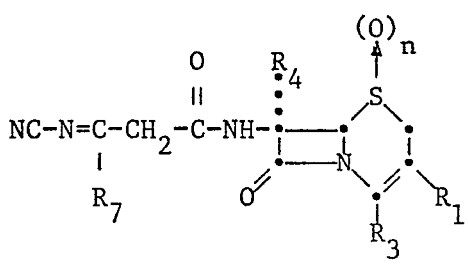

(XII),

worin $R_7$ für veräthertes Hydroxy steht, n, $R_1$, $R_3$ und $R_4$ die unter Formel I angegebenen Bedeutungen haben und worin gegebenenfalls vorhandene funktionelle Gruppen in Resten $R_1$ und/oder $R_3$ in geschützter Form vorliegen können, mit einem Hydrazin der Formel $R_6$-NH-$NH_2$ (XI) umsetzt, oder

j) zur Herstellung einer Verbindung der Formel I, worin $R_6$ einen gegebenenfalls substituierten Kohlenwasserstoffrest, organisches Sulfonyl oder Acyl darstellt, in eine Verbindung der Formel

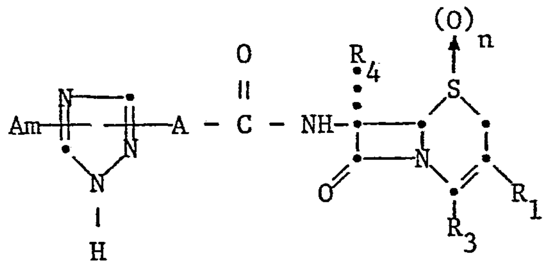

(XIII),

worin n, $R_1$, $R_3$, $R_4$, A und Am die unter Formel I angegebenen Bedeutungen haben und worin funktionelle Gruppen erforderlichenfalls in geschützter Form vorliegen, den Rest $R_6$ einführt, und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin n 0 bedeutet, in eine Verbindung der Formel I überführt, worin n 1 bedeutet, oder eine Verbindung der Formel I, worin n 1 bedeutet, in eine Verbindung der Formel I überführt, worin n 0 bedeutet, und/oder in einer Verbindung der Formel I in geschützter Form vorliegende funktionelle Gruppen in die freien funktionellen Gruppen überführt und/oder ein erhältliches Salz in die freie Verbindung der Formel (I) oder in ein anderes Salz überführt, und/oder eine erhältliche freie Verbindung der Formel (I) mit einer salzbildenden Gruppe in ein Salz überführt und/oder ein erhältliches Gemisch von isomeren Verbindungen der Formel I in die einzelnen Isomeren auftrennt.

27. Verfahren zur Herstellung von Verbindungen der Formel I, worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$

oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest oder Acyl darstellt und Am Amino ist, und Salzen von solchen Verbindungen der Formel I, die eine salzbildende Gruppierung aufweisen, dadurch gekennzeichnet, dass man

a) eine Verbindung der Formel II, worin n, $R_1$, $R_3$ und $R_4$ die unter Formel I angegebenen Bedeutungen haben und worin die 7β-Aminogruppe gegebenenfalls durch eine die Acylierungsreaktion erlaubende Gruppe substituiert ist und gegebenenfalls vorhandene funktionelle Gruppen geschützt sein können, durch Umsetzung mit einem den Acylrest einer Carbonsäure der Formel III einführenden Acylierungsmittel, worin A, $R_6$ und Am die unter Formel I angegebenen Bedeutungen haben und vorhandene funktionelle Gruppen gegebenenfalls geschützt sind, acyliert oder

b) zur Herstellung einer Verbindung der Formel I, worin $R_1$ den Rest der Formel $-CH_2-R_2$ darstellt, worin $R_2$ Ammonio oder Heterocyclylthio ist, eine Verbindung der Formel IV, worin n, $R_3$, A, $R_4$, $R_6$ und Am die unter Formel I angegebenen Bedeutungen haben, $R_2'$ einen durch nucleophile Reaktion ersetzbaren Rest darstellt und worin gegebenenfalls vorhandene funktionelle Gruppen in geschützter Form vorliegen können, mit einem tertiären Amin oder Mercaptan der Formel $R_2-H$ behandelt, oder

c) zur Herstellung einer Verbindung der Formel I, worin $R_4$ Niederalkoxy bedeutet, in eine Verbindung der Formel V, worin n, $R_1$, $R_3$, A, $R_6$ und Am die unter Formel I angegebenen Bedeutungen haben und worin funktionelle Gruppen vorzugsweise geschützt sind, in 7α-Stellung eine Niederalkoxygruppe einführt, oder

d) eine 2-Cephemverbindung der Formel VI, worin n, $R_1$, $R_3$, $R_4$, A, $R_6$ und Am die unter Formel I angegebenen Bedeutungen haben und worin gegebenenfalls vorhandene funktionelle Gruppen in geschützter Form vorliegen können, zur entsprechenden 3-Cephemverbindung isomerisiert, oder

e) zur Herstellung einer Verbindung der Formel I, worin A eine Gruppe $-C(=N-O-R_5)-$ ist, eine Verbindung der Formel VII, worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Hydroxylamin der Formel $H_2N-O-R_5$ umsetzt, oder

f) zur Herstellung einer Verbindung der Formel I, worin A eine Gruppe $-C(=N-O-R_5)-$ ist, worin $R_5$ gegebenenfalls substituiertes Niederalkyl oder Cycloalkyl, Cycloalkenyl, Niederalkenyl oder gegebenenfalls N-substituiertes Carbamoyl bedeutet, eine Verbindung der Formel VIII, worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem den Rest $R_5$ einführenden Mittel behandelt, oder

g) zur Herstellung einer Verbindung der Formel I, worin A eine Gruppe $-C(=N-O-R_5)-$ ist, worin $R_5$ Wasserstoff bedeutet, eine Verbindung der Formel IX, worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Nitrosierungsmittel behandelt, oder

h) zur Herstellung einer Verbindung der Formel I, worin $R_3$ eine geschützte Carboxylgruppe darstellt, in einer Verbindung der Formel X, worin n, $R_1$, $R_4$, A, $R_6$ und Am die unter Formel I angegebenen Bedeutungen haben und worin gegebenenfalls vorhandene funktionelle Gruppen in geschützter Form vorliegen können, die 4-Carboxylgruppe in eine geschützte Carboxylgruppe überführt, oder

i) zur Herstellung einer Verbindung der Formel I, worin A für eine Gruppe $-CH_2-$ steht, eine Verbindung der Formel XII, worin $R_7$ für veräthertes Hydroxy steht, n, $R_1$, $R_3$ und $R_4$ die unter Formel I angegebenen Bedeutungen haben und worin gegebenenfalls vorhandene funktionelle Gruppen in Resten $R_1$ und/oder $R_3$ in geschützter Form vorliegen können, mit einem Hydrazin der Formel $R_6$-NH-$NH_2$ (XI) umsetzt, oder

j) zur Herstellung einer Verbindung der Formel I, worin $R_6$ einen gegebenenfalls substituierten Kohlenwasserstoffrest oder Acyl darstellt, in eine Verbindung der Formel XIII, worin n, $R_1$, $R_3$, $R_4$, A und Am die unter Formel I angegebenen Bedeutungen haben und worin funktionelle Gruppen erforderlichenfalls in geschützter Form vorliegen, den Rest $R_6$ einführt, und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin n 0 bedeutet, in eine Verbindung der Formel I überführt, worin n 1 bedeutet, oder eine Verbindung der Formel I, worin n 1 bedeutet, in eine Verbindung der Formel I überführt, worin n 0 bedeutet, und/oder in einer Verbindung der Formel I in geschützter Form vorliegende funktionelle Gruppen in die freien funktionellen Gruppen überführt und/oder ein erhältliches Salz in die freie Verbindung der Formel (I) oder in ein anderes Salz überführt, und/oder eine erhältliche freie Verbindung der Formel (I) mit einer salzbildenden Gruppe in ein Salz überführt und/oder ein erhältliches Gemisch von isomeren Verbindungen der Formel I in die einzelnen Isomeren auftrennt.

28. Die nach dem Verfahren von Anspruch 26 erhältlichen Verbindungen.

29. Die nach dem Verfahren von Anspruch 27 erhältlichen Verbindungen.

30. Verbindungen der Formel

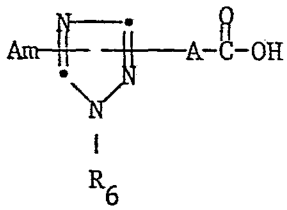

(III),

worin A eine Gruppe $-CH_2-$, $-C(=O)-$ oder $-C(=N-O-R_5)$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und ihre zur Acylierung geeigneten, reaktionsfähigen funktionellen Derivate, mit Ausnahme der 5-Amino-1,2,4-triazol-3-yl-essigsäure.

31. Verbindungen der Formel

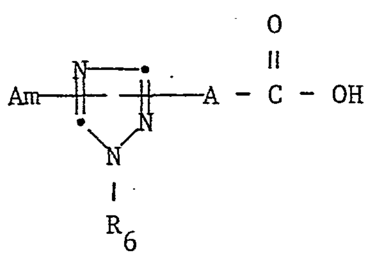

(III),

worin A eine Gruppe $-CH_2-$, $-C(=O)-$ oder $-C(=N-O-R_5)$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest oder Acyl darstellt und Am eine gegebenenfalls geschützte Aminogruppe ist, und ihre zur Acylierung geeigneten, reaktionsfähigen funktionellen Derivate, mit Ausnahme der 5-Amino-1,2,4-triazol-3-yl-essigsäure.

32. Verfahren zur Herstellung von Verbindungen der Formel

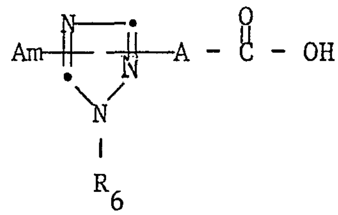

(III)

worin A eine Gruppe $-CH_2-$, $-C(=O)-$ oder $-C(=N-O-R_5)$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und ihrer zur Acylierung geeigneten, reaktionsfähigen funktionellen Derivate, dadurch gekennzeichnet, dass man

a) zur Herstellung einer Verbindung der Formel III, worin A eine Gruppe $-CH_2-$ und Am Amino darstellt, eine Verbindung der Formel

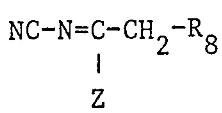

(XIV),

worin $R_8$ für Wasserstoff, Cyano oder geschütztes Carboxy und Z für veräthertes Hydroxy steht, mit einer Verbindung der Formel $R_6-NH-NH_2$, worin $R_6$ die unter Formel III angegebene Bedeutung hat, umsetzt und in einer erhaltenen Verbindung der Formel

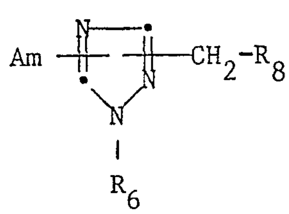

(XV),

worin $R_8$ Wasserstoff oder Cyano bedeutet, Wasserstoff oder Cyano durch Carboxy ersetzt, oder

b) zur Herstellung einer Verbindung der Formel III, worin A eine Gruppe -C(=O)- darstellt, eine Verbindung der Formel

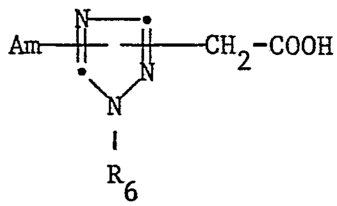

(IIIa),

worin $R_6$ die unter Formel III angegebene Bedeutung hat, Am eine substituierte Aminogruppe ist und die Carboxygruppe gegebenenfalls in geschützter Form vorliegt, mit einem Oxidationsmittel behandelt, oder

c) zur Herstellung einer Verbindung der Formel III, worin A eine Gruppe -C(=NOH)- darstellt, eine Verbindung der Formel

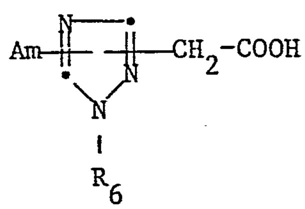

(IIIa),

worin $R_6$ die unter Formel III angegebene Bedeutung hat, Am eine substituierte Aminogruppe ist und die Carboxygruppe gegebenenfalls in geschützter Form vorliegt, mit einem Nitrosierungsmittel behandelt, oder

d) zur Herstellung einer Verbindung der Formel III, worin Am Amino und A eine Gruppe $-CH_2-$ darstellt, wobei eine solche Verbindung von 5-Amino-1,2,4-triazol-3-ylessigsäure verschieden ist, eine Verbindung der Formel

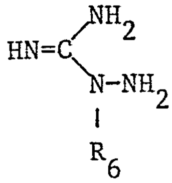

(XVI),

worin $R_6$ die unter Formel III angegebene Bedeutung hat, oder ein Salz davon mit Malonsäure umsetzt, oder

e) zur Herstellung einer Verbindung der Formel III, worin A eine Gruppe $-C(=NOR_5)-$ ist, wobei $R_5$ die unter Formel III angegebene Bedeutung hat, eine Verbindung der Formel

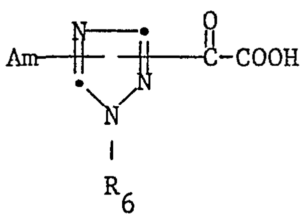

(IIIb),

worin $R_6$ und Am die unter Formel III angegebenen Bedeutungen haben, und die Carboxygruppe gegebenenfalls in geschützter Form vorliegt, mit einem Hydroxylamin der Formel $H_2N-OR_5$ umsetzt, und gewünschtenfalls in einer erfindungsgemäss erhältlichen Verbindung der Formel III eine geschützte Aminogruppe Am in eine freie oder in eine andere geschützte Aminogruppe Am überführt, oder eine freie Aminogruppe Am in eine substituierte Aminogruppe Am überführt, und/oder eine geschützte Carboxygruppe in die freie Carboxygruppe oder in ein reaktionsfähiges funktionelles Derivat davon überführt, und/oder, wenn gewünscht, in einer erfindungsgemäss erhältlichen Verbindung der Formel III den Rest $R_6$ in einen anderen Rest $R_6$ überführt, und/oder ein erhältliches Gemisch von isomeren Verbindungen der Formel III in die einzelnen Isomeren auftrennt.

33. Verfahren zur Herstellung von Verbindungen der Formel III, worin $R_6$ Wasserstoff, einen gegebenenfalls substituierten Kohlenwasserstoffrest oder Acyl darstellt, Am eine gegebenenfalls geschützte Aminogruppe ist und A die in Anspruch 32 angegebene Bedeutung hat, und ihrer zur Acylierung geeigneten, reaktionsfähigen funktionellen Derivate, dadurch gekennzeichnet, dass man

a) zur Herstellung einer Verbindung der Formel III, worin A eine Gruppe $-CH_2-$ darstellt, eine Verbindung der Formel XIV, worin $R_8$ für Wasserstoff, Cyano oder geschütztes Carboxy und Z für veräthertes Hydroxy steht, mit einer Verbindung der Formel $R_6-NH-NH_2$, worin $R_6$ die unter Formel III angegebene Bedeutung hat, umsetzt und in einer erhaltenen Verbindung der Formel XV, worin $R_8$ Wasserstoff oder Cyano bedeutet, Wasserstoff oder Cyano durch Carboxy ersetzt, oder

b) zur Herstellung einer Verbindung der Formel III, worin A eine Gruppe -C(=O)- darstellt, eine Verbindung der Formel IIIa, worin $R_6$ die unter Formel III angegebene Bedeutung hat, Am eine geschützte Aminogruppe ist und die Carboxygruppe gegebenenfalls in geschützter Form vorliegt, mit einem Oxidationsmittel behandelt, oder

c) zur Herstellung einer Verbindung der Formel III, worin A eine Gruppe -C(=NOH)- darstellt, eine Verbindung der Formel IIIa, worin $R_6$ die unter Formel III angegebene Bedeutung hat, Am eine geschützte Aminogruppe ist und die Carboxygruppe gegebenenfalls in geschützter Form vorliegt, mit einem Nitrosierungsmittel behandelt, oder

d) zur Herstellung einer Verbindung der Formel III, worin A eine Gruppe $-C(=NOR_5)-$ ist, wobei $R_5$ die unter Formel III angegebene Bedeutung hat, eine Verbindung der Formel IIIb, worin $R_6$ und Am die unter Formel III angegebenen Bedeutungen haben und die Carboxygruppe gegebenenfalls in geschützter Form vorliegt, mit einem Hydroxylamin der Formel $H_2N-OR_5$ umsetzt, und gewünschtenfalls in einer erfindungsgemäss erhältlichen Verbindung der Formel III eine geschützte Aminogruppe Am in eine freie oder in eine andere geschützte Aminogruppe Am überführt, oder eine freie Aminogruppe Am in eine geschützte Aminogruppe Am überführt, und/oder eine geschützte Carboxygruppe in die freie Carboxygruppe oder in ein reaktionsfähiges funktionelles Derivat

davon überführt, und/oder, wenn gewünscht, in einer erfindungsgemäss erhältlichen Verbindung der Formel III den Rest $R_6$ in einen anderen Rest $R_6$ überführt, und/oder ein erhältliches Gemisch von isomeren Verbindungen der Formel III in die einzelnen Isomeren auftrennt.

34. Die nach dem Verfahren gemäss Anspruch 32 erhältlichen Verbindungen.

35. Die nach dem Verfahren gemäss Anspruch 33 erhältlichen Verbindungen.

Patentansprüche (für Oesterreich):

1. Verfahren zur Herstellung von Verbindungen der Formel

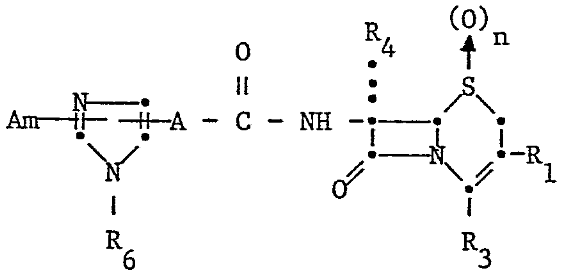

(I),

worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$ oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und Salzen von solchen Verbindungen der Formel I, die salzbildende Gruppen aufweisen, dadurch gekennzeichnet, dass man

a) eine Verbindung der Formel

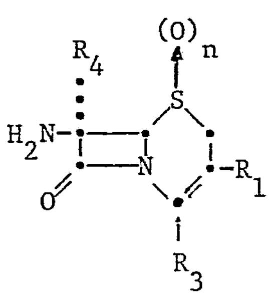

(II),

worin n, $R_1$, $R_3$ und $R_4$ die unter Formel I angegebenen Bedeutungen haben und worin die 7β-Aminogruppe gegebenenfalls durch eine die Acylierungsreaktion erlaubende Gruppe substituiert ist und gegebenenfalls vorhandene funktionelle Gruppen geschützt sein können, durch Umsetzung mit einem den Acylrest einer Carbonsäure der Formel

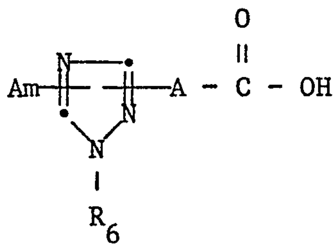

(III)

einführenden Acylierungsmittel, worin A, $R_6$ und Am die unter Formel I angegebenen Bedeutungen haben und vorhandene funktionelle Gruppen gegebenenfalls geschützt sind, acyliert oder

b) zur Herstellung einer Verbindung der Formel I, worin $R_1$ den Rest der Formel $-CH_2-R_2$ darstellt, worin $R_2$ Ammonio oder Heterocyclylthio ist, eine Verbindung der Formel

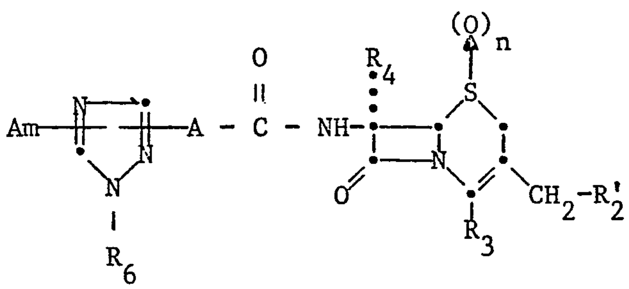

(IV),

worin n, $R_3$, $R_4$, A, $R_6$ und Am die unter Formel I angegebenen Bedeutungen haben, $R_2'$ einen durch nucleophile Reaktion ersetzbaren Rest darstellt und worin gegebenenfalls vorhandene funktionelle Gruppen in geschützter Form vorliegen können, mit einem tertiären Amin oder einem Mercaptan der Formel $R_2-H$ behandelt, oder

0070803

c) zur Herstellung einer Verbindung der Formel I, worin $R_4$ Nieder-alkoxy bedeutet, in eine Verbindung der Formel

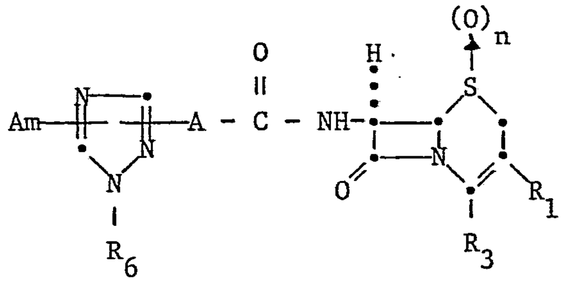

(V),

worin n, $R_1$, $R_3$, A, $R_6$ und Am die unter Formel I angegebenen Bedeutungen haben und worin funktionelle Gruppen vorzugsweise geschützt sind, in 7α-Stellung eine Niederalkoxygruppe einführt, oder

d) eine 2-Cephemverbindung der Formel

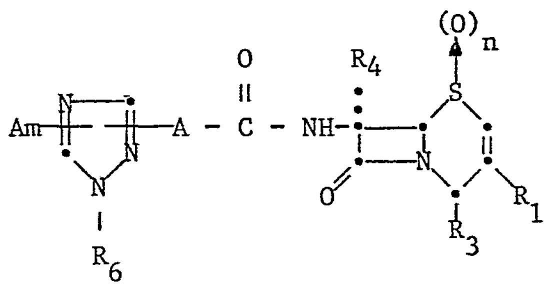

(VI),

worin n, $R_1$, $R_3$, $R_4$, A, $R_6$ und Am die unter Formel I angegebenen Bedeutungen haben und worin gegebenenfalls vorhandene funktionelle Gruppen in geschützter Form vorliegen können, zur entsprechenden 3-Cephemverbindung isomerisiert, oder

e) zur Herstellung einer Verbindung der Formel I, worin A eine Gruppe $-C(=N-O-R_5)-$ ist, eine Verbindung der Formel

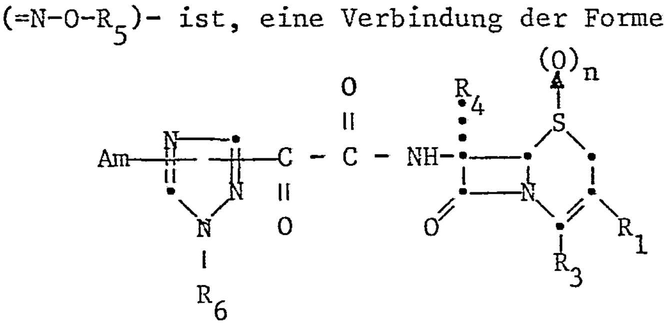

(VII),

worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Hydroxylamin der Formel $H_2N-O-R_5$ umsetzt, oder

f) zur Herstellung einer Verbindung der Formel I, worin A eine Gruppe $-C(=N-O-R_5)-$ ist, worin $R_5$ gegebenenfalls substituiertes Niederalkyl oder Cycloalkyl, Cycloalkenyl, Niederalkenyl oder gegebenenfalls N-substituiertes Carbamoyl bedeutet, eine Verbindung der Formel

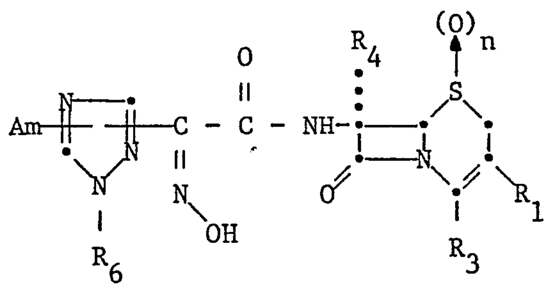

(VIII),

worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem den Rest $R_5$ einführenden Mittel behandelt, oder

g) zur Herstellung einer Verbindung der Formel I, worin A eine Gruppe $-C(=N-O-R_5)-$ ist, worin $R_5$ Wasserstoff bedeutet, eine Verbindung der Formel

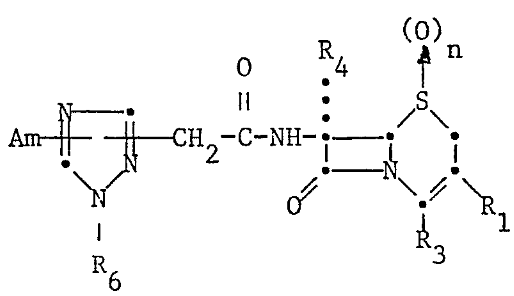

(IX),

worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Nitrosierungsmittel behandelt, oder

h) zur Herstellung einer Verbindung der Formel I, worin $R_3$ eine geschützte Carboxylgruppe darstellt, in einer Verbindung der Formel

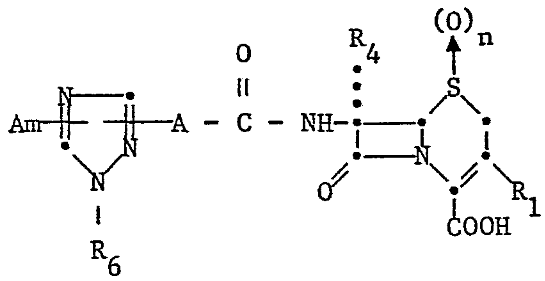

(X),

worin n, $R_1$, $R_4$, A, $R_6$ und Am die unter Formel I angegebenen Bedeutungen haben und worin gegebenenfalls vorhandene funktionelle Gruppen in geschützter Form vorliegen können, die 4-Carboxylgruppe in eine geschützte Carboxylgruppe überführt, oder

i) zur Herstellung einer Verbindung der Formel I, worin A für eine Gruppe $-CH_2-$ und Am für Amino steht, eine Verbindung der Formel

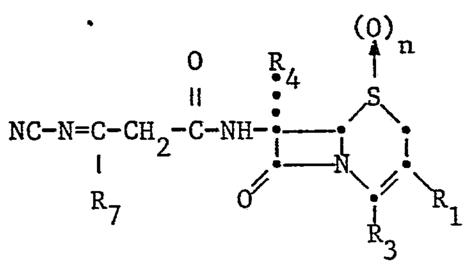

(XII),

worin $R_7$ für veräthertes Hydroxy steht, n, $R_1$, $R_3$ und $R_4$ die unter Formel I angegebenen Bedeutungen haben und worin gegebenenfalls vorhandene funktionelle Gruppen in Resten $R_1$ und/oder $R_3$ in geschützter Form vorliegen können, mit einem Hydrazin der Formel $R_6-NH-NH_2$ (XI) umsetzt, oder

j) zur Herstellung einer Verbindung der Formel I, worin $R_6$ einen gegebenenfalls substituierten Kohlenwasserstoffrest, organisches Sulfonyl oder Acyl darstellt, in eine Verbindung der Formel

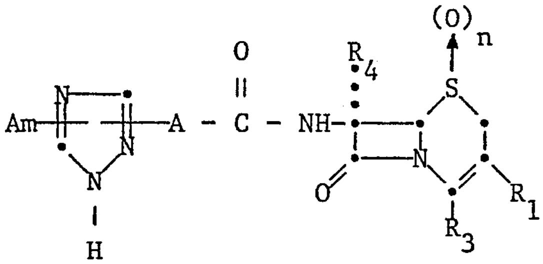

(XIII),

worin n, $R_1$, $R_3$, $R_4$, A und Am die unter Formel I angegebenen Bedeutungen haben und worin funktionelle Gruppen erforderlichenfalls in geschützter Form vorliegen, den Rest $R_6$ einführt, und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin n 0 bedeutet, in eine Verbindung der Formel I überführt, worin n 1 bedeutet, oder eine Verbindung der Formel I, worin n 1 bedeutet, in eine Verbindung der Formel I überführt, worin n 0 bedeutet, und/oder in einer Verbindung der Formel I in geschützter Form vorliegende funktionelle Gruppen in die freien funktionellen Gruppen überführt und/oder ein erhältliches Salz in die freie Verbindung der Formel (I) oder in ein anderes Salz überführt, und/oder eine erhältliche freie Verbindung der Formel (I) mit einer salzbildenden Gruppe in ein Salz überführt und/oder ein erhältliches Gemisch von isomeren Verbindungen der Formel I in die einzelnen Isomeren auftrennt.

2. Verfahren zur Herstellung von Verbindungen der Formel I, worin n für 0 oder 1 steht, $R_1$ Wasserstoff, veräthertes oder verestertes Hydroxy, veräthertes Mercapto, Formyl oder einen Rest $-CH_2-R_2$ darstellt, worin $R_2$ Wasserstoff, gegebenenfalls funktionell abgewandeltes Carboxy, Acyloxy, Hydroxy, Heterocyclylthio oder Ammonio ist, $R_3$ eine gegebenenfalls geschützte Carboxylgruppe bedeutet, $R_4$ für Wasserstoff oder Niederalkoxy steht, A eine Gruppe $-CH_2-$, $-C(=O)-$

oder $-C(=N-O-R_5)-$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest oder Acyl darstellt und Am Amino ist, und Salzen von solchen Verbindungen der Formel I, die eine salzbildende Gruppierung aufweisen, dadurch gekennzeichnet, dass man

a) eine Verbindung der Formel II, worin n, $R_1$, $R_3$ und $R_4$ die unter Formel I angegebenen Bedeutungen haben und worin die 7β-Aminogruppe gegebenenfalls durch eine die Acylierungsreaktion erlaubende Gruppe substituiert ist und gegebenenfalls vorhandene funktionelle Gruppen geschützt sein können, durch Umsetzung mit einem den Acylrest einer Carbonsäure der Formel III einführenden Acylierungsmittel, worin A, $R_6$ und Am die unter Formel I angegebenen Bedeutungen haben und vorhandene funktionelle Gruppen gegebenenfalls geschützt sind, acyliert oder

b) zur Herstellung einer Verbindung der Formel I, worin $R_1$ den Rest der Formel $-CH_2-R_2$ darstellt, worin $R_2$ Ammonio oder Heterocyclylthio ist, eine Verbindung der Formel IV, worin n, $R_3$, A, $R_4$, $R_6$ und Am die unter Formel I angegebenen Bedeutungen haben, $R_2'$ einen durch nucleophile Reaktion ersetzbaren Rest darstellt und worin gegebenenfalls vorhandene funktionelle Gruppen in geschützter Form vorliegen können, mit einem tertiären Amin oder Mercaptan der Formel $R_2-H$ behandelt, oder

c) zur Herstellung einer Verbindung der Formel I, worin $R_4$ Niederalkoxy bedeutet, in eine Verbindung der Formel V, worin n, $R_1$, $R_3$, A, $R_6$ und Am die unter Formel I angegebenen Bedeutungen haben und worin funktionelle Gruppen vorzugsweise geschützt sind, in 7α-Stellung eine Niederalkoxygruppe einführt, oder

d) eine 2-Cephemverbindung der Formel VI, worin n, $R_1$, $R_3$, $R_4$, A, $R_6$ und Am die unter Formel I angegebenen Bedeutungen haben und worin gegebenenfalls vorhandene funktionelle Gruppen in geschützter Form vorliegen können, zur entsprechenden 3-Cephemverbindung isomerisiert, oder

e) zur Herstellung einer Verbindung der Formel I, worin A eine Gruppe $-C(=N-O-R_5)-$ ist, eine Verbindung der Formel VII, worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Hydroxylamin der Formel $H_2N-O-R_5$ umsetzt, oder

f) zur Herstellung einer Verbindung der Formel I, worin A eine Gruppe $-C(=N-O-R_5)-$ ist, worin $R_5$ gegebenenfalls substituiertes Niederalkyl oder Cycloalkyl, Cycloalkenyl, Niederalkenyl oder gegebenenfalls N-substituiertes Carbamoyl bedeutet, eine Verbindung der Formel VIII, worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem den Rest $R_5$ einführenden Mittel behandelt, oder

g) zur Herstellung einer Verbindung der Formel I, worin A eine Gruppe $-C(=N-O-R_5)-$ ist, worin $R_5$ Wasserstoff bedeutet, eine Verbindung der Formel IX, worin n, $R_1$, $R_3$, $R_4$, $R_6$ und Am die unter Formel I angegebenen Bedeutungen haben und worin gegebenenfalls vorhandene funktionelle Gruppen in geschützter Form vorliegen können, mit einem Nitrosierungsmittel behandelt, oder

h) zur Herstellung einer Verbindung der Formel I, worin $R_3$ eine geschützte Carboxylgruppe darstellt, in einer Verbindung der Formel X, worin n, $R_1$, $R_4$, A, $R_6$ und Am die unter Formel I angegebenen Bedeutungen haben und worin gegebenenfalls vorhandene funktionelle Gruppen in geschützter Form vorliegen können, die 4-Carboxylgruppe in eine geschützte Carboxylgruppe überführt, oder

i) zur Herstellung einer Verbindung der Formel I, worin A für eine Gruppe $-CH_2-$ steht, eine Verbindung der Formel XII, worin $R_7$ für veräthertes Hydroxy steht, n, $R_1$, $R_3$ und $R_4$ die unter Formel I angegebenen Bedeutungen haben und worin gegebenenfalls vorhandene funktionelle Gruppen in Resten $R_1$ und/oder $R_3$ in geschützter Form vorliegen können, mit einem Hydrazin der Formel $R_6-NH-NH_2$ (XI) umsetzt, oder

j) zur Herstellung einer Verbindung der Formel I, worin $R_6$ einen gegebenenfalls substituierten Kohlenwasserstoffrest oder Acyl darstellt, in eine Verbindung der Formel XIII, worin n, $R_1$, $R_3$, $R_4$, A und Am die unter Formel I angegebenen Bedeutungen haben und worin funktionelle Gruppen erforderlichenfalls in geschützter Form vorliegen, den Rest $R_6$ einführt, und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin n 0 bedeutet, in eine Verbindung der Formel I überführt, worin n 1 bedeutet, oder eine Verbindung der Formel I, worin n 1 bedeutet, in eine Verbindung der Formel I überführt, worin n 0 bedeutet, und/oder in einer Verbindung der Formel I in geschützter Form vorliegende funktionelle Gruppen in die freien funktionellen Gruppen überführt und/oder ein erhältliches Salz in die freie Verbindung der Formel (I) oder in ein anderes Salz überführt, und/oder eine erhältliche freie Verbindung der Formel (I) mit einer salzbildenden Gruppe in ein Salz überführt und/oder ein erhältliches Gemisch von isomeren Verbindungen der Formel I in die einzelnen Isomeren auftrennt.

3. Verfahren nach Anspruch 1 zur Herstellung von Verbindungen der Formel I, worin n 0 oder 1 ist, $R_1$ Wasserstoff, Niederalkoxy, Phenylniederalkoxy, Halogen, Niederalkanoyloxy, Niederalkansulfonyloxy, Arensulfonyloxy, Heterocyclylthio, worin Heterocyclyl über ein Ringkohlenstoffatom an die Thiogruppe gebundenes, unsubstituiertes oder durch Niederalkyl, das Diniederalkylamino, Carboxy oder Sulfo als Substituenten enthalten kann, substituiertes Thiadiazolyl, Oxadiazolyl, Triazolyl oder Tetrazolyl bedeutet, oder einen Rest $-CH_2-R_2$ darstellt, worin $R_2$ Wasserstoff, Hydroxy, Carboxy, Niederalkoxycarbonyl, Niederalkanoyloxy, Carbamoyloxy, N-Niederalkylcarbamoyloxy, Heterocyclylthio, worin Heterocyclyl einen über ein Ringkohlenstoffatom an die Thiogruppe gebundenen monoheterocyclischen Rest mit 1 bis 4 Ringstickstoffatomen und gegebenenfalls einem weiteren Ringheteroatom der Gruppe Sauerstoff und Schwefel darstellt, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell gesättigt und/oder unsubstituiert oder durch Niederalkyl, durch Carboxy, Sulfo, Diniederalkylamino, Niederalkanoylamino, Hydroxy oder Halogen substituiertes Niederalkyl, ferner durch Cycloalkyl, Phenyl, Benzyl, Carboxy, Carbamoyl oder N-Niederalkylcarbamoyl substituiert sein können, oder Ammonio, wie Triniederalkylammonio oder unsubstituiertes oder durch Niederalkyl, Hydroxyniederalkyl, Carboxyniederalkyl, Sulfoniederalkyl, Carboxy, Carbamoyl, N-Niederalkylcarbamoyl, Halogen, Sulfo, Niederalkanoyl, Hydroxy oder Cyano substituiertes Pyrazolio, Triazolio, Pyridinio, Pyrimidinio, Pyridazinio, Thiazolinio oder Chinolinio, bedeutet, $R_3$ Carboxyl oder in physiologisch spaltbarer Form verestertes Carboxyl darstellt, $R_4$ für Wasserstoff oder Niederalkoxy steht, A eine Gruppe $-CH_2-$, $-C(=O)-$ oder $-C(=N-O-R_5)-$ ist, worin $R_5$ Wasserstoff, Niederalkyl, Cycloalkyl, durch Phenyl, Niederalkoxy, Carboxy, Amino oder Diniederalkylamino substituiertes Niederalkyl oder Cycloalkyl, ferner Cycloalkenyl, Niederalkenyl, Carbamoyl oder N-Niederalkylcarbamoyl bedeutet, $R_6$ Wasserstoff, Niederalkyl, durch Hydroxy, Niederalkoxy, Carboxy, Niederalkoxycarbonyl, Amino, Niederalkylamino, Diniederalkylamino, Carbamoyl, Halogen, Sulfo, Niederalkylthio, Cyano oder Aminosulfinyl substituiertes Niederalkyl, ferner Niederalkenyl, Cycloalkyl, Cycloalkenyl, Phenyl, Phenylniederalkyl, Niederalkylsulfonyl, unsubstituiertes oder durch Niederalkyl oder Halogen substituiertes Phenylsulfonyl oder Phenylniederalkylsulfonyl, Niederalkanoyl, Niederalkoxycarbonyl, Carbamoyl oder N-Niederalkylcarbamoyl darstellt

und Am Amino oder Diniederalkylamino-niederalkylidenamino ist, und Salzen, insbesondere pharmazeutisch verwendbaren Salzen von solchen Verbindungen der Formel I, die salzbildende Gruppen aufweisen.

4. Verfahren nach Anspruch 2 zur Herstellung von Verbindungen der Formel I, worin $R_6$ Wasserstoff, Niederalkyl, durch Hydroxy, Niederalkoxy, Carboxy, Niederalkoxycarbonyl, Amino, Niederalkylamino, Diniederalkylamino, Carbamoyl, Cyano, Halogen, Sulfo, Niederalkylthio oder Aminosulfinyl substituiertes Niederalkyl, ferner Niederalkenyl, Cycloalkyl, Cycloalkenyl, Phenyl, Phenylniederalkyl, Niederalkanoyl, Niederalkoxycarbonyl, Carbamoyl oder N-Niederalkylcarbamoyl darstellt, Am Amino ist und n, $R_1$, $R_3$, $R_4$ und A die in Anspruch 3 angegebenen Bedeutungen haben, und Salzen, insbesondere pharmazeutisch verwendbaren Salzen von solchen Verbindungen der Formel I, die salzbildende Gruppen aufweisen.

5. Verfahren nach Anspruch 1 zur Herstellung von Verbindungen der Formel I, worin n 0 oder 1 ist, $R_1$ Wasserstoff, Niederalkoxy, z.B. Methoxy, Phenylniederalkoxy, z.B. Benzyloxy, Halogen, z.B. Chlor, Heterocyclylthio, worin Heterocyclyl über ein Ringkohlenstoffatom an die Thiogruppe gebundenes, unsubstituiertes oder durch Niederalkyl, z.B. Methyl, substituiertes Thiadiazolyl, z.B. 1,3,4-Thiadiazol-2-yl, oder Oxadiazolyl, z.B. 1,3,4-Oxadiazol-2-yl, bedeutet, oder einen Rest $-CH_2-R_2$ darstellt, worin $R_2$ Wasserstoff, Niederalkanoyloxy, z.B. Acetyloxy, Carbamoyloxy, N-Niederalkylcarbamoyloxy, z.B. N-Methylcarbamoyloxy, unsubstituiertes oder durch Niederalkyl, z.B. Methyl, Carboxyniederalkyl, z.B. Carboxymethyl oder 2-Carboxyäthyl, Sulfoniederalkyl, z.B. Sulfomethyl oder 2-Sulfoäthyl, Diniederalkylaminoniederalkyl, z.B. 2-Dimethylaminoäthyl, oder Niederalkanoylamino, z.B. Acetylamino, substituiertes Tetrazol-5-ylthio, unsubstituiertes oder durch Niederalkyl, z.B. Methyl, oder Carboxyniederalkyl, z.B. Carboxymethyl oder 2-Carboxyäthyl, substituiertes 1,3,4-Thiadiazol-2-ylthio, unsubstituiertes oder durch Niederalkyl, z.B. Methyl, substituiertes 1,3,4-Oxadiazol-2-ylthio, unsubstituiertes oder durch Niederalkyl, z.B. Methyl, oder Carboxyniederalkyl, z.B. Carboxymethyl, substituiertes 1,2,4-Triazol-3-ylthio, unsubstituiertes oder durch Hydroxy substituiertes Pyridazin-6-ylthio, oder unsubstituiertes oder durch Hydroxy substituiertes Pyrimidin-2-ylthio, oder Triniederalkylammonio, z.B. Trimethyl- oder Triäthylammonio, durch Niederalkyl, z.B. Methyl, N-substituiertes 1,2,3-Triazolio, durch Niederalkyl, z.B. Methyl, oder Carboxyniederalkyl, z.B. Carboxymethyl, N-substituiertes Pyrazolio, oder unsubstituiertes oder durch Niederalkyl, z.B. Methyl, Carboxy, Carbamoyl, Hydroxyniederalkyl, z.B. Hydroxymethyl, Carboxyniederalkyl, z.B. Carboxymethyl oder 2-Carboxyäthyl, Sulfoniederalkyl, z.B. Sulfomethyl, Halogen, z.B. Brom oder Chlor, oder Hydroxy substituiertes Pyridinio, bedeutet, $R_3$ Carboxyl oder in physiologisch spaltbarer Form verestertes Carboxyl, wie Niederalkanoyloxymethoxycarbonyl, z.B. Pivaloyloxy- oder Acetyloxymethoxycarbonyl, 2-Phthalidyloxycarbonyl oder Niederalkoxycarbonyloxymethoxycarbonyl, z.B. Aethoxycarbonyloxymethoxycarbonyl, darstellt, $R_4$ Wasserstoff oder, vorzugsweise wenn -A- eine Gruppe $-CH_2-$ ist, Methoxy bedeutet, A eine Gruppe $-CH_2-$, -C(=O)- oder $-C(=N-O-R_5)-$ ist, worin $R_5$ Wasserstoff, Niederalkyl, z.B. Methyl, Phenylniederalkyl, z.B. Benzyl, Carboxyniederalkyl, z.B. 2-Carboxy-2-propyl, Carboxycycloalkyl, z.B. 1-Carboxy-1-cyclopropyl, Carbamoyl, N-Niederalkylcarbamoyl, z.B. N-Methylcarbamoyl, oder Niederalkenyl, z.B. Allyl, bedeutet, und worin die Gruppe $-C(=N-O-R_5)-$ vorzugsweise in der syn-(oder Z-) Konfiguration vorliegt, wobei -A- mit der 3-Stellung des 1,2,4-Triazolringes verknüpft ist, $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. Dimethylaminoäthyl, Niederalkoxycarbonyl, z.B. Methoxy- oder Aethoxycarbonyl, Carbamoyl, Niederalkenyl, z.B. Allyl, Phenyl, Phenylniederalkyl, z.B. Benzyl oder Diphenylmethyl, oder Niederalkylsulfonyl,

z.B. Methylsulfonyl, darstellt und Am Amino ist, und Salzen, insbesondere pharmazeutisch verwendbaren Salzen, von solchen Verbindungen der Formel I, die salzbildende Gruppen aufweisen.

6. Verfahren nach Anspruch 2 zur Herstellung von Verbindungen der Formel I, worin $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. Dimethylaminoäthyl, Niederalkoxycarbonyl, z.B. Methoxy- oder Aethoxycarbonyl, Carbamoyl, Niederalkenyl, z.B. Allyl, Phenyl oder Phenylniederalkyl, z.B. Benzyl oder Diphenylmethyl, darstellt, und n, $R_1$, $R_3$, $R_4$, A und Am die in Anspruch 5 angegebenen Bedeutungen haben, und Salzen, insbesondere pharmazeutisch verwendbaren Salzen, von solchen Verbindungen der Formel I, die salzbildende Gruppen aufweisen.

7. Verfahren nach einem der Ansprüche 1 oder 2 zur Herstellung von Verbindungen der Formel I, worin n für 0 steht, $R_1$ Wasserstoff, Niederalkoxy, z.B. Methoxy, oder einen Rest $-CH_2-R_2$ darstellt, worin $R_2$ Niederalkanoyloxy, z.B. Acetyloxy, in 1-Stellung durch Niederalkyl, z.B. Methyl, Carboxyniederalkyl, z.B. Carboxymethyl oder 2-Carboxyäthyl, Sulfoniederalkyl, z.B. Sulfomethyl oder 2-Sulfoäthyl, oder Diniederalkylaminoniederalkyl, z.B. 2-Dimethylaminoäthyl, substituiertes 1H-Tetrazol-5-ylthio, in 2-Stellung durch Niederalkyl, z.B. Methyl, oder Carboxyniederalkyl, z.B. Carboxymethyl, substituiertes 1,3,4-Thiadiazol-5-ylthio, 2-Niederalkylpyrazolio, z.B. 2-Methylpyrazolio, 3-Niederalkyl-1-(1,2,3-triazolio), z.B. 3-Methyl-1-(1,2,3-triazolio), Carboxypyridinio, z.B. 3- oder 4-Carboxypyridinio, Carbamoylpyridinio, z.B. 3- oder 4-Carbamoylpyridinio, Hydroxyniederalkylpyridino, z.B. 4-Hydroxymethylpyridinio, Carboxyniederalkylpyridinio, z.B. 3-Carboxymethylpyridinio, oder Halogenpyridinio, z.B. 3- oder 4-Brompyridinio, bedeutet, $R_3$ Carboxyl oder Niederalkanoyloxymethoxycarbonyl, z.B. Pivaloyloxymethoxycarbonyl, oder Niederalkoxycarbonyloxymethoxycarbonyl, z.B. Aethoxycarbonyloxymethoxycarbonyl, darstellt, $R_4$ für Wasserstoff oder, vorzugsweise wenn A eine Gruppe $-CH_2-$ ist, für Methoxy steht, A eine Gruppe $-CH_2-$ oder $-C(=N-O-R_5)-$ ist, worin $R_5$ Wasserstoff, Niederalkyl, z.B. Methyl, Carboxyniederalkyl, z.B. 2-Carboxy-2-propyl, Carbamoyl oder N-Niederalkylcarbamoyl, z.B. N-Methylcarbamoyl, bedeutet und worin die Gruppe $-C(=N-O-R_5)-$ in syn-(oder Z-)Konfiguration vorliegt, wobei -A- mit der 3-Stellung des 1,2,4-Triazolringes verknüpft ist, $R_6$ Wasserstoff, Niederalkyl, z.B. Methyl, Carboxyniederalkyl, z.B. Carboxymethyl oder 2-Carboxyäthyl, Diniederalkylaminoniederalkyl, z.B. 2-Dimethylaminoäthyl, oder Phenylniederalkyl, z.B. Benzyl oder Diphenylmethyl, darstellt und Am Amino ist, und Salzen, insbesondere pharmazeutisch verwendbaren Salzen, von solchen Verbindungen der Formel I, die salzbildende Gruppen aufweisen.

8. Verfahren nach Anspruch 1 zur Herstellung von 7β-[2-(5-Amino-1-methyl-1H-1,2,4-triazol-3-yl)-2-Z-methoxyimino-acetamino]-3-(4-hydroxymethylpyridiniomethyl)-3-cephem-4-carboxylat und pharmazeutisch verwendbaren Salzen davon.

9. Verfahren nach Anspruch 1 zur Herstellung von 7β-[2-(5-Amino-1-carboxymethyl-1H-1,2,4-triazol-3-yl)-2-Z-methoxyimino-acetamino]-3-acetoxymethyl-3-cephem-4-carbonsäure und pharmazeutisch verwendbaren Salzen davon.

10. Verfahren nach Anspruch 1 zur Herstellung von 7β-[2-(5-Amino-1-methyl-1H-1,2,4-triazol-3-yl)-2-Z-methoxyimino-acetamino]-3-cephem-4-carbonsäure-pivaloyloxymethylester und pharmazeutisch verwendbaren Salzen davon.

11. Verfahren nach Anspruch 2 zur Herstellung von 7β-[2-(5-Amino-1-methyl-1H-1,2,4-triazol-3-yl)-acetamino]-3-acetoxymethyl-3-cephem-4-carbonsäure und pharmazeutisch verwendbaren Salzen davon.

12. Verfahren nach Anspruch 2 zur Herstellung von 7β-[2-(5-Amino-1-methyl-1H-1,2,4-triazol-3-yl)-2-Z-methoxyimino-acetamino]-3-acetoxymethyl-3-cephem-4-carbonsäure und pharmazeutisch verwendbaren Salzen davon.

13. Verfahren nach Anspruch 2 zur Herstellung von 7β-[2-(5-Amino-1-methyl-1H-1,2,4-triazol-3-yl)-2-Z-methoxyimino-acetamino]-3-(1-methyltetrazol-5-yl-thiomethyl)-3-cephem-4-carbonsäure und pharmazeutisch verwendbaren Salzen davon.

14. Verfahren nach Anspruch 2 zur Herstellung von 7β-[2-(5-Amino-1-methyl-1H-1,2,4-triazol-3-yl)-2-Z-methoxyimino-acetamino]-3-(4-carbamoylpyridiniomethyl)-3-cephem-4-carboxylat und pharmazeutisch verwendbaren Salzen davon.

15. Verfahren nach Anspruch 2 zur Herstellung von 7β-[2-(5-Amino-1-methyl-1H-1,2,4-triazol-3-yl)-2-Z-methoxyimino-acetamino]-3-cephem 4-carbonsäure und pharmazeutisch verwendbaren Salzen davon.

16. Verfahren nach Anspruch 2 zur Herstellung von 7β-[2-(5-Amino-1-methyl-1H-1,2,4-triazol-3-yl)-2-hydroxyimino-acetamino]-3-acetoxymethyl-3-cephem-4-carbonsäure und pharmazeutisch verwendbaren Salzen davon.

17. Verfahren nach Anspruch 2 zur Herstellung von 7β-[2-(5-Amino-1H-1,2,4-triazol-3-yl)-2-Z-methoxyimino-acetamino]-3-acetoxymethyl-3-cephem-4-carbonsäure und pharmazeutisch verwendbaren Salzen davon.

18. Verfahren zur Herstellung von Verbindungen der Formel

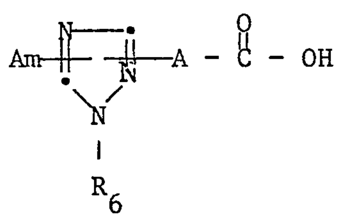

(III)

worin A eine Gruppe $-CH_2-$, $-C(=O)-$ oder $-C(=N-O-R_5)$ darstellt, worin $R_5$ Wasserstoff, gegebenenfalls substituiertes Niederalkyl oder Cycloalkyl, Cycloalkenyl, Niederalkenyl oder gegebenenfalls N-substituiertes Carbamoyl bedeutet, $R_6$ Wasserstoff, einen gegebenenfalls substituierten Kohlenwasserstoffrest, organisches Sulfonyl oder Acyl darstellt und Am eine gegebenenfalls substituierte Aminogruppe ist, und ihrer zur Acylierung geeigneten, reaktionsfähigen funktionellen Derivate, dadurch gekennzeichnet, dass man

a) zur Herstellung einer Verbindung der Formel III, worin A eine Gruppe $-CH_2-$ und Am Amino darstellt, eine Verbindung der Formel

$$NC-N=C(Z)-CH_2-R_8 \quad \text{(XIV)},$$

worin $R_8$ für Wasserstoff, Cyano oder geschütztes Carboxy und Z für veräthertes Hydroxy steht, mit einer Verbindung der Formel $R_6$-NH-$NH_2$, worin $R_6$ die unter Formel III angegebene Bedeutung hat, umsetzt und in einer erhaltenen Verbindung der Formel

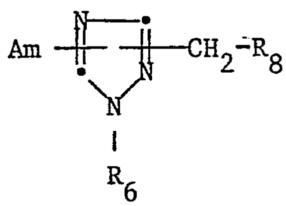

(XV),

worin $R_8$ Wasserstoff oder Cyano bedeutet, Wasserstoff oder Cyano durch Carboxy ersetzt, oder

b) zur Herstellung einer Verbindung der Formel III, worin A eine Gruppe -C(=O)- darstellt, eine Verbindung der Formel

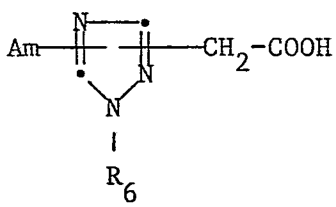

(IIIa),

worin $R_6$ die unter Formel III angegebene Bedeutung hat, Am eine substituierte Aminogruppe ist und die Carboxygruppe gegebenenfalls in geschützter Form vorliegt, mit einem Oxidationsmittel behandelt, oder

c) zur Herstellung einer Verbindung der Formel III, worin A eine Gruppe -C(=NOH)- darstellt, eine Verbindung der Formel

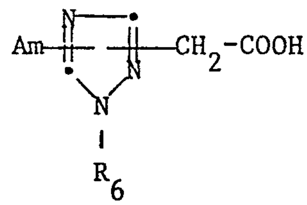

(IIIa),

worin $R_6$ die unter Formel III angegebene Bedeutung hat, Am eine substituierte Aminogruppe ist und die Carboxygruppe gegebenenfalls in geschützter Form vorliegt, mit einem Nitrosierungsmittel behandelt, oder

d) zur Herstellung einer Verbindung der Formel III, worin Am Amino und A eine Gruppe $-CH_2-$ darstellt, wobei eine solche Verbindung von 5-Amino-1,2,4-triazol-3-ylessigsäure verschieden ist, eine Verbindung der Formel

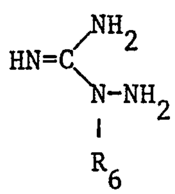

(XVI),

worin $R_6$ die unter Formel III angegebene Bedeutung hat, oder ein Salz davon mit Malonsäure umsetzt, oder

e) zur Herstellung einer Verbindung der Formel III, worin A eine Gruppe $-C(=NOR_5)-$ ist, wobei $R_5$ die unter Formel III angegebene Bedeutung hat, eine Verbindung der Formel

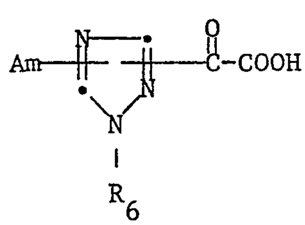

(IIIb),

worin $R_6$ und Am die unter Formel III angegebenen Bedeutungen haben, und die Carboxygruppe gegebenenfalls in geschützter Form vorliegt, mit einem Hydroxylamin der Formel $H_2N-OR_5$ umsetzt, und gewünschtenfalls in einer erfindungsgemäss erhältlichen Verbindung der Formel III

eine geschützte Aminogruppe Am in eine freie oder in eine andere geschützte Aminogruppe Am überführt, oder eine freie Aminogruppe Am in eine substituierte Aminogruppe Am überführt, und/oder eine geschützte Carboxygruppe in die freie Carboxygruppe oder in ein reaktionsfähiges funktionelles Derivat davon überführt, und/oder, wenn gewünscht, in einer erfindungsgemäss erhältlichen Verbindung der Formel III den Rest $R_6$ in einen anderen Rest $R_6$ überführt, und/oder ein erhältliches Gemisch von isomeren Verbindungen der Formel III in die einzelnen Isomeren auftrennt.

19. Verfahren zur Herstellung von Verbindungen der Formel III, worin $R_6$ Wasserstoff, einen gegebenenfalls substituierten Kohlenwasserstoffrest oder Acyl darstellt, Am eine gegebenenfalls geschützte Aminogruppe ist und A die in Anspruch 21 angegebene Bedeutung hat, und ihrer zur Acylierung geeigneten, reaktionsfähigen funktionellen Derivate, dadurch gekennzeichnet, dass man

a) zur Herstellung einer Verbindung der Formel III, worin A eine Gruppe $-CH_2-$ darstellt, eine Verbindung der Formel XIV, worin $R_8$ für Wasserstoff, Cyano oder geschütztes Carboxy und Z für veräthertes Hydroxy steht, mit einer Verbindung der Formel $R_6-NH-NH_2$, worin $R_6$ die unter Formel III angegebene Bedeutung hat, umsetzt und in einer erhaltenen Verbindung der Formel XV, worin $R_8$ Wasserstoff oder Cyano bedeutet, Wasserstoff oder Cyano durch Carboxy ersetzt, oder

b) zur Herstellung einer Verbindung der Formel III, worin A eine Gruppe $-C(=O)-$ darstellt, eine Verbindung der Formel IIIa, worin $R_6$ die unter Formel III angegebene Bedeutung hat, Am eine geschützte Aminogruppe ist und die Carboxygruppe gegebenenfalls in geschützter Form vorliegt, mit einem Oxidationsmittel behandelt, oder

c) zur Herstellung einer Verbindung der Formel III, worin A eine Gruppe -C(=NOH)- darstellt, eine Verbindung der Formel IIIa, worin $R_6$ die unter Formel III angegebene Bedeutung hat, Am eine geschützte Aminogruppe ist und die Carboxygruppe gegebenenfalls in geschützter Form vorliegt, mit einem Nitrosierungsmittel behandelt, oder

d) zur Herstellung einer Verbindung der Formel III, worin A eine Gruppe $-C(=NOR_5)-$ ist, wobei $R_5$ die unter Formel III angegebene Bedeutung hat, eine Verbindung der Formel IIIb, worin $R_6$ und Am die unter Formel III angegebenen Bedeutungen haben und die Carboxygruppe gegebenenfalls in geschützter Form vorliegt, mit einem Hydroxylamin der Formel $H_2N-OR_5$ umsetzt, und gewünschtenfalls in einer erfindungsgemäss erhältlichen Verbindung der Formel III eine geschützte Aminogruppe Am in eine freie oder in eine andere geschützte Aminogruppe Am überführt, oder eine freie Aminogruppe Am in eine geschützte Aminogruppe Am überführt, und/oder eine geschützte Carboxygruppe in die freie Carboxygruppe oder in ein reaktionsfähiges funktionelles Derivat davon überführt, und/oder, wenn gewünscht, in einer erfindungsgemäss erhältlichen Verbindung der Formel III den Rest $R_6$ in einen anderen Rest $R_6$ überführt, und/oder ein erhältliches Gemisch von isomeren Verbindungen der Formel III in die einzelnen Isomeren auftrennt.

20. Verfahren zur Herstellung von pharmazeutischen Präparaten, dadurch gekennzeichnet, dass man eine nach dem Verfahren gemäss Anspruch 1 erhaltene Verbindung mit einem pharmazeutisch verwendbaren Trägerstoff mischt.

21. Verfahren zur Herstellung von pharmazeutischen Präparaten, dadurch gekennzeichnet, dass man eine nach dem Verfahren gemäss Anspruch 2 erhaltene Verbindung mit einem pharmazeutisch verwendbaren Trägerstoff mischt.